# EUROPÄISCHE PATENTANMELDUNG

(11) **EP 2 997 883 A1**
(43) Veröffentlichungstag der Anmeldung: **23.03.2016**
(21) Anmeldenummer: 14003225.1
(22) Anmeldetag: 17.09.2014
(51) Int. Cl.: A61B 5/00, A61B 10/02

(54) **Verfahren und Anordnung zur optischen Absorptionsmessung**

(71) Anmelder: Universität Stuttgart, 70174 Stuttgart (DE)
(72) Erfinder: Körner, Klaus, 70563 Stuttgart (DE); Pruß, Christof, 73760 Ostfildern (DE); Herkommer, Alois, 73431 Aalen (DE); Osten, Wolfgang, 70569 Stuttgart (DE); Claus, Daniel, 70195 Stuttgart (DE)
(74) Vertreter: Müller-Boré & Partner Patentanwälte PartG mbB

(57) **Zusammenfassung**

Die vorliegende Erfindung betrifft eine Messsonde und eine Anordnung zur spektralen Absorptionsmessung, vorzugsweise im Infrarot. Des Weiteren betrifft die vorliegende Erfindung ein Verfahren zur spektroskopischen Absorptionsmessung. Die Messsonde (100) umfasst eine Schneideeinrichtung (13, 17), die ausgelegt ist, eine Schicht bzw. einen Lappen aus einer zu vermessenden Probe (16) abzuschneiden; einen Messspalt (12), der ausgelegt ist, die Probenschicht aufzunehmen; ein optisches Fensterelement (10) zum Ein- und Auskoppeln von Messlicht in den bzw. aus dem Messspalt (12); und einen Endreflektor (19), der ausgelegt und angeordnet ist, das durch den Messspalt propagierte Messlicht zurück in den Messspalt (12) zu reflektieren. Die Anordnung zur spektralen Absorptionsmessung umfasst die Messsonde, eine Lichtquelle und eine Einrichtung zur spektralen Analyse des von dem Messspalt ausgekoppelten Messlichts.

## Beschreibung

Die vorliegende Erfindung betrifft eine Messsonde und eine Anordnung zur spektralen Absorptionsmessung, vorzugsweise durch Infrarot-Strahlung. Des Weiteren betrifft die vorliegende Erfindung ein Verfahren zur spektroskopischen Absorptionsmessung.

Eine Standard-Methode für die Krebsgewebe-Erkennung ist die histopathologische mikroskopische Untersuchung mittels Mikroskopie durch einen erfahrenen Histopathologen. Dabei werden Gewebeproben entnommen, geschnitten und stark ausgedünnt, gegebenenfalls angetrocknet und gegebenenfalls chemisch für die spektrale Messung in Transmission oder Transflexion präpariert. Die Analyse des Gewebes erfolgt im Labor, oft weit weg vom Entnahmeort entfernt. Die Messungen sind somit sehr zeit- und arbeitsaufwendig und hängen entscheidend von der Erfahrung des Histopathologen ab. Darüber hinaus sind die bekannten Lösungen zu voluminös für in-vivo Applikationen und würden gesundes Gewebe in erheblichen Maßstab verletzen oder zerstören. Dies führt zu nicht unerheblichen Kollateralschaden.

Ebenfalls sind Verfahren zur Krebsgewebe-Erkennung bekannt, die auf Infrarote Spektroskopie im mittleren Infrarotbereich (MIR), insbesondere die Fourier-Spektroskopie basieren. Beispielhafte Verfahren und Vorrichtungen zur in-vivo Messung mittels infraroter Spektroskopie, die auf miniaturisierte ATR-Prismen (ATR - Attenuated Total Reflection bzw. abgeschwächte Totalreflexion) in Verbindung mit Fourier-Transformations-Spektroskopie im MIR basieren, sind z.B. aus US 8,452,356 B2 bekannt.

Bedingt durch das Prinzip der ATR-Messung sind die beim Stand der Technik verwendeten ATR-Prismen jedoch eher stumpf und können leicht das untersuchte Gewebe verletzen oder zerstören. Eine ATR-Messung wird zudem stark durch den Anpressdruck des Gewebes an die totalreflektierende Fläche des ATR-Prismas beeinflusst. Der Anpressdruck ist in-vivo jedoch nicht sehr leicht zu beeinflussen oder zu garantieren und ebenfalls nicht leicht zu bestimmen. Davon hängt aber die Zuverlässigkeit des Messergebnisses sehr stark ab. Darüber hinaus ist die Ortsauflösung von auf ATR-Prismen basierten Messungen in der Regel nicht sehr hoch.

Eine Aufgabe der Erfindung ist es, eine optische Messvorrichtung bereitzustellen, die in miniaturisierter Form (insbesondere hinsichtlich deren lateraler Ausdehnung) gebaut werden kann und/oder genauere Messergebnisse in vorzugsweise kürzere Zeit liefert. Insbesondere ist es eine Aufgabe der Erfindung, eine optische Messvorrichtung zur minimal invasiven, in-vivo Untersuchungen von biologischen Proben (z.B. Gewebe) bereitzustellen. Eine weitere Aufgabe ist es, ein entsprechendes optisches Messverfahren bereitzustellen.

Um diese Aufgabe(n) zu lösen, wird gemäß einem ersten Aspekt der Erfindung eine Messsonde zur spektralen Absorptionsmessung bereitgestellt. Die Messsonde umfasst:
eine Schneideeinrichtung, die ausgelegt ist, eine Schicht bzw. einen Lappen aus einer zu vermessenden Probe abzuschneiden;
einen Messspalt, der ausgelegt ist, die Probenschicht aufzunehmen; und
ein optisches Fensterelement (Ein- und Auskoppelement) zum Ein- und Auskoppeln von Messlicht in den und aus dem Messspalt; und
einen Endreflektor, der ausgelegt und angeordnet ist, das durch den Messspalt propagierte Messlicht zurück in den Messspalt zu reflektieren.

Die Messsonde kann in einer Anordnung zur spektralen Absorptionsmessung integriert werden. Die Anordnung zur spektralen Absorptionsmessung kann ferner eine Lichtquelle und eine Einrichtung zur spektralen Analyse des vom Messspalt ausgekoppelten Messlichts umfassen.

Die Messsonde bzw. die Anordnung zur spektralen Absorptionsmessung kann z.B. eine Messsonde bzw. Anordnung zur spektralen Messungen (z.B. Molekülspektroskopie) im sichtbaren Bereich, Infraroten Bereich (z.B. in NIR-, MIR-, FIR- Bereich), Terahertz-Spektralbereich, etc. sein. Vorzugsweise ist die Messsonde bzw. Anordnung zur spektralen Absorptionsmessung ausgelegt, Messungen im infraroten Bereich (IR), insbesondere im mittleren infraroten Bereich (z.B. in den Wellenlängenbereich von 6 µm bis 11 µm, vorzugsweise von 7 µm bis 10,5 µm) durchzuführen. In diesem Bereich können einige der spektralen Schlüssel-Signaturen für Krebsgewebe bzw. Krebszellen, die als Krebsmarker dienen, im Spektrum lokalisiert werden. So kann beispielsweise das Verhältnis der Absorptionen in den Banden 1080 cm⁻¹ und 1236 cm⁻¹ einen Hinweis auf die Veränderung des Zahlenverhältnis von RNA zu DNA geben, das sich beim Vorhandensein von Krebszellen vergrößert. Ebenfalls sind aus der Fachliteratur andere "Krebsmarker" im MIR Bereich bekannt.

Es ist jedoch möglich die Messsonde zu Messungen im NIR-Bereich (z.B. für RAMAN-Spektroskopie), für Fluoreszenz-Spektroskopie, etc. einzusetzen.

Die Lichtquelle kann eine spektral breitbandige Lichtquelle, eine Multi-Wellenlängen Lichtquelle mit mindestens zwei Wellenlängen bzw. Wellenlängenbereichen, eine Wellenlängendurchstimmbarer Quelle, oder eine Lichtquelle mit adressierbarem Spektrum sein.

Die spektral breitbandige Lichtquelle kann z.B. ein (IR) Breitbandlaser, eine Synchrotron-Lichtquelle (insbesondere im IR Bereich) oder eine andere geeignete breitbandige Lichtquelle umfassen. Synchrotron-Strahlung im MIR erzeugt bei entsprechender Dosis keine Strahlenschäden (wie energiereiche Strahlung, Röntgen o.ä.) am Menschen, sondern "nur" thermische Schäden, die lokal begrenzt sind (z.B. in der 0,01 mm bis 0,1 mm Region). Durch das stets vorhandene Gewebewasser - auch in Knochen - besteht eine extrem starke (Absorptions-)Barriere im Körper, die weitere Schäden am gesunden Gewebe verhindert.

Die Multi-Wellenlängen Lichtquelle kann z.B. ein Quantenkaskaden-Laser oder eine Laserbatterie sein oder umfassen. Vorzugsweise ist der Quantenkaskaden-Laser wellenlängendurchstimmbar ausgebildet.

Die Lichtquelle mit adressierbarem bzw. adressiertem Spektrum kann z.B. eine Modulationsvorrichtung umfassen, die vorzugsweise eine Vielzahl (d.h. mindestens zwei) von Modulationskanälen mit jeweils vorbestimmter unterschiedlicher Modulationsfrequenz in den Modulationskanälen in einem vorgegebenen Modulationsfrequenzbereich (z.B. von 0,1 Hz bis 10 MHz) aufweist, wobei einem jeden Modulationskanal jeweils elektromagnetische Strahlung unterschiedlicher physikalischer Wellenlänge zugeordnet ist. Ferner kann die Lichtquelle mit adressierbarem bzw. adressiertem Spektrum eine spektral breitbandige Quelle, eine Vorrichtung zur spektralen Zerlegung und gegebenenfalls eine Vorrichtung zum Zusammenführen der einzelnen modulierten spektralen Kanäle umfassen. Eine Lichtquelle mit adressierbarem bzw. adressiertem Spektrum ist z.B. in der deutschen Patentanmeldung DE 10 2014 002 514.4 beschrieben.

Die Lichtquelle kann mit zumindest einem Faserausgang versehen sein. Ferner kann die Messsonde bzw. die Anordnung zur spektralen Absorptionsmessung einen optischen Kanal (Beleuchtungskanal) zum Transportieren des von der Lichtquelle ausgestrahlten Messlichts zum Fensterelement umfassen. Der Beleuchtungskanal kann z.B. eine Faser umfassen oder aus einer Faser bestehen. Die Faser kann z.B. eine Silberhalide-Faser sein. Es ist jedoch möglich, das von der Lichtquelle ausgestrahlte Messlicht direkt in das Fensterelement einzukoppeln.

Der Beleuchtungskanal kann weitere optische Elemente umfassen, wie z.B. Linsen, Spiegel, etc. Vorzugsweise umfasst der Beleuchtungskanal ein Kollimator oder ein Linsensystem zum Erzeugen eines im Wesentlichen kollimierten bzw. parallelen oder eines schwach fokussierten Messlichtstrahls. Es ist ferner möglich, einen konvergierenden bzw. fokussierten Messlichtstrahl zu verwenden.

Die Messsonde bzw. die Anordnung zur spektralen Absorptionsmessung kann ferner einen spektralen Filter umfassen, der ausgelegt ist, vorbestimmte Spektralbanden bzw. Spektralbereiche aus einem breitbandigen Spektrum oder aus einem Spektrum mit mehreren Wellenlängen oder Wellenlängenbereichen herauszufiltern.

Herkömmliche breitbandige Lichtquellen können breitbandiges Licht mit einem kontinuierlichen oder diskreten Spektrum liefern, das sich über mehrere Wellenlängen oder Wellenlängenbereiche erstreckt. Häufig liegt jedoch die gesuchte spektrale Signatur nur bei einer kleineren Anzahl (beispielsweise 20) von Absorptionsbanden und/oder deren Verhältnis zueinander. Die relevanten Absorptionsbanden können vorab bekannt sein und z.B. in einer Datenbank abgespeichert werden. Es wird gemäß einem Aspekt der Erfindung vorgeschlagen, aufgrund einer begrenzten Anzahl vorab bekannter und in einem definierten Kontext (z.B. zur Krebsuntersuchung) relevanter Absorptionsbanden, nur diese relevanten Absorptionsbanden, vorzugsweise mit einer gewissen spektralen Verbreiterung, zur Absorptionsmessung der Probe zuzuführen und anschließend auszuwerten. Somit kann die beim Absorptionsmessen eingebrachte Strahlungslast reduziert werden. Die Messsonde bzw. die Anordnung zur spektralen Absorptionsmessung umfasst in diesem Fall einen Filter, welcher der Lichtquelle nachgeordnet ist und ausgelegt ist, vorbestimmte Spektralbanden aus dem breitbandigen Spektrum herauszufiltern. Der Filter kann z.B. ein Frequenzkamm-Filter sein, welcher ausgelegt ist, im Wesentlichen nur die zur Absorptionsmessung relevanten Spektralanteile, wo sich mit einer gewissen Wahrscheinlichkeit die relevanten Absorptionsbanden befinden, passieren zu lassen. Der Frequenzkamm-Filter kann z.B. wie ein dispersives Spektrometer mit einem Beugungsgitter aufgebaut sein. Im Bereich der spektralen Zerlegung des als ein dispersives Spektrometer aufgebauten Frequenzkamm-Filters können sich stark reflektierende und stark absorbierende Bereiche befinden, wobei die unerwünschten Spektralanteile durch die stark absorbierenden Bereiche dunkel geschaltet werden. Die stark reflektierten (erwünschten bzw. relevanten) Spektralanteile können mittels Diffraktion an einem Beugungsgitter wieder in einem Fokusfleck vereinigt und z.B. möglichst gut in eine Faser (z.B. eine Monomode-Faser) eingekoppelt werden.

Des Weiteren kann die Messsonde bzw. die Anordnung zur spektralen Absorptionsmessung eine Einrichtung zur spektralen Analyse des vom Messspalt ausgekoppelten Messlichts umfassen. Die Einrichtung zur spektralen Analyse kann z.B. als ein dispersives Spektrometer oder als ein Fourier-Transform-Spektrometer ausgebildet sein. Bei einer durchstimmbaren Quelle elektromagnetischer Strahlung kann die Einrichtung zur spektralen Analyse nur den Detektor für elektromagnetische Strahlung umfassen. Bei einer multispektralen Lichtquelle mit adressierbaren Spektrum kann die Einrichtung zur spektralen Analyse einen Detektor für elektromagnetische Strahlung und eine Vorrichtung zur zeitlichen Frequenzanalyse (z.B. Fourier- oder Waveletanalyse) des detektierten Signals umfassen. Vorzugsweise ist in diesem Fall der Detektor ein gerasterter Detektor. Der Detektor kann z.B. ein Detektor (insbesondere in Integraldetektor) für IR Strahlung sein, z.B. ein Quecksilber-Cadmium-Tellurid-Detektor, MCM Detektor, welcher mit flüssigem Stickstoff gekühlt wird.

Ferner kann die Einrichtung zur spektralen Analyse ein, in der Zeit scannendes oder räumliches Zweistrahl-Interferometer umfassen, das einer breitbandigen oder Multi-Wellenlängen-Lichtquelle nachgeordnet ist.

Das vom Detektor erfasste Signal kann mittels einer Signalauswerteeinrichtung analysiert werden, um spektrale Merkmale aus der untersuchten Probe zu gewinnen und gegebenenfalls diese Merkmale bestimmten Gruppen (z.B. gesund, krank, etc.) zuzuordnen. Die Signalauswerteinrichtung kann zumindest einen Rechner oder ein Rechensystem umfassen, der/das programmiert ist, z.B. eine Hauptachsentransformation (PCA, Principal Component Analysis), um eine räumliche und/oder zeitliche Fourier Analyse (insbesondere eine FFT-Analyse), eine Waveletanalyse, eine Lock-in-Detektion oder eine andere geeignete Analyse des detektierten Intensitätsprofils bzw. der detektierten zeitlichen Variation der Intensität durchzuführen.

Ferner kann die Anordnung zur spektralen Absorptionsmessung einen optischen Kanal (Detektionskanal) zum Transportieren des aus dem Messspalt ausgekoppelten Messlichts zur Einrichtung zur spektralen Analyse umfassen. Der Detektionskanal kann eine Faser umfassen oder aus einer Faser bestehen. Ferner kann der optische Kanal weitere optische Elemente umfassen, wie z.B. Linsen, Spiegel, etc. Der Detektionskanal kann z.B. eine Fokussieroptik umfassen, die vor dem Detektor angeordnet ist. Vorzugsweise ist in der Brennebene der Fokussieroptik eine (feine) Blende (Abschattblende) angeordnet, um unerwünschtes Streulicht (z.B. Mie-Streuung) und/oder unerwünschte, unter Umständen starke Reflexionsspots auszublenden oder zumindest zu reduzieren. Dies gilt insbesondere bei der Anwendung eines Lasers (z.B. eines QCL). Die Blende kann als Spaltblende ausgebildet sein. Die Vorrichtung zur spektralen Absorptionsmessung kann ebenfalls mehrere Detektionskanäle umfassen, die z.B. das Messlicht in unterschiedlichen Spektralbereichen zu entsprechenden Detektoren transportieren. Somit kann z.B. einer starken spektralen Abspaltung in der Messsonde (z.B. durch Verwendung diffraktiver Elemente) Rechnung getragen werden.

In einer bevorzugten Ausführungsform können sich der Beleuchtungskanal und der Detektionskanal gemeinsame optische Elemente teilen. So können sich der Beleuchtungskanal und der Detektionskanal zumindest eine gemeinsame Faser und/oder einen Strahlteiler teilen. Das Messlicht kann über diese gemeinsame Faser in das Fensterelement eingekoppelt und aus dem Fensterelement ausgekoppelt werden. Ein Vorteil dieser Anordnung ist die Miniaturisierbarkeit der Messsonde, insbesondere in lateraler Richtung. Bei medizinischen Anwendungen kann die Messsonde somit patientenschonender gestaltet werden.

Vorzugsweise wird die Messsonde vibrations-unterstützt in die Probe (z.B. Gewebe eingebracht). Die Messsonde kann ebenfalls roboterarmgeführt in die Probe eingebracht werden.

Die Messsonde umfasst ferner eine Schneideeinrichtung, die ausgelegt ist, eine Schicht bzw. einen Lappen aus einer zu vermessenden Probe abzuschneiden. In einem Beispiel wird ein "quasi" endloser Probeschnitt erzeugt, der in der Sonde am Messspalt zeitnah nach Abschneiden vorbeitransportiert wird. Die Schneideeinrichtung kann ausgelegt sein, die Probenschicht durch mechanisches Trennen (z.B. durch Mikro-Hobeln, optional ultraschall- und/oder unterdruckunterstützt) zu erzeugen. Die Schneideeinrichtung kann ebenfalls ausgelegt sein, die Probenschicht mittels eines Femtosekunden-Laser-Mikrotoms zu erzeugen.

Da die Schneideeinrichtung mit einer sehr spitzen Schneidespitze bzw. Schneidekante ausgestaltet werden kann, wird die untersuchte Probe (z.B. das untersuchte Gewebe) nur wenig verletzt. Ferner ist es möglich, dichter (im Vergleich zum Stand der Technik) an der Spitze der Messsonde zu messen, so dass auch tiefer liegende Bereiche einer Probe vermessen werden können. Würde man dagegen eine feine Spitze auf ein aus dem Stand der Technik bekanntes ATR-Prisma einsetzen, um das Schneiden zu erleichtern, wäre es nicht möglich, auch Stellen im Tiefraum des Gewebes (z.B. Knochenhaut) zu vermessen, da das ATR-Prisma diese Stellen nicht erreichen kann.

Vorzugsweise ist die Schneideeinrichtung ausgelegt, eine Probenschicht bzw. -Lappen mit einer Dicke, die kleiner als 100 µm, vorzugsweise zwischen 1 µm und 30 µm ist, abzuschneiden. Typischerweise ist die Dicke der abgeschnittenen Probenschicht bzw. -Lappen zwischen ungefähr 4 µm bis 12 µm. Die von der Schneideeinrichtung ausgeschnittene Schicht (Probenschicht) ist somit relativ dünn und besitzt in Relation zu ihrer geringen Dicke zwei vergleichsweise groß ausgedehnte Begrenzungsflächen, die beispielsweise durch mechanisches Trennen erzeugt werden.

Eine der Begrenzungsflächen der Probenschicht kann an einer ersten Fensterfläche des für das Messlicht optisch transparenten Fensterelements liegen. Die erste Fensterfläche stellt somit eine der Begrenzungsflächen des Messspalts dar. Durch die erste Fensterfläche wird das Licht in dem Messspalt eingekoppelt und nach einem zweifachen Durchgang durch die sich im Messspalt befindliche Probenschicht wieder aus dem Messspalt ausgekoppelt. Die andere Begrenzungsfläche der Probenschicht kann an einer zweiten Fensterfläche (d.h. eine Fläche, die im Wesentlichen transparent für das Messlicht ist) eines zweiten Elements (z.B. eines Endspiegel-Trägerelements) oder direkt am Endreflektor liegen. Die zweite Fensterfläche oder der Endreflektor stellen in diesem Fall die zweite Begrenzungsfläche des Messspalts dar. Im Messvorgang liegt an jeder der Fensterflächen oder an der Fensterfläche und dem Endreflektor ein Teil des zumindest teilweise freigeschnittenen Probenschicht oder vereinzelten Probenschichtstücken an.

Zumindest eine der beiden optischen Fensterflächen (vorzugsweise beide Fensterflächen) und/oder der Endreflektor sind vorzugsweise plane Flächen oder schwach gekrümmte Flächen (z.B. Flächen mit einem Krümmungsradius größer als 500 Mikrometer), z.B. schwach gekrümmte sphärische oder zylindrische Flächen. Das optische Fensterelement kann ferner zumindest eine plane oder eine gekrümmte Fläche mit Brechkraft aufweisen. Die gekrümmte Fläche kann eine sphärische oder asphärische Fläche sein, z.B. eine Freiformfläche. Die gekrümmte Fläche kann die Eintrittsfläche des Messstrahls in das Fensterelement oder eine andere Fläche des optischen Fensterelements sein. Die gekrümmte Fläche kann z.B. so ausgelegt sein, dass das einfallende Messstrahlenbündel kollimiert wird.

Die dünne Probenschicht (z.B. ein dünner Gewebelappen) wird durchstrahlt, um z.B. Absorptionsspektren von Molekülen (z.B. Biomolekülen) zu diagnostischen Zwecken oder anderen Messzwecken direkt in der Probe (z.B. direkt am Gewebe) zu messen. Der optische Kontakt mit der sich im Messspalt befindlichen Probenschicht wird dabei über eine optische Fensterfläche des Fensterelements hergestellt.

Das durch das Fensterelement in den Messspalt eingekoppelte Messlicht durchläuft den Messspalt, so dass die dünne Probenschicht ein erstes Mal durchgestrahlt wird, um dort Absorptionen zu erzeugen. Das durch den Messspalt bzw. die Probenschicht propagierte Messlicht gelangt anschließend an den Endreflektor und wird vom Endreflektor zurück in den Messspalt reflektiert. Nach Reflexion am Endreflektor wird somit die dünne Probenschicht ein zweites Mal durchstrahlt, um noch einmal Absorptionen an derselben zu erzeugen. Dies ergibt eine Verstärkung des Messeffekts und begünstigt die Realisierung einer kleineren Messsonde.

Nach dem zweifachem Strahlungsdurchgang des Messlichts durch die sich im Messspalt befindliche Probenschicht kann das Messlicht entweder zumindest näherungsweise in sich selbst zurückgehen, um anschließend, z.B. durch Strahlteilung, in einem Detektionskanal eingekoppellt zu werden (Autofokus Ansatz), oder es breitet sich in einem vom Eingangsbündel-Strahlengang separierten Detektionskanal aus (Doppelfokus Ansatz). Die bei dem zweifachen Durchlauf durch die Probe nicht absorbierte und nicht oder nur schwach gestreute Photonen des Messlichts werden mittels eines Detektors erfasst.

Vorzugsweise sind die Schneideeinrichtung, der Messspalt, das optische Fensterelement und der Endreflektor und gegebenenfalls weitere Elemente (wie z.B. Endreflektor-Trägerelement, Abstransportkanal, etc.) in einem Gehäuse integriert. Vorzugsweise ist das Gehäuse aus Stahl oder einem anderen biokompatiblen Material und weist eine längliche Katheter- bzw. Nadelform auf. Vorzugsweise weist die Messsonde eine längliche Form mit einer Längsachse auf, z.B. eine Sonden-, Nadel- oder Katheterform, wobei der Durchmesser der Messsonde vorzugsweise im Bereich von 0,8 mm bis 3 mm und die Länge der Messsonde vorzugsweise im Bereich von 8 mm bis 150 mm liegen.

Die Schneideeinrichtung (die vorzugsweise in der Spitze der Messsonde angeordnet ist) weist vorzugweise zumindest eine Schneide auf, welche eine plankonkave, keilförmige, hobelförmige oder eine andere geeignete Form aufweisen kann. Die zumindest eine Schneide kann z.B. eine Hohlschneide sein. Die Hohl-Schneide kann einen Kanal aufweisen, der in den Messspalt mündet. Die Schneideeinrichtung kann ebenfalls eine Doppelschneide mit einer ersten und einer zweiten Schneide aufweisen. In diesem Fall wird vorzugsweise jede Begrenzungsfläche der dünnen Probenschicht bzw. -lappen durch Trennen mit einer eigenen Schneide erzeugt.

Die zumindest eine Schneide kann ein integraler Bestandteil des optischen Fensterelements sein oder kann (vorzugsweise fest) mit dem optischen Fensterelement verbunden sein. Die zumindest eine Schneide kann auch integraler Bestandteil eines Endreflektor-Trägerelements sein oder kann (vorzugsweise fest) mit dem Endreflektor-Trägerelement verbunden sein. Vorzugsweise ist die Verbindung zwischen der zumindest einen Schneide und dem optischen Fensterelement und/oder Endreflektor-Trägerelement mechanisch starr und erfolgt auf dem kürzesten Weg. Die Verbindung kann ferner eine optische Verbindung sein. Durch die Integration des optischen Fensterelements oder des Endreflektor-Trägerelements und der Schneideeinrichtung in einem einzigen Element, das sowohl eine optische Funktion als auch eine Schneidefunktion hat, kann die Messsonde klein und kompakt gebaut werden. Da die Messsonde eine geringere Anzahl mechanischer Teile aufweist, können die Stabilität und die Robustheit der Messsonde erhöht werden.

Die zumindest eine Schneide weist vorzugsweise eine Schneidespitze oder eine Schneidekante mit einem Winkel (Schneidewinkel) kleiner als 90°, vorzugsweise kleiner oder gleich 70°, weiter bevorzugt kleiner oder gleich 40°, besonders bevorzugt kleiner oder gleich 30°. So können extrem dünne Probenschichten (z.B. Gewebeschichten) aus der Probe ausgeschnitten werden, ohne die Probe unnötig zu verletzen (z.B. zerquetschen).

In einem Beispiel kann die Schneideeinrichtung eine zweite Schneide umfassen, welche vorzugsweise verschiebbar zur ersten Schneide ausgebildet ist. Die erste und die zweite Schneide bilden den Frontbereich der Messsonde bzw. die Spitze der Messsonde.

Die zweite Schneide kann ebenfalls eine plankonkave, keilförmige, hobeiförmige oder eine andere geeignete Form aufweisen. Die zweite Schneide kann zwei Schneideflächen aufweisen, welche eine Schneidespitze oder Schneidekante mit einem Winkel kleiner als 90°, vorzugsweise kleiner oder gleich 70°, weiter bevorzugt kleiner oder gleich 40°, besonders bevorzugt kleiner oder gleich 30° bilden.

In einem Beispiel kann die erste Schneide integraler Bestandteil des optischen Fensterelements sein oder kann mit diesem (vorzugsweise fest) verbunden sein. Die zweite Schneide kann integraler Bestandteil eines Endreflektor-Trägerelements sein oder kann mit diesem (vorzugsweise fest) verbunden sein. Die umgekehrte Anordnung ist ebenfalls denkbar. In einem Beispiel können die erste Schneide und die zweite Schneide so ausgelegt und angeordnet sein, dass die Schneideflächen der ersten und der zweiten Schneide den Einlass in den Messspalt bzw. den Probeneinlass begrenzen bzw. formen. Anders ausgedrückt können die in einem Abstand voneinander angeordneten erste und zweite Schneiden einen Kanal bzw. Probeneinlassraum bilden, der in den Messspalt mündet.

Die erste Schneide kann z.B. so angeordnet sein, dass sich eine der Schneideflächen nahtlos an eine der den Messspalt begrenzende Flächen, z.B. an die Fensterfläche des Fensterelements oder des Endreflektor-Trägerelements oder an den Endreflektor, anschließt. In einem Beispiel bildet diese Schneidefläche zusammen mit der Fensterfläche oder dem Endspiegel eine der den Messspalt begrenzenden Wände. Eine der beiden Schneideflächen der zweiten Schneide kann in der Ebene der anderen den Messspalt begrenzenden Flächen liegen und sich vorzugsweise nahtlos an diese Fläche anschließen.

Vorzugsweise ist die zweite Schneide beweglich in Bezug auf die erste Schneide angeordnet. Die zweite Schneide kann z.B. entlang der Längsrichtung der Anordnung zur Absorptionsmessung bzw. entlang der Vorschubrichtung und/oder entlang einer anderen Richtung (z.B. in einer zur Vorschubrichtung senkrechten oder geneigten Richtung) beweglich sein. Insbesondere kann sich die zweite Schneide durch eine Auf- und Ab-Schwingbewegung relativ zur Hauptschneide bewegen, was das Erzeugen eines sehr dünnen Gewebelappens sehr begünstigen kann. Die Schwingbewegung kann eine kleine Schwingamplitude hoher Frequenz aufweisen. So kann der mechanische Trennvorgang der Nebenschneide durch Vibration bzw. Mikroschwingungen im Ultraschall-Bereich unterstütz werden. Neben einer Verbesserung der Trennwirkung an der Probe können die Vibrationen bzw. die Mikroschwingungen der beweglichen Schneide eine Verdichtung der abgeschnittenen Probenschicht bewirken. Die Messsonde bzw. die Anordnung zur spektralen Absorptionsmessung kann folglich eine steuerbare Vorrichtung zum Bewegen der zweiten und gegebenenfalls der ersten Schneide umfassen (z.B. einen Piezoaktuator, einen elektrisch betriebenen Aktuator, etc.). Zur feinen und hochpräzisen und reibungsarmen Verschiebbarkeit der beweglichen Schneide kann in die Messsonde vorzugsweise ein Mikro-Lager (z.B. ein aerostatisches Mikro-Lager) integriert sein, das die bewegliche Schneide lagert. Zusätzlich oder alternativ können die Mikroschwingungen auf die Messsonde als Ganzes wirken. Die für das Bewegen der Schneide und/oder der Messsonde als Ganzes notwendige Energie kann z.B. durch Fluide (z.B. Gas oder Flüssigkeit) unter Druck, elektrisch (z.B. mittels Piezoeffekt), Wärme (z.B. Bimetall) oder in einer anderen Form zugeführt werden.

Vorzugsweise entspricht der Vorschub eines Bewegungsvorganges der beweglichen Schneide mindestens der halben Länge des lateralen Abtastbereichs beim Messen. Dabei bestimmt die Länge des Abtastbereichs die örtliche Auflösung der Messsonde.

In einem Beispiel kann die Schneideeinrichtung eine Hauptschneide und eine Nebenschneide zum Trennen von Proben umfassen, um eine vergleichsweise dünne Probenschicht (z.B. einen vergleichsweise dünnen Gewebelappen) zu erzeugen. Vorzugsweise ist die Hauptschneide der Messsonde derart angeordnet, dass sie beim "Anfahren" des Zielgebietes einer Messung stets als erste Schneide in tiefere Abschnitte der Probe eindringt. Die vorzugsweise relativ zur Hauptschneide bewegliche Nebenschneide bleibt zurück hinter der Hauptschneide, d.h. befindet sich nie vor der Hauptschneide. Die Nebenschneide kann in diesem Fall kleiner und schwächer gebildet sein.

In einem Beispiel kann die Hauptschneide integraler Bestandteil des optischen Fensterelements sein oder kann mit diesem (vorzugsweise fest) verbunden sein. Der Hauptschneide kann die optische Fensterfläche des Fensterelements zugeordnet werden. Die Nebenschneide kann integraler Bestandteil eines Endreflektor-Trägerelements sein oder kann mit diesem (vorzugsweise fest) verbunden sein. Der Nebenschneide kann z.B. entweder eine optische Fensterfläche mit einem hinter der optischen Fensterfläche angeordneten Reflektor (Endreflektor) oder nur ein Vorder-Endreflektor zugeordnet werden. Die umgekehrte Anordnung ist ebenfalls denkbar.

Weiter bevorzugt ist die zumindest eine Schneide aus einem für das Messlicht transparenten Material und/oder ist mit Komponenten aus transparenten Material starr verbunden (vorzugsweise auf kürzestem Weg und in optischem Kontakt). Für Messlicht im infraroten Bereich kann die zumindest eine Schneide z.B. aus Diamant sein. Vorteile dieses Materials sind die hohe Biokompatibilität, Schneidefähigkeit und Transparenz im MIR Bereich, in welchem wichtige spektrale Informationen über die Zusammensetzung organischer Proben gewonnen werden können. Ebenfalls ist mittels Diamant möglich, sehr dünne und gleichmäßige Probenschichten zu erzeugen. Eine aus Diamant gebildete Schneide kann folglich gleichzeitig die Funktionen Schneiden, Abkapseln und MIR-Transmission erfüllen.

Umfasst die Schneideeinrichtung zwei Schneiden, kann eine oder beide dieser Schneiden aus transparenten Material sein. Zumindest eine der Schneiden kann jedoch auch aus einem nicht transparenten Material sein.

Vorzugsweise sind sowohl die erste Schneide als auch das Fensterelement aus Diamant. Die erste Schneide und das optische Fensterelement können z.B. Bestandteile eines einzigen Elements sein, das sowohl eine Schneidefunktion als auch eine optische Funktion hat. Dieses Element kann z.B. ein Prisma (im Weiteren als Hauptschneiden-Prisma bezeichnet) sein, z.B. ein Prisma mit planen oder gekrümmten (z.B. sphärischen oder asphärischen) Flächen. Das Prisma weist die den Messspalt begrenzende optische Fensterfläche und eine Lichteintrittsfläche auf, die eine plane oder eine gekrümmte (z.B. eine sphärische oder asphärische) Fläche sein kann. Die Lichteintrittsfläche am Hauptschneiden-Prismas kann in Bezug auf der Achse des Einfallslichtbündels etwas geneigt sein, so dass Reflexionen an derselben durch eine Blende (Abschattblende) im Detektionsstrahlengang (weitestgehend) geblockt werden können.

Ferner kann die Schneideeinrichtung zumindest eine Hilfsschneide umfassen, die die Probenschicht an der schmalen Kante durchschneidet. Die freigeschnittene Probenschicht kann aus der Anordnung entfernt werden oder in einem Reservoir in der Anordnung zumindest temporär aufbewahrt werden.

Der Messspalt kann z.B. parallel oder keilförmig sein, mit einem Winkel von weniger als 1 Altgrad, vorzugsweise weniger als 0,1 Altgrad.

Bei einer Anordnung mit einem parallelen Messspalt kann ein unerwünschtes Spektrum auftreten, welches durch Mehrfachreflexionen im nahezu parallelen Messspalt hervorgerufen wird und das Messsignal überlagert (s.g. "Channeled Spektrum" oder "Standing Wave" Problem).

Das Problem des unerwünschten "Channeled Spektrums" kann durch eine nicht zu geringe Absorption im Gewebelappen aufgrund der gewählten Lappen-Dicke und des doppelten Strahlengangs, vermindert werden. Ferner kann der Effekt des unerwünschten Spektrums durch eine Antireflex-Schicht auf den den Messspalt begrenzenden Flächen verringert werden. Es ist ferner möglich, das überlagerte unerwünschte Spektrum aus den erfassten Messdaten herauszurechnen, z.B. durch numerisches Differenzieren der Spektraldaten. Aufgrund des kleinen Spaltes, was nur wenige Wellenlängen Optische Weglänge (OPD) für den Effekt des unerwünschten "Channeled Spektrums" bedeutet, bewirkt der Effekt des unerwünschten "Channeled Spektrums" ohnehin eher sehr langperiodische Modulationen über der Wellenzahl, die numerisch herausgerechnet werden können.

Ferner kann der Messspalt so angeordnet werden, dass der Einfallswinkel des Strahlenbündels an die den Messspalt begrenzenden Flächen unterschiedlich von 90° ist. In diesem Fall können die einzelnen Reflexionen nur bedingt zu einem unerwünschten "Channeled Spektrum" führen. Jedoch kann in diesem Fall ein - durch eine Zick-Zack-Reflexion an den Fensterflächen erzeugtes unerwünschtes - Teilbündel-Paar sich mit dem Messbündel kohärent überlagern, da es am Parallelspalt dann die gleiche Ausbreitungsrichtung aufweist.

Der Effekt des unerwünschten "Channeled Spektrums" kann weiter durch eine keilförmige Gestaltung des Messspalts verringert werden, da sich schon bei einer geringen Kippung des unerwünschten Teilbündels zum Messbündel der Effekt des unerwünschten "Channeled Spektrums" abschwächt. Der Keilwinkel kann so gewählt werden, dass die Keildickenänderung etwa eine halbe mittlere Wellenlänge auf die effektive Messlänge beträgt, um durch den Integrationseffekt über die Messfläche auf die erste Nullstelle der Interferenz-Modulation zu kommen. Bei wässrigem Medium genügen um die 3µm bis 4µm Keildicken-Änderung auf die Messlänge (Spalthöhe/Spaltlänge). Ein Nachteil der Anordnung mit einem keilförmigen Messspalt ist jedoch, dass wegen des keilförmigen Messspalts bzw. der keilförmigen Probenschicht die Absorption merklich abhängig vom Ort des Messspaltes ist, was zu einer Gewichtung des Messfeldes führt.

Die Breite des Messspalts beträgt vorzugsweise unter 200 µm , vorzugsweise unter 100 µm, weiter bevorzugt gleich oder unter 50 µm, besonders bevorzugt gleich oder unter 20 µm. Vorzugsweise ist die Breite des Messspalts variabel.

Insbesondere bei Messung von wässrigen Proben (wie z.B. Gewebe) in Infrarot, kann es aufgrund der extrem starken Wasserabsorption vorteilhaft sein, die Dicke der zu vermessenden Probenschicht gering zu halten. Vorzugsweise ist die Dicke der zur vermessenden Probenschicht ungefähr oder kleiner als λ_mittel/2 bis λ_mittel, wobei λ_mittel die mittlere Wellenlänge des Spektrums des Messlichts ist (z.B. gleich oder unter 10 µm bis 5 µm für eine mittlere Wellenlänge von z.B. 10 µm). Da es schwierig sein kann, eine so dünne Probenschicht abzuschneiden, kann es vorteilhaft sein, vor der Messung die ausgeschnittene Probenschicht zusätzlich auszudünnen und/oder zu dehydrieren. Der Wassergehalt der verdichteten bzw. ausgedünnten Probenschicht ist geringer, was i.d.R. zu einer Verbesserung des Signal-Rausch Verhältnisses, einer geringeren Messzeit und weniger erforderlichen Kalibrierungen führt.

Das Ausdünnen und/oder Dehydrieren der ausgeschnittenen Probenschicht kann z.B. durch eine Gestaltung des Messspalts derart, dass dessen Breite variabel ist, erzielt werden. So können beispielsweise das Fensterelement und das zweite, den Messspalt begrenzende Element (z.B. ein Endspiegel-Trägerelement) beweglich zueinander ausgebildet werden. Bei einem Messspalt, der schräg zur vertikalen Richtung bzw. zur Längsachse der Messsonde angeordnet ist, kann die Breite des Messspalts z.B. durch eine vertikale Bewegung eines oder beides der den Messspalt begrenzenden Elemente variiert werden. Vorzugsweise sind die beweglichen Elemente auf einem Mikro-Lager (z.B. auf einem aerostatischen Mikro-Lager) gelagert.

So kann in einer ersten Messposition der Messspalt breiter geöffnet werden, so dass die mittels Schneideeinrichtung ausgeschnittene Probenschicht (z.B. eine Probenschicht mit einer Dicke von 20 µm bis 30 µm) in dem Messspalt befördert werden kann. Nach Einführen der Probenschicht in den Messspalt kann in einer zweiten Messposition die Breite des Messspalts z.B. auf 5µm bis 10µm verringert werden. Die sich im Messspalt befindliche wässrige Probenschicht wird somit "ausgequetscht" und deren Wassergehalt verringert. Die Messung des Absorptionsspektrums kann in dieser Position durchgeführt werden. In einer anderen Ausführungsform kann die Breite des Messspalts zunächst minimal (z.B. ungefähr Null) sein. In diesem Zustand kann die Messsonde in den Tiefraum der zu vermessenden Probe (z.B. des Gewebes) eingebracht werden. Erst in der Probe (vor Ort an sinnvoller Stelle) kann sich der feine Messspalt öffnen, um eine Probenschicht abzuschneiden. Vorzugsweise erfolgt anschließend eine Verdichtung der Probenschicht durch eine Verringerung der Breite des Messspalts.

Eine Dehydrierung von frisch getrenntem Gewebe in der Messsonde kann ferner auch durch:
- Mikro-Vibration an der Messsonde, durch die das Wasser aus getrenntem Gewebe etwas "rausgeschüttelt" wird;
- Einsatz von Pulslasern und moderates Erwärmen des Gewebes;
- Einsatz eines Lasers bei einer Wellenlänge um 3 µm, da dort besonders hohe Wasserabsorption gegeben ist;
- Einsatz von Mikrowellen, wenn die Sonde metallfrei ausgebildet ist;
- Einsatz von kaltem, trockenem Spülgas (N2)
erzielt werden. Kombinationen von mehreren der obigen Methoden sind ebenfalls möglich.

Die Messsonde bzw. die Anordnung zur spektralen Absorptionsmessung kann folglich zumindest eine steuerbare Vorrichtung zur Pressung und/oder Verdichtung der Probe; und/oder zur Wasseranteilsverringerung umfassen. Ferner kann die Messsonde bzw. die Anordnung zur spektralen Absorptionsmessung eine steuerbare Vorrichtung zum Öffnen und Schließen des Messspalts bzw. des Messvolumens enthalten. Das gibt z.B. die Möglichkeit, zuerst tief mittels der Sonde zu stechen und erst beim Erreichen des Zielgebiets eine Miniklappe im Frontbereich der Sonde zu öffnen und wieder zu schließen. Die für das Ausführen der obigen Funktionen notwendige Energie kann z.B. durch Fluide (z.B. Gas oder Flüssigkeit) unter Druck, elektrisch (z.B. mittels Piezoeffekt), Wärme (z.B. Bimetall) oder in einer anderen Form zugeführt werden.

Bei ausreichend dünnen Probenschichten und ausreichend starken Messlicht kann eine Verdichtung und/oder Dehydrierung der Probe entfallen.

Die Messsonde kann eine längliche Form mit einer Längsachse aufweisen und der Messspalt kann in einem von Null unterschiedlichen Winkel zu der Längsachse der Messsonde, d.h. schräg zur Längsachse angeordnet sein. Der Winkel zwischen der Längsrichtung des Messspalts und der Längsachse der Anordnung kann z.B. von 3° bis 70°, bevorzugt von 10° bis 60°, weiter bevorzugt von 20° bis 50° betragen.

In einem Beispiel entspricht die Längsachse der Messsonde zumindest näherungsweise der Vorschubrichtung der Messsonde (d.h. die Längsachse der Messsonde ist im Wesentlichen parallel zu der Vorschubrichtung).

Die schräge Anordnung des Messspalts ermöglicht eine Stauchung bzw. Verdichtung der bereits abgetrennten Probenschicht (z.B. der Gewebeschicht) durch eine reine Vertikalbewegung in der Sonde und/oder der zweiten Schneide. Für Messungen im mittleren Infrarot kann die Dicke der gemessenen Probenschicht vorzugsweise auf eine Dicke zwischen 1 µm und 30 µm, typischerweise zwischen ungefähr 4 µm bis 12 µm reduziert werden. Insbesondere bei wässrigen Proben (z.B. Gewebeproben) kann somit eine Reduktion der Verluste durch die Wasser-Absorption im MIR erreicht werden. Ferner kann durch eine schräge Anordnung des Messspalts der Effekt des unerwünschten "Channeled Spektrums" reduziert werden. Ein weiterer Vorteil einer schrägen Anordnung des Messspalts ist, dass eine seitliche Entsorgung der gemessenen Probenschicht erleichtert werden kann. Dies ermöglicht einen kompakteren Aufbau der Anordnung zur Absorptionsmessung.

Der Messspalt kann jedoch auch im Wesentlichen parallel zu der Längsachse angeordnet sein, d.h. der Winkel zwischen der Längsrichtung des Messspalts und der Längsachse der Messsonde ist vorzugsweise im Wesentlichen 0°. Ein Vorteil dieser Anordnung kann die Miniaturisierbarkeit der Sonde sein. Um direkte Reflexionen an Grenzflächen zu vermeiden, kann als Endreflektor ein diffraktives optisches Element eingesetzt werden, wie nachfolgend im Detail beschrieben wird.

Die Messsonde umfasst ferner einen Endreflektor, der auf einem Endreflektor-Trägerelement angeordnet ist. Das Endreflektor-Trägerelement kann mit einer der Schneiden der Schneideeinrichtung fest verbunden sein, wie oben beschrieben.

Der Endreflektor kann auf einer der den Messspalt begrenzenden Flächen oder in der Nähe einer der den Messspalt begrenzenden Fläche angeordnet sein (Vorder-Endspiegel). In diesem Beispiel wird der Messspalt zumindest teilweise von der Fensterfläche und dem Endreflektor begrenzt. Das Endreflektor-Trägerelement kann, muss jedoch nicht transparent für das Messlicht sein. So kann das Endreflektor-Trägerelement aus einem geeigneten harten Material sein, wie z.B. Metall, Keramik, Diamant, etc.

Der Endreflektor kann jedoch auf eine andere Fläche eines im Wesentlichen transparenten Endreflektor-Trägerelements angeordnet sein (so genannten Hinter-Endreflektor bzw. Endreflektor "im Hintergrund"). Der Hinter-Endreflektor bzw. Endreflektor "im Hintergrund" weist vorzugsweise eine Neigung zur planen Fensterfläche des Fensterelements auf. Damit wird ein unerwünschtes registrierbares "Channelled Spectrum" vermieden oder in seiner das Messergebnis verfälschenden Wirkung abgeschwächt.

Der Endreflektor kann beispielsweise als ein Spiegel, insbesondere ein Planspielgel, z.B. ein Spiegel aus Gold, etc. sein. In diesem Fall ist der Endreflektor vorzugsweise als Hinterspiegel bzw. Hinter-Endreflektor realisiert. Der Endreflektor, der vorzugsweise als Hinter-Endreflektor ausgebildet ist, kann als ein Dachkantspiegelprisma, z.B. aus Diamant oder ZnSe, ausgebildet sein.

In einem anderen Beispiel kann der Endreflektor, der z.B. als ein Vorder- oder ein Hinter-Endreflektor ausgebildet ist, ein diffraktives optisches Element (DOE) sein oder ein solches Element umfassen. Das diffraktive optische Element kann ein Reflexions-Beugungsgitter (z.B. ein mikroprofiliertes, geblaztes Reflexions-Beugungsgitter) sein oder ein Reflexions-Beugungsgitter umfassen. Der Endreflektor kann in diesem Beispiel sowohl als Vorder- als auch als Hinterspiegel ausgebildet sein. Das Mikroprofil kann z.B. eine Treppen-, Sägezahn-, Dachkant-, Prismenform oder eine andere geeignete Form aufweisen. Der Endreflektor kann z.B. eine Mikro-Cats-Eye-Struktur in Tripel oder Dachkantausbildung in der Reflektoroberfläche aufweisen. Dies ermöglicht ein volumenminimiertes Design der Messsonde. Die tiefenprofilierte Mikrostruktur kann z.B. in Massiv-Metall oder in einer hinreichend dicken Metallschicht auf dem Endreflektor-Trägerelement geformt werden.

Bei einem diffraktiven Endreflektor als Vorder-Spiegel bzw. Vorder-Reflektor ist das DOE vorzugsweise als ein vergrabenes DOE ausgelegt. Die Probenschichten können zwischen die glatten Wände des Messspalts gleiten, so dass Messungen auch an dünnsten Probenschichten möglich sind. Vorzugsweise gibt es im Bereich der Fensterflächen bzw. des optischen Fensters des Messspalts keine Mikro-Kanten vom DOE. Außerhalb des optischen Fensters (z.B. an einer der Schneiden und/oder oberhalb des Messkanals) kann ein Mikroprofil (Grip) angeordnet sein, damit die Probenschicht besser, z.B. durch Mikro-Vibration "nach oben" in einem Abführkanal transportiert werden kann.

Der Endreflektor kann im Wesentlichen plan oder schwach gekrümmt sein. Die Normale des Endreflektors kann zumindest näherungsweise parallel zur Normale der Fensterfläche oder des Flächenbereichs im Zentrum der Fensterfläche sein. Der Endreflektor kann jedoch eine Neigung zur Fensterfläche aufweisen, so dass die Normale des Endreflektors nicht parallel zu der Fensterfläche oder des Flächenbereichs im Zentrum der Fensterfläche ist. Dies hat den Vorteil, dass ein unerwünschtes registrierbares Spektrum (das so genannte "Channeled Spektrum") vermieden oder in seiner das Messergebnis verfälschender Wirkung abgeschwächt wird.

In einem Beispiel kann der Endreflektor zumindest näherungsweise zylindrisch gekrümmt sein. Vorzugsweise koinzidiert die Achse der zylinderförmigen Ausgleichsfläche des Endreflektors zumindest näherungsweise mit der Längsachse der Messsonde. Ferner kann der Endreflektor ein mikroprofiliertes DOE sein oder ein solches Element umfassen, das vorzugsweise eine Zylinder-Phasenfunktion (z.B. als Mikroprofil) aufweist. Ein Vorteil dieser Anordnung besteht in der eindimensionalen Fokussierung, wodurch eine längliche Fläche der Probe durchstrahlt werden kann.

Vorzugsweise ist der Endreflektor ein Retroreflektor. So ist es möglich, den gleichen optischen Kanal sowohl für den Transport der Messstrahlung zum Messspalt bzw. für die Beleuchtung der Probe als auch für die Detektion zu benutzen. Die Messsonde kann somit mit einer geringeren Anzahl von optischen Elementen gebaut werden, was eine Miniaturisierung der Sonde ermöglicht.

Der diffraktive Endreflektor kann für die erste Beugungsordnung ausgelegt sein. Es ist jedoch möglich, den diffraktiven Endreflektor für eine andere Beugungsordnung auszulegen. Vorzugsweise ist das diffraktive optische Element derart ausgelegt, den Strahlungstransport zur Detektion bzw. in einem Detektionskanal zu optimieren.

Dabei ist der Einfallswinkel des Schwerstrahls des auftreffenden Bündels in einem im wesentlichen nichtstreuendem Medium (wie Wasser, Gewebesekret, etc.) auf die Ausgleichsfläche des diffraktiven Reflektors größer oder gleich als 15° und der Winkel zwischen einfallendem Schwerstrahl des auftreffenden Bündels und Schwerstrahl des weggehendem Bündels auf die o. g. Ausgleichsfläche kleiner als der doppelte Einfallswinkel des Schwerstrahls des auftreffenden Bündels auf die Reflektorfläche. Das kann z.B. durch eine geeignete Gestaltung eines diffraktiven optischen Reflektors mit einer geblazten Mikrostruktur erreicht werden.

Für die in die Messsonde eintretende elektromagnetische Strahlung mit einem breiten Spektrum soll vorzugsweise der möglichst vollständige Wiedereintritt in einen Detektionskanal oder in einen gemeinsamen Beleuchtungs- und Detektionskanal gewährleistet werden. Nicht kompensierte chromatische Quer-Aberrationen können dies jedoch behindern oder sogar verhindern. Eine erste Möglichkeit ist es, zum Transport des Messlichts eine Faser mit ausreichend großem Faserkern zu verwenden. Dies hat den Nachteil, dass die Anordnung zur Absorptionsmessung vergrößert wird.

Eine andere Möglichkeit ist es, die Chromasie der Anordnung soweit einzugrenzen, dass es zu keinen größeren Verlusten infolge chromatischer Quer-Aberrationen kommt. Vorzugsweise ist die optische Anordnung zumindest teilweise achromatisch bzw. achromatisiert. Um dies zu erreichen, kann die Messsonde beispielsweise mindestens ein Kompensations-Beugungsgitter umfassen, das ausgelegt ist, das optische System der Messsonde, vorzugsweise bis zur Detektion, zumindest näherungsweise zu achromatisieren. Dadurch kann eine bessere Effizienz für die Messstrahlung erzielt werden. Des Weiteren kann eine Unterdrückung von Störlicht (Reflexion) im Detektionskanal erzielt werden, da das Kompensationsgitter so ausgelegt werden kann, dass ein möglichst geringerer Anteil des unerwünschten Störlichts in dem Detektionskanal eingekoppelt wird.

Das Kompensations-Beugungsgitter kann insbesondere ausgelegt sein, die durch die Verwendung eines diffraktiven Gitters als Endreflektor bedingten chromatischen Aberrationen zumindest teilweise zu kompensieren. Das Kompensations-Beugungsgitter kann zusätzlich oder alternativ ausgelegt sein, andere chromatische Fehler der optischen Elemente der Anordnung zur spektralen Absorptionsmessung zumindest teilweise zu kompensieren.

Das Kompensations-Beugungsgitter kann z.B. ein Reflexionsbeugungsgitter, ein Transmissionsbeugungsgitter oder ein anderes geeignetes diffraktives optisches Element sein. In einem Beispiel ist das Kompensations-Beugungsgitter auf einer Fläche des optischen Fensterelements angeordnet. Das diffraktive optische Element kann zum Beispiel auf der Lichteintrittsfläche des optischen Fensterelements angeordnet sein. Das diffraktive optische Element kann auf einem zusätzlichen optischen Element angeordnet sein, wie z.B. auf einem Prisma, einer Linse, etc. Werden insbesondere optische Elemente aus Diamant verwendet, kann es vorteilhaft sein, das Kompensationsbeugungsgitter auf einem zusätzlichen optischen Element aus ZnSe zu formen, da Diamant schwer zu mikroprofilieren ist. Das zusätzliche optische Element aus ZnSe kann, muss jedoch nicht fest mit dem optischen Element aus Diamant verbunden sein.

Vorzugsweise ist das optische System der Messsonde bzw. der Anordnung zur spektralen Absorptionsmessung derart ausgelegt, dass der Lichteinfall des Messlichts auf die refraktiven optischen Flächen der Anordnung nicht senkrecht ist. Anders ausgedrückt können die optischen Komponente der Messsonde bzw. der Anordnung derart ausgelegt und angeordnet sein, dass der Lichteinfallswinkel auf die refraktiven Flächen der Messsonde bzw. der Anordnung unterschiedlich von 0° ist. Vorzugsweise sind die Einfallswinkel auf die refraktiven Flächen relativ klein und nur gerade so groß, dass eine direkte Reflexion in dem Detektionskanal verhindert wird. Vorzugsweise ist der Einfallswinkel von 10°, besonders bevorzugt von 5°.

Ist der Einfallswinkel 0° können unerwünschte Reflexionen wie oben beschrieben durch die Verwendung diffraktiver optischer Elemente minimiert werden. Alternativ oder zusätzlich können die refraktiven Flächen entspiegelt werden.

Eine andere Möglichkeit, unerwünschte Reflexionen im Detektionskanal zu vermeiden, ist es, mehrere Detektionskanäle einzusetzen. So können bei einer Lichtquelle mit drei MIR-Lasern mit unterschiedlichen Wellenzahlen bzw. Wellenlängen (z.B. 1084cm⁻¹, 1235cm⁻¹, 1650cm⁻¹) drei Detektionskanäle, z.B. drei Detektionsfaser, verwendet werden. Das baut jedoch eher groß und ist somit nicht patientenschonend. Ebenfalls ist es möglich, diffraktive Elemente in der Messsonde wegen der notwendigen Strahlablenkung zu verwenden und die daraus resultierende spektrale Abspaltung in Kombination mit unterschiedlichen Detektionskanälen zu verwenden.

Ferner sind das Fensterelement und der Endspiegel vorzugsweise so ausgelegt und angeordnet, dass der durch die Fensterfläche des Fensterelements austretende Schwerstrahl des Messstrahlenbündels zumindest näherungsweise zum Zentrum der Fensterfläche geneigt ist, wobei der Neigungswinkel vorzugsweise größer als der Randstrahlenwinkel des Messstrahlenbündels ist. Mit dieser Anordnung ist es möglich, dass so gut wie kein oder wenig Licht zurück in den Beleuchtungs- und/oder Detektionskanal (beispielsweise in den Faserkern eines Beleuchtungs- und/oder Detektionsfaser) reflektiert wird. Dies minimiert das Verfälschen des Messsignals durch unerwünschtes Störlicht.

Des Weiteren ist der Mess-Strahlenbündel vorzugsweise im Wesentlichen parallel bzw. kollimiert. Der Mess-Strahlenbündel kann z.B. durch zumindest ein optisches Element der Messsonde parallelisiert werden. Es ist jedoch möglich, auch fokussierte Strahlenbündel einzusetzen.

Die Messsonde kann ferner einen Abtransportkanal zum Abtransportieren der sich im Messspalt befindlichen Probenschicht vom Messspalt umfassen. Ebenfalls kann die Messsonde ein Reservoir zur Aufnahme und/oder Zwischenlagerung der vom Messspalt abtransportierten Probenschicht umfassen. Ein Vorteil der erfindgungsgemäßen Messsonde mit dem Endreflektor ist es, dass es nicht notwendig ist, den Abtransportkanal zwischen zwei Fasern anzuordnen, was eine Minuaturisierung der Probe sehr erschweren würde.

Die freigeschnittene und vermessene Probenschicht kann vom Messspalt abtransportiert werden. Dies kann z.B. durch Vibration, durch eine geeignete Saugvorrichtung, etc. erfolgen. Die abtransportierte Probenschicht kann in einem Reservoir (vorzugsweise in einem Mikro-Reservoir) permanent oder temporär (z.B. für die unmittelbare Zeit der Messung) gelagert werden. Die gelagerte(n) Probenschicht(en) bzw. Probeschnitte(n) können in situ abgesaugt werden oder durch andere Mittel extrahiert oder entpackt werden. Bei Bedarf können die Probeschnitte einer weiteren histopathologischen Untersuchung zugeführt werden. Die durch diese Untersuchung gewonnenen Daten können mit den spektralen Daten der in-situ Sonden-Messung verglichen bzw. gegenübergestellt werden. Die Ergebnisse können in einem Protokoll (z.B. in einem OP Protokoll) dokumentiert werden.

In einem Beispiel kann die 3D-Position der Messsonde detektiert und beispielsweise in Echtzeit mitverfolgt werden. Zu diesem Zweck kann die Anordnung zur spektralen Absorptionsmessung eine Vorrichtung zur Erfassung der 3D Position der Messsonde umfassen. Die Vorrichtung zur Erfassung der 3D Position kann eine Ultraschall-Bildgebungsvorrichtung, eine CT (Computer Tomographie)-Vorrichtung oder eine andere geeignete Vorrichtung sein. Die 3D-Position der Messsonde kann in einem zentralen Rechensystem eingespeist werden.

Gemäß einem weiteren Aspekt der Erfindung wird ein Verfahren zur spektralen Absorptionsmessung einer Probe mittels der Messsonde oder der Anordnung zur spektralen Absorptionsmessung nach einem Aspekt der Erfindung vorgeschlagen. Das Verfahren umfasst:
Aufnehmen einer Probenschicht in dem Messspalt der Messsonde;
Durchführen zumindest einer spektralen Messung der sich im Messspalt befindlichen Probenschicht, umfassend
   Einkoppeln von Messlicht in den Messspalt durch das Fensterelement und Durchstrahlen der sich im Messspalt befindlichen Probenschicht;
   Reflektieren des einmal durch die Probenschicht propagierten Messlichts an dem Endreflektor zurück in den Messspalt und erneutes Durchstrahlen der sich im Messspalt befindlichen Probenschicht;
   Auskoppeln des zweifach durch die sich im Messspalt befindliche Probenschicht propagierten Messlichts aus dem Messspalt durch das Fensterelement;
   Detektieren zumindest eines Teils des ausgekoppelten Messlichts.

Vorzugsweise wird die spektrale Messung mehrfach wiederholt, wobei jede neue Messung an einer neuen räumlichen Position in der Probe erfolgt, z.B. in einer neuen Tiefe und/oder an einer neuen lateralen Position. Vorzugsweise wird das ermittelte Spektrum der jeweiligen Tiefen- und/oder lateralen Position der Messsonde in der zur untersuchenden Probe beim Messen zugeordnet und so die spektrale Verteilung ortsaufgelöst zur Verfügung gestellt. Vorzugsweise wird die Tiefen- und/oder laterale Position der Messsonde in der Probe permanent oder in kurzen Abständen wiederholt gemessen und z.B. in einem Speicher gespeichert. Die Messsonde kann dabei eine vor- und zurück-Schraubenlinien-Bewegung durchführen und dabei Gewebeproben dünn abtrennen und der spektroskopischen Untersuchung zuführen.

Insbesondere kann mit dem Eindringen der Messsonde in einen zunehmend tieferen Bereich der Sondenspitze jeweils eine Absorptionsmessung erfolgen, so dass eine Tiefenabtastung (mit jeweiligem Absorptionsspektrum) vor Ort, in frei wählbaren Mess-Abständen und frei wählbarer Messpunktdichte gewonnen werden kann. Ebenfalls ist es möglich, einen lateralen Scan durchzuführen. In einem Beispiel kann eine Multi-Punkt Aufnahme (z.B. 5x5 Pixel a 100 µm Durchmesser) durch einen lateralen Scan (z.B. durch einen lateralen Mikro-Scan einer Monomode-Faser am Eingang der Messsonde) durchgeführt werden. Anhand der einzelnen Aufnahmen kann ein Mittelwert bestimmt werden. Dies reduziert die Wahrscheinlichkeit, per Zufall ein "Loch" in der Probe (z.B. ein "Gewebe-Loch") getroffen zu haben. In diesem Fall wird vorzugsweise eine Messsonde mit einer numerischen Apertur Na von 0,05 bis 0,1 eingesetzt.

Das Verfahren zur spektralen Absorptiönsmessung kann ferner eine zwei- oder eine dreidimensionale Messung der Form der zu vermessenden Probe umfassen. So kann das Messvolumen, dessen Ort in der zu untersuchenden Probe (z.B. ein Organ, ein System oder eine Komponente) und die Spektralverteilungen der jeweiligen Absorptionsmessung fehlerarm einander zugeordnet werden. Daraus kann rechnerisch oder unter Einsatz von humanem Fachwissen eine Bewertung des Zustandes der gemessenen Probe erfolgen. Bei einer chirurgischen OP können somit z.B. optimale Schnittlinien ermittelt werden.

Das Verfahren kann ferner eine spektrale Analyse des detektierten Messlichts und optional eine Klassifizierung der untersuchten Probenschicht in einer von mehreren Kategorien umfassen.

Ebenfalls kann das Verfahren zur spektralen Absorptionsmessung ein Abschneiden einer Probenschicht mit zumindest näherungsweise konstanter Dicke aus der Probe umfassen, z.B. mittels der oben beschriebenen Schneidevorrichtung. Vorzugsweise erfolgt das Abschneiden durch mechanisches Trennen mittels zumindest einer scharfen Schneide.

Die zumindest eine Schneide kann, wie oben beschrieben, eine Auf- und Ab-Schwingbewegung durchführen. Das kann das Erzeugen einer sehr dünnen Probenschicht sehr begünstigen. Die Schwingbewegung kann eine kleine Schwingamplitude hoher Frequenz aufweisen, z.B. 0,5 µm Schwingamplitude und 2 kHz Frequenz. In einem Beispiel kann die zumindest eine Schneide im Ultraschall-Bereich schwingen bzw. vibrieren.

In einem Beispiel wird jede der Begrenzungsflächen der Probenschicht durch eine eigene Schneide mechanisch abgetrennt. Wie oben beschrieben kann eine der beiden Schneiden die Hauptschneide und die andere Schneide die Nebenschneide sein. Das Abschneiden einer Probenschicht kann in diesem Fall ein Einführen der Hauptschneide in die Probe gefolgt von einem Einführen der Nebenschneide umfassen. Dabei kann die Nebenschneide eine Schwingbewegung relativ zur Hauptschneide durchführen.

In einem Beispiel entspricht der Vorschub eines Bewegungsvorganges der zumindest einer beweglichen Schneide mindestens der halben Länge des lateralen Abtastbereichs beim Messen. Dabei bestimmt die Länge des Abtastbereichs die örtliche Auflösung der Messsonde.

Nach dem Auskoppeln aus dem Messspalt kann das Messlicht zumindest näherungsweise in sich selbst zurückgehen (Retroreflexion) bzw. in sich selbst zurückgeführt werden, um anschließend in einem Detektionskanal eingekoppelt zu werden. Das Messlicht kann jedoch in einem vom Eingangsbündel-Strahlengang separierten Detektionskanal eingekoppelt werden.

Das Verfahren zur Absorptionsmessung kann ferner ein Verändern der Breite des Messspalts der Messsonde umfassen. Insbesondere kann das Verfahren ein Öffnen und Schließen des Messspalts (der z.B. in Form eines Mikro-Greifers an der Sondenspizte ausgebildet ist), gegebenenfalls nur an einem vorab definierten Zielort, umfassen. Somit können unnötige Verletzungen der untersuchten Probe verhindert werden. Des Weiteren kann eine Veränderung der Breite des Messspalts zu einer Verdichtung oder Dehydrierung der in den Messspalt aufgenommenen Probenschicht führen.

Das Verfahren zur spektralen Absorptionsmessung kann dementsprechend eine Verdichtung oder Dehydrierung der in den Messspalt aufgenommenen Probenschicht umfassen. Wie oben beschrieben erfolgt die Verdichtung oder Dehydrierung der sich in den Messspalt aufgenommenen Probenschicht vor der eigentlichen Messung. Der Schritt des Verdichtens oder Dehydrierens der Probenschicht kann jedoch entfallen, z.B. wenn ausreichend dünne Probenschichten und/oder ausreichend starke Lichtquellen eingesetzt werden. Auch bei einer Verwendung der Messsonde in NIR Bereich für RAMAN-Spektroskopie kann der Schritt des Verdichtens oder Dehydrierens der Probenschicht entfallen, da für die RAMAN-Spektroskopie die oben beschriebene Wasserbarriere nicht besteht.

Ein Vorteil der Messsonde gemäß einem Aspekt der Erfindung ist die Möglichkeit, diese zu miniaturisieren, insbesondere deren laterale Ausdehnung (Querausdehnung, Dicke, Durchmesser). Bei Anwendungen in der Medizin kann die Messsonde somit patientenschonender gestaltet werden. Ferner ist es möglich, eine örtlich viel feinere Durchmusterung der Probe (z.B. eines lebenden Organs) durchzuführen. Dies ermöglicht einerseits das weitgehende Erhalten der Funktionsfähigkeit der Probe und liefert andererseits zuverlässige und rückverfolgbare Daten über den Zustand und/oder Beschaffenheit der Probe. Mit der erfindungsgemäßen Messsonde ist es ferner möglich, dichter (im Vergleich zum Stand der Technik) an der Spitze der Messsonde zu messen und somit auch Stellen im Tiefraum des Gewebes zu vermessen, ohne das Gewebe unnötig zu verletzen.

Weitere Vorteile können die kurze Messzeit und/oder die hohe Aussagekraft der Messdaten bzw. die geringe Messunsicherheit bei den Absorptionsbanden sein, da insbesondere der Anpressdruck keinen Einfluss auf die Messergebnisse hat und Mess-Artefakte (z.B. durch Lichtstreuung oder Standing-Waves) in der optischen Primär-Signalen sehr klein gehalten werden können. Ebenfalls kann eine bessere laterale Auflösung erreicht werden.

Ferner ist es nicht notwendig, die untersuchte Probe (z.B. das untersuchte Gewebe) zu fixieren (z.B. durch Paraffinierung oder Anfrieren). Die Probe kann in ihrem weitgehend originalen Zustand (z.B. im originalen hydrierten oder leicht dehydriertem Zustand), unmittelbar (d.h. in wenigen Sekunden oder Minuten) nach dem Schneiden vermessen werden, was die Aussagekraft der Messungen erhöht.

Die Messsonde, die Anordnung und/oder das Verfahren zur spektralen Absorptionsmessung können in-vivo bei chirurgischen Operationen an Weichteilgewebe oder für das Analysieren von Weichteilen oder technischen Weichwerkstoffe eingesetzt werden. Ebenfalls ist es möglich, auch härteres Gewebe (z.B. Knorpel) in-vivo zu messen, was beim Stand der Technik aufgrund des zu hohen Anpressdrucks sehr schwierig oder sogar ausgeschlossen ist.

Die Messsonde, die Anordnung und/oder das Verfahren zur spektralen Absorptionsmessung können in unterschiedliche medizinische und veterinär-medizinische Anwendungen eingesetzt werden.

So können die Messsonde, die Anordnung und/oder das Verfahren zur spektralen Absorptionsmessung zu einer minimal invasiven Bestimmung von Absorptionsspektren an Weich- oder Knorpelgewebe (z.B. während einer chirurgischen Operation, vorzugsweise in Echtzeit oder Quasi-Echtzeit) eingesetzt werden, insbesondere zu einer Bestimmung von Molekül-Absorptionsspektren im mittleren Infrarot-Spektralbereich, da dort nach heutigen Wissen die mit Abstand beste Signifikanz und Aussagekraft der Spektren (Molekül-Vibrationen) gegeben sind.

Anhand der ermittelten Absorptionsspektren kann (z.B. während einer chirurgischen OP) eine schnelle und zuverlässige Entscheidung über das Vorhandensein eines Tumors und/oder die Phase der Tumor-Entwicklung (z.B. premalign, malign, benign, Zwischenzustände) getroffen werden. Ferner können Schnittlinien, entlang welchen das Gewebe herausgeschnitten werden soll, mit einer hohen Genauigkeit und Präzision, vorzugsweise automatisch ermittelt werden und dem Chirurgen zur Verfügung gestellt werden.

Außerhalb einer chirurgischen Operation können die Messsonde, die Anordnung und/oder das Verfahren zur spektralen Absorptionsmessung für in-vivo Messungen an Weichteilgewebe, Knorpelgewebe und/oder Körperflüssigkeiten (wie z.B. Sekrete, Urin, etc.) in der Präventiv-Medizin und/oder bei Verdachtsmomente eingesetzt werden. Anhand der spektralen Daten kann z.B. eine Gewebeanalyse, einschließlich eine Stoffwechsel-Analyse durchgeführt werden.

Die Messsonde, die Anordnung und/oder das Verfahren zur spektralen Absorptionsmessung können ebenfalls in unterschiedliche technische Anwendungen eingesetzt werden. So ist es z.B. möglich, bei geringer Werkstoffzerstörung Absorptionsspektren an Weichwerkstoffen, vorzugsweise in Betriebs-Echtzeit, zu ermitteln. Technische Anwendungsgebiete umfassen:
die Veterinärmedizin und die Tieraufzucht;
die Materialwissenschaften;
die Lebensmittel-Kontrolle, z.B. bei Ein- und Ausfuhrkontrolle oder bei der Lebensmittel-Produktions-Überwachung;
die Forensik und die Kriminalistik;
die automatisierte Probenanalyse in der Biotechnologie, Medizin und chemischen Produktion.

Weiteren Aufgaben, Merkmale und Vorteile der vorliegenden Erfindung werden offensichtlich aus einer detaillierten Beschreibung einer bevorzugten Ausführungsform der vorliegenden Erfindung mit Bezug auf die Zeichnungen, welche zeigen:
Fig. 1 das Spektrum von typischen Biomolekülen
Fig. 2 die Absorptionsspektren typischer Biomoleküle
Fig. 3 bis 19 beispielhafte Messsonden und Anordnungen zur Absorptionsmessung umfassend die Messsonden;
Fig. 20 und 21 beispielhafte Anordnungen zur Absorptionsmessung;
Fig. 22 eine beispielhafte Lichtquelle mit adressierbarem Spektrum;
Fig. 23 bis 25 beispielhafte Anordnungen zur Absorptionsmessung.

In den Figuren werden die gleichen Bezugszeichen für die gleichen oder ähnlichen Elemente verwendet. Zum besseren Verständnis sind die Figuren nicht maßstabgetreu. Insbesondere sind diffraktive Strukturen stark vergrößert und nicht maßstabgetreu dargestellt.

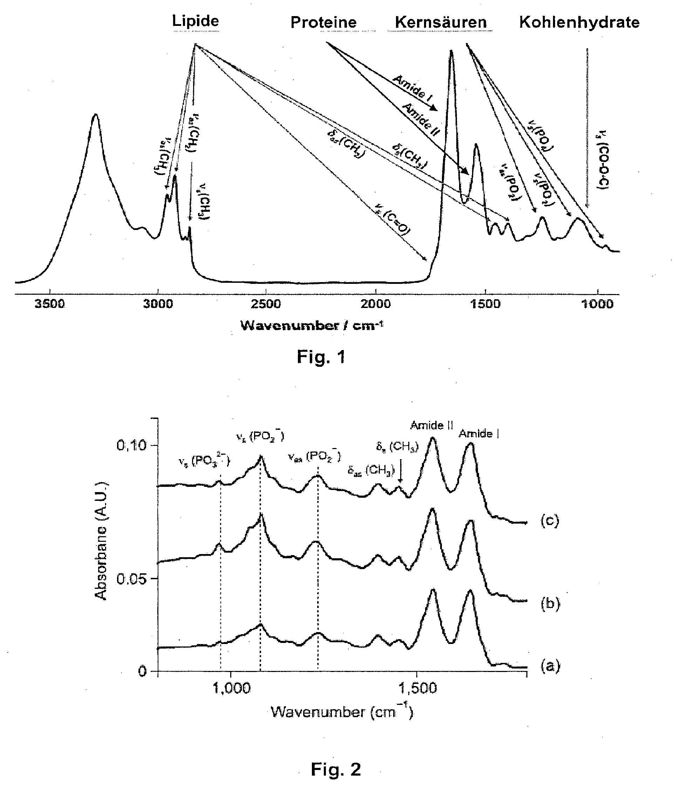

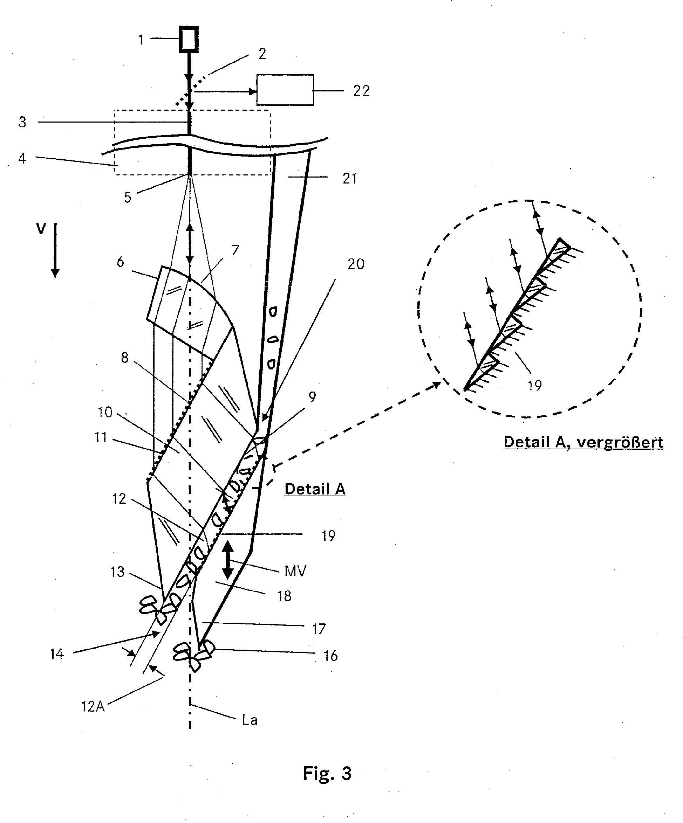

Fig. 1 und 2 zeigen Spektren (Absorptionsspektren) typischer Biomoleküle. Fig. 1 zeigt Spektren typischer Biomoleküle, die Bestandteile von lebenden Zellen sind (Ref. Hughes, Caryn [Thesis], Development of Fourier Transform Infrared spectroscopy for Drug Response Analysis, Fig. 3.6, Seite 83, University of Manchester, 2011). Fig. 2 zeigt die im Fachartikel "Infrared spectroscopy characterization of normal and lung cancer cells..." von So Yeong Lee et al., in J. Vet. Sci (2009) S. 299-304 (DOI: 10.4142/jvs.2009.10.4.299 beschriebene Spektren von normalen und von Krebszellen der Lunge. Die normalen und von Krebs befallenen Zellen der Lunge stellen hier nur ein Beispiel dar.

In einer Vorabuntersuchung können Zellen aus verschiedenen Organbereichen und Umgebungen eines z.B. zu operierenden Organs eines Patienten entnommen und vom Histopathologen spektroskopisch untersucht und klassifiziert werden. Diese Zellen und die zugehörigen Spektren dienen somit als Referenzzellen bzw. Referenz-Spektren. Die Spektren können durch außerordentlich viele Umstände erheblich moduliert sein, so dass eine zeitnahe Untersuchung von Referenzzellen/Referenzgewebe vom Patienten wünschenswert ist. Anhand von Absorptionsspektren, die z.B. währen einer chirurgischen Operation gewonnen werden und anhand der Referenz-Spektren können Rückschlüsse auf die Zusammensetzung der untersuchten Probe (z.B. Gewebe) gezogen werden. Insbesondere kann das untersuchte Gewebe in Krebs- bzw. kein Krebs klassifiziert werden.

Fig. 3 zeigt eine Messsonde gemäß einem ersten Beispiel und eine beispielhafte Anordnung zur spektralen Absorptionsmessung umfassend die Messsonde. Die Messsonde weist eine längliche Form auf (z.B. eine Nadel- oder Katheterform) mit einer Längsachse La. Die Längsachse La entspricht zumindest näherungsweise der Vorschubrichtung der Messsonde.

Die Anordnung zur spektralen Absorptionsmessung umfasst eine Lichtquelle 1. Die Lichtquelle 1 kann eine brillante breitbandige Lichtquelle mit einem kontinuierlichen oder diskreten Spektrum, eine durchstimmbare Lichtquelle, eine Lichtquelle mit adressierbaren Spektrum oder eine andere geeignete Lichtquelle sein oder umfassen. Ferner kann die Lichtquelle einen Filter und/oder einen Lichtmodulator umfassen. Vorzugsweise wird die Intensität der Lichtquelle so gewählt, dass zuverlässige Messergebnisse geliefert werden können, ohne die untersuchte Probe (in diesem Beispiel das Gewebe 16) während des Messvorgangs zu zerstören. Die Lichtquelle 1 kann Licht im MIR Bereich emittieren, z.B. von ungefähr 6 µm bis ungefähr 11 µm. Andere Spektralbereiche sind ebenfalls möglich.

Das von der Lichtquelle ausgestrahlte Licht wird in einer Faser 4 mit einem Faserkern 3 und einem Faserkernende 5 eingekoppelt und zur Messsonde geführt. Für das mittlere Infrarot können spezielle Fasern eingesetzt werden, z.B. Silberhalide-Fasern. Der Außendiameter der Faser kann beispielsweise zwischen 0,5 mm und 1 mm sein, vorzugsweise 0,9 mm oder kleiner. Der Kern der Faser kann ungefähr 0,1 mm sein. Die Faser 4 kann eine monomode oder eine nicht monomode Faser sein. Die Verwendung einer nicht monomoden Faser kann vorteilhaft sein, um parasitäre Interferenzen zu unterdrücken.

Bei dem in Fig. 3 gezeigten Beispiel ist die Faser 4 sowohl Teil des Beleuchtungs- als auch Teil des Detektionskanals, so dass sowohl das Beleuchtungslicht als auch das zu detektierende Messlicht nach der Messung durch die Faser 4 propagiert. Die Ein-Faser-Variante hat den Vorteil, dass diese am dünnsten (wie eine Stricknadel) baut, und folglich minimal invasiv ist. Ein Nachteil dieser Lösung kann die Reflexions-Problematik an den Übergangsflächen (d.h. von einem optisch dünnen zu einem optisch dichten Material und umgekehrt) sein. Bei einer Anwendung zur Messung der Absorptionsspektren wässriger Proben, wie z.B. Gewebeproben, ist die Schwächung des eingestrahlten Lichts wegen des dünnen Wasserfilms im frisch geschnittenen Gewebe sehr stark, so dass ein unerwünschter Frontreflexion 100-Fach stärker als das auszuwertende Restlicht, das nach einer Reflexion aus dem Gewebe kommt, sein kann. Vorzugsweise ist das optische System der Messsonde derart ausgelegt, dass kein oder nur ein geringer Anteil des direkt reflektierten Lichts, das nicht durch die Probenschicht propagiert hat, in die Faser 4 zurückgeht.

Eine Möglichkeit die unerwünschten Direktreflexionen zu minimieren, ist es, die refraktiven Flächen zu entspiegeln. Dies könnte jedoch insbesondere bei optischen Elementen aus Diamant problematisch sein, da das Entspiegeln von Diamant sehr schwierig ist oder nur mit radioaktiven, giftigen Substanzen machbar ist, was bei Anwendungen der Messsonde in der Medizin ausscheidet. Eine andere Möglichkeit ist es, die refraktiven Flächen hinreichend geneigt in Bezug auf das einfallende Licht anzuordnen, so dass direkte Reflexionen nicht zurück in die Faser 4 bzw. in den Detektionskanal gehen. Zusätzlich oder alternativ ist auch der Einsatz von diffraktiven optischen Elementen (DOE) möglich.

Die Messsonde umfasst eine Linse 6, z.B. eine asphärische Linse 6 mit einer Freiformfläche 7. Die asphärische Linse 6 kann z.B. aus ZnSe oder aus einem anderen geeigneten transparenten Material sein. Bei dem in Fig. 3 gezeigten Beispiel ist die Freiformfläche 7 die Lichteintrittsfläche (Eintrittsfläche) der asphärischen Linse 6. Die Freiformfläche kann jedoch eine andere Fläche sein, z.B. die Lichtaustrittsfläche (Austrittsfläche) der asphärischen Linse 6. Das Strahlenbündel, das die Linse 6 verlässt und auf die Eintrittsfläche 8 des Fensterelements 10 einfällt, ist vorzugsweise im Wesentlichen parallel und weist einen Durchmesser von beispielsweise 0,8 mm. Der Einfallswinkel des Strahlenbündels auf die Eintrittsfläche 8 kann in Abhängigkeit von der konkreten Geometrie des optischen Systems gewählt werden. Bei dem in Fig. 3 gezeigten Beispiel ist der Einfallswinkel des parallelen Strahlenbündels auf die Eintrittsfläche 8 ungefähr 43°, andere Werte sind ebenfalls möglich. Auf der Eintrittsfläche 8 ist ein Kompensations-Beugungsgitter 11 angeordnet. Das Kompensations-Beugungsgitter 11 kann z.B. ein geblaztes diffraktives Transmissions-Beugungsgitter mit eingeschriebener Korrektur-Phasenfunktion sein, das so ausgelegt ist, um eine Achromasie des optischen Systems der Messsonde in einem vorgegebenen spektralen Bereich zu erreichen. Dies ermöglicht den möglichst vollständigen Rücktransport der Strahlungsenergie in den Faserkern 3. Vorzugsweise ist das Kompensations-Beugungsgitter 11 für die erste (m = 1 oder m = -1) Beugungsordnung ausgelegt, es kann jedoch für andere Beugungsordnung ausgelegt sein.

Ferner umfasst die Messsonde eine Schneideeinrichtung mit einer ersten Schneide 13 und einer zweiten Schneide 17, wobei die beiden Schneiden die Spitze der Messsonde bilden. Die erste Schneide ist integraler Bestandteil des aus Diamant geformten Fensterelements 10 (d.h. die erste Schneide 13 und das Fensterelement 10 bilden einen Diamantmonolith). Die zweite Schneide 17 ist integraler Bestandteil des Endreflektor-Trägerelements 18, das aus einem geeigneten Hartmaterial (z.B. aus einem geeigneten Hartmetall) gebildet ist. Die beiden Schneiden 13 und 17 sind ein einem Abstand von einander angeordnet, so dass ein "Rachen" bzw. ein Spalt gebildet wird, dessen Bestandteil der Messspalt 12 ist. Der Breite 12A des "Rachens" bzw. des Spalts ist vorzugsweise variabel ist. So kann - wie oben beschrieben - der "Rachen" vor und nach einer spektralen Messung geöffnet bzw. geschlossen werden. Der "Rachen" mündet in den Probe-Einlauf 14, der zugleich den Einlauf des "Rachens" bzw. des Messspalts 12 darstellt.

Das Endreflektor-Trägerelement 18 ist beweglich in Bezug zu Fensterelement 10 ausgebildet. Insbesondere kann das Endreflektor-Trägerelement mit der zweiten Schneide 17 entlang der vertikalen Richtung (die im Wesentlichen der Vorschubrichtung der Messsonde und der Längsachse La der Messsonde entspricht) bewegt werden und/oder in diese Richtung vibrieren. Zusätzlich kann optional das Endreflektor-Trägerelement 18 entlang einer zweiten Achse, die in Bezug auf die Längsachse La geneigt ist, bewegt werden bzw. in diese Richtung vibrieren. Durch die Bewegung des Endreflektor-Trägerelements 18 kann der Schneidevorgang unterstützt werden. Zusätzlich kann eine Verdichtung des sich im Messkanal 12 befindlichen Gewebes erzielt werden, um die Absorptionsverluste durch Wasser zu minimieren.

Auf einer der Flächen des Endreflektor-Trägerelements 18 ist der Endreflektor 19 angeordnet. Der Endreflektor 19 stellt eine der Begrenzungsflächen des Messspalts 12 dar. Auf der anderen Seite wird der Messspalt 12 von einer der Flächen (Fensterfläche 9) des Fensterelements begrenzt. Der Endreflektor ist als ein Reflexions-Beugungsgitter, beispielsweise als ein mikroprofiliertes, geblaztes Reflexions-Beugungsgitter mit eingeschriebener Linsen-Korrektur-Phasen-Funktion ausgebildet. Der Endreflektor 19 ist ferner als Retroreflektor ausgebildet und ermöglicht den Rücktransport der Strahlungsenergie in die m=-1 Beugungsordnung. Der Endreflektor 19 kann jedoch für eine andere Beugungsordnung ausgelegt sein. Fig. 3, rechts zeigt eine (nicht maßstabsgetreue) vergrößerte Ansicht des Endreflektors und des Strahlengangs durch den Endreflektor 19 (vgl. Fig. 3, Detail A).

Der Messspalt 12 ist schräg zur Längsachse La der Messsonde angeordnet. Die Vorschubrichtung der Messsonde in das zu vermessende (analysierende) Gewebe und die Spaltrichtung schließen vorzugsweise einen Winkel von mindestens 3° (5°) Altgrad ein, typischerweise einen Winkel von 20° bis 50°. In dem gezeigten Beispiel ist der Messspalt 12 parallel. Der Messspalt 12 kann jedoch keilförmig sein, wobei der Keilwinkel vorzugsweise eine mittlere Wellenlänge "Lambda-mittel" über der Spaltlänge beträgt.

Der schräg gestellte, vorzugsweise parallele Messspalt 12 hat den Vorteil, dass bei einer schwingenden Auf- und Ab-Bewegung des Endreflektor-Trägerelements 18 mit der zweiten Schneide 17 eine Verdichtung des bereits abgetrennten Gewebes durch Druck erreicht werden kann. Vorzugsweise wird das abgeschnittene Gewebe auf ungefähr 5 µm bis 10 µm verdichtet, um die Absorptionsverluste durch Wasser zu minimieren. Höhere Dicken sind jedoch auch möglich, insbesondere bei einer höheren Intensität des Messlichts. Der schräg gestellte Parallelspalt hat ferner den Vorteil, dass gemessenes Gewebe bei Bedarf seitlich durch den Entsorgungskanal 21 entsorgt werden kann. Dies hat den Vorteil, dass die Messsonde kompakter bzw. kleiner gestaltet werden kann. Die Breite des Messspalts 12A ist variabel, so dass eine Verdichtung der sich im Messspalt 12 befindliche Probenschicht erzielt werden kann.

Zur in-vivo Messung wird die Messsonde 12 zunächst im tiefen Raum der Probe 16 (z.B. in tiefen Raum eines Organs bzw. des Gewebes) eingeführt (Einstechvorgang). Das Einstechen der Messsonde erfolgt vorzugsweise mit zumindest näherungsweise konstanter Geschwindigkeit, z.B. von ungefähr 0,1 mm/s bis 0,5 mm/s. Vorzugsweise wird die Messsonde mittels Präzisions-Roboterarm in das Organ bzw. in das Gewebe 16 eingebracht. Dadurch können die erforderlichen Präzision, der Kraftaufwand und die Konstanz der Geschwindigkeit der Tiefenbewegung gewährleistet werden. Der Einstechvorgang kann jedoch auch manuell erfolgen. Ob eine manuelle Einfuhr der Messsonde möglich ist, hängt z.B. vom jeweiligen Organ und der Patientensituation ab.

Nachdem die Messsonde zu der gewünschten Messstelle vorgedrungen ist, wird der Messvorgang gestartet. Die Position der Messsonde 12 in Bezug zum untersuchten Organ während des Einstechvorgangs kann anhand von vorab ermittelten Daten bezüglich der räumlichen Orientierung des Patienten-Körpers und/oder des untersuchten Organs bestimmt werden. Zusätzlich oder alternativ kann die Position der Messsonde im untersuchten Organ mittels geeigneter Messverfahren (z.B. CT, Ultraschall, etc.), vorzugsweise in Echtzeit, gemessen werden und gegebenenfalls auf einer geeigneten Anzeige dargestellt werden. Anhand der Daten bezüglich der Position der Messsonde kann der Messvorgang gestartet und/oder beendet werden.

Mittels der in der Messsonde integrierten Schneideeinrichtung (umfassend die Schneiden 13 und 17) wird eine Gewebeschicht bzw. -lappen (Probenschicht) freigeschnitten und in den Messkanal 12 befördert. Anschließend kann eine Verdichtung des sich im Messkanal 12 befindlichen Gewebes erfolgen. Dies kann durch Mikrovibrationen des Endreflektor-Trägerelements 18 erzielt werden. Die spektrale Absorptionsmessung erfolgt vorzugsweise unmittelbar nach dem Schneidevorgang und der optionalen Verdichtung des Gewebes.

Während des Messvorgangs wird das im Wesentlichen parallele Messstrahlenbündel mittels des Fensterelements 10 in den Messspalt 12 eingekoppelt und nach zweifachem Durchgang durch das sich im Messspalt befindliche Gewebe aus dem Messspalt 12 ausgekoppelt. Dabei erfolgt ein, vorzugsweise schräger, Durchgang zumindest eines Hauptteils des parallelen Messstrahlenbündels durch die Fensterfläche 9 des Fensterelements 10. Das Messlicht propagiert durch das sich im Messspalt 12 befindliche Gewebe, wird von dem Endreflektor 19 reflektiert und propagiert ein zweites Mal durch das sich im Messspalt 12 befindliche Gewebe. Der Endreflektor 19 ist so ausgelegt und angeordnet, dass nach einer Reflexion und einem zweifachen Durchgang durch den Messspalt 12 zumindest ein Hauptteil des Messlichts in die Faser 4 zurückgeht. Mittels des Strahlteilers 2 und gegebenenfalls geeigneter Umlenk- und/oder Fokussieroptik wird das Messlicht zu der Einrichtung zur spektralen Analyse 22 geleitet. Durch die Verwendung eines kollimierten Bündels mit einem kleinen Durchmesser, das vom Endreflektor 19 in sich zurück reflektiert wird, insbesondere in Kombination mit einer konfokalen Diskriminierung beim Eintritt des Messlichts in das Faserende 5, kann das unerwünschte Streulicht weitgehend ausgeblendet werden. Insbesondere können Mie-Streuungs-Einflüsse erheblich reduziert werden.

Nach der Messung wird das vermessene Gewebe in dem Abtransport bzw. Entsorgungskanal 21 befördert. Der Entsorgungskanal 21 mündet bzw. steht in Verbindung mit dem Ausgang des Messkanals (Probeaustritt) 20. Das vermessene Gewebe kann z.B. mittels einer Absaugvorrichtung (nicht gezeigt), gegebenenfalls unterstützt durch Mikrovibrationen in den Entsorgungskanal 21 und aus den Entsorgungskanal 21 befördert werden. Wenn es nicht notwendig ist, eine weitere histologische Untersuchung des in-situ spektral vermessenen Gewebes durchzuführen, ist es möglich den Entsorgungskanal wegzulassen, was den Vorteil hat, dass die Messsonde dünner und somit patientenschonender gebaut werden kann. Das bereits spektral vermessene Gewebe kann in diesem Fall z.B. mittels höher energetischen fs-Laserpulsen zerstört werden. Das abgetötete Gewebe kann anschließend entsorgt oder in das Körpergewebe entlassen. werden, wo dieses resorbiert wird. Die höher energetischen fs-Laserpulse werden vorzugsweise zeitversetzt_zum spektralen Messen durch dieselbe Faser 4 zum bereits vermessenen Gewebe geleitet.

Die Einrichtung zur spektralen Analyse 22 umfasst einen Detektor, z.B, einen Integraldetektor. Für IR Bereich kann der Detektor z.B. ein Quecksilber-Cadmium-Tellurid-Detektor sein, welcher mit flüssigem Stickstoff gekühlt wird. Die Spektren-Aufnahmezeit pro Messort im Messmodus ist vorzugsweise möglichst kurz und liegt vorzugsweise unter 1s bis 10s. Dabei darf im Messvorgang das Gewebe nicht durch die beim Absorptionsmessen eingebrachte Strahlungslast zu schnell zerstört werden, d.h. nicht, bevor es seine spektrale Signatur abgeliefert hat. Um zuverlässige Messungen in der kurzen Zeit zu ermöglichen, kann die Lichtquelle eine brillante, breitbandige Lichtquelle sein, z.B. eine brillante Lichtquelle im MIR Bereich.

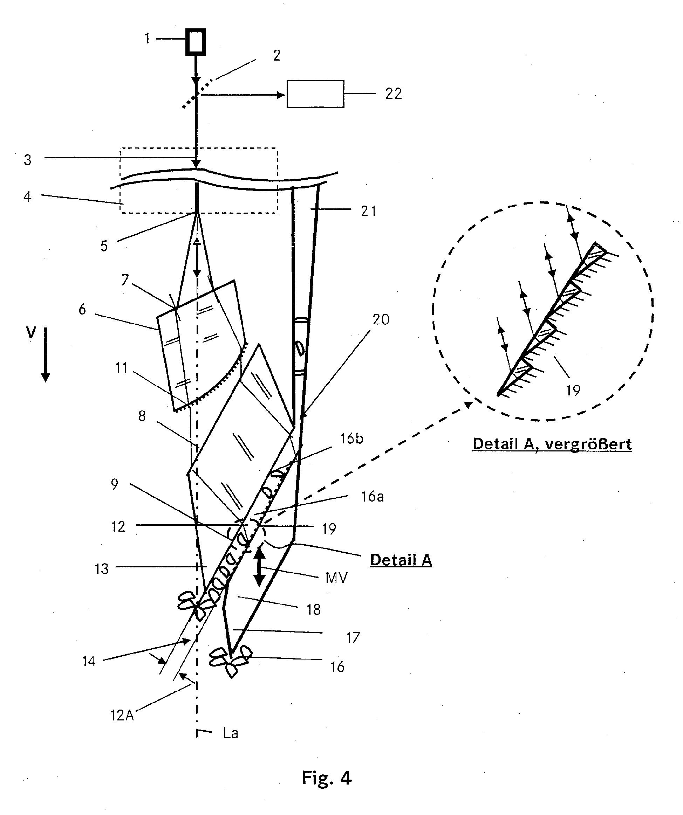

Fig. 4 zeigt eine Messsonde gemäß einem zweiten Beispiel und eine beispielhafte Anordnung zur spektralen Absorptionsmessung umfassend die Messsonde. Die optische Anordnung der Messsonde bzw. der Anordnung zur spektralen Absorptionsmessung entspricht weitgehend der optischen Anordnung der in Fig. 3 gezeigten Messsonde bzw. Anordnung zur spektralen Absorptionsmessung, mit dem Unterschied, dass das Kompensations-Beugungsgitter 11 an die Austrittsfläche der asphärischen Linse 6 angeordnet ist. Die Austrittsfläche der Linse 6 ist in diesem Beispiel eine gekrümmte Fläche, z.B. eine sphärische oder asphärische (z.B. FreiformFläche). Das Kompensations-Beugungsgitter 11 kann - wie bei dem in Fig. 3 gezeigtem Beispiel - ein geblaztes diffraktives Transmissions-Beugungsgitter mit eingeschriebener Korrektur-Phasen-Funktion sein, um Achromasie zu erreichen.

Die ZnSe-Linse 6 ist Single-point-Diamant-bearbeitbar. Das gilt ebenfalls für die Fertigung des Kompensations-Beugungsgitters auf der ZnSe Linse. Somit kann eine teure und aufwendige Aufbringung des Kompensations-Beugungsgitters auf dem aus Diamant gebildeten Fensterelement vermieden werden. Im Hinblick auf eine unerwünschte chromatische Queraufspaltung im Messspalt 12 ist es vorteilhaft, das Kompensations-Beugungsgitter 11 so nah wie möglich an dem Messspalt 12 anzuordnen.

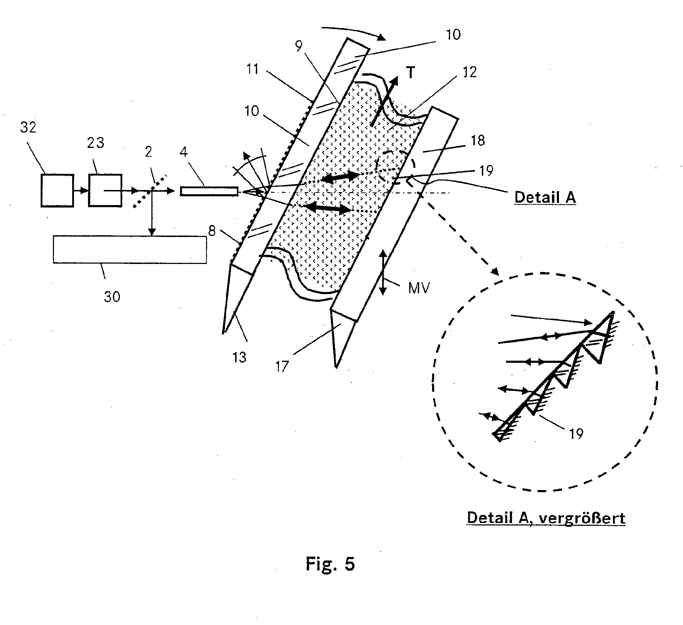

**Fig.** 5 zeigt eine Messsonde gemäß einem dritten Beispiel und eine beispielhafte Anordnung zur spektralen Absorptionsmessung umfassend die Messsonde. Wie im ersten und zweiten Beispiel umfasst die Anordnung zur spektralen Absorptionsmessung eine Lichtquelle, welche in diesem Beispiel eine brillante MIR-Breitband Quelle 32 ist. Ferner umfasst die Anordnung zur spektralen Absorptionsmessung ein Michelson-Interferometer 23 als Modulator, wobei das Interferometer 23 der MIR-Breitband Quelle 32 nachgeordnet ist. Die Lichtquelle kann jedoch eine andere Lichtquelle sein, z.B. eine Wellenlängen-durchstimmbare Lichtquelle (z.B. einen QCL-Laser).

Wie in den vorigen Beispielen wird das modulierte Beleuchtungslicht in eine Faser 4 eingekoppelt und zur Messsonde bzw. zum Fensterelement 10 geleitet.

Das Fensterelement 10 (nicht maßstabgetreu dargestellt) ist als ein im Wesentlichen planparalleles Diamantstück ausgebildet, welches zusammen mit der ersten Schneide 13 einen Diamantmonolith bildet. Auf die Eintrittsfläche 8 des Fensterelements 10 ist das Kompensations-Beugungsgitter 11 angeordnet. Das Kompensations-Beugungsgitter 11 ist ein Transmissions-Beugungsgitter, das ausgelegt ist, das optische System zu achromatisieren.

Die Messsonde umfasst ferner ein Endreflektor-Trägerelement 18, in diesem Fall in Form eines planparallelen Stücks mit integrierter zweiter Schneide 17. Das Endreflektor-Trägerelement 18 kann ebenfalls aus Diamant gebildet werden. Es ist jedoch möglich, das Endreflektor-Trägerelement 18 aus einem anderen geeigneten, vorzugsweise harten Material zu bilden. Das Endreflektor-Trägerelement 18 bildet mit der zweiten Schneide 17 ebenfalls einen Monolith.

Der Messspalt 12 wird von der im Wesentlichen planen Fensterfläche 9 des Fensterelements 10 und einer im Wesentlichen planen Fläche des Endreflektor-Trägerelements 18 abgegrenzt. Auf die dem Messspalt zugewandte Fläche des Endreflektor-Trägerelements 18 ist der Endreflektor 19 angeordnet. Der Endreflektor kann - wie oben beschrieben - ein diffraktives optisches Element sein, z.B. ein mikroprofiliertes Reflexions-Beugungsgitter in Auto-Reflexions bzw. "Quasi-Auto-Reflexions" Modus. Das diffraktive optische Element weist eine Phasenfunktion auf, welche derart gewählt ist, dass Achromasie erreicht wird. Das diffraktive optische Element kann ein vergrabendes diffraktives optisches Element sein (vgl. Fig. 5, Detail A). Dies hat den Vorteil, dass die den Messspalt 12 begrenzende Wand glatt ist, was die Beförderung des Gewebes in und aus dem Messspalt 12 erleichtern kann.

Die Funktionsweise der Messsonde und der Anordnung zur spektralen Absorptionsmessung gemäß dem dritten Beispiel ist ähnlich der Funktionsweise des in Fig. 3 gezeigten Beispiels, mit dem Unterschied, dass das Mess-Strahlenbündel nicht kollimiert wird. Das vom Faserende kommende divergente Strahlenbündel fällt auf die Eintrittsfläche 8 mit dem Kompensations-Beugungsgitter des Fensterelements 10 ein, wird gebeugt, propagiert durch das Ein-und Auskoppelelement und wird durch die Fensterfläche 9 des Fensterelements 10 in den Messspalt 12 eingekoppelt. Nach Reflexion an dem Endspielgel 19 propagiert das Messlicht erneut durch den Messspalt 12 und wird in die Faser 4 eingekoppelt. Mittels Strahlteiler 2 wird das Messlicht zum Detektor 30 (als Teil der Einrichtung zur spektralen Analyse) geleitet. Für Licht im MIR Bereich kann der Detektor ein Quecksilber-Cadmium-Tellurid-Detektor sein, der mit flüssigem Stickstoff gekühlt wird. Das detektierte Messsignal wird anschließend analysiert, um Informationen über das untersuchte Gewebe zu erhalten.

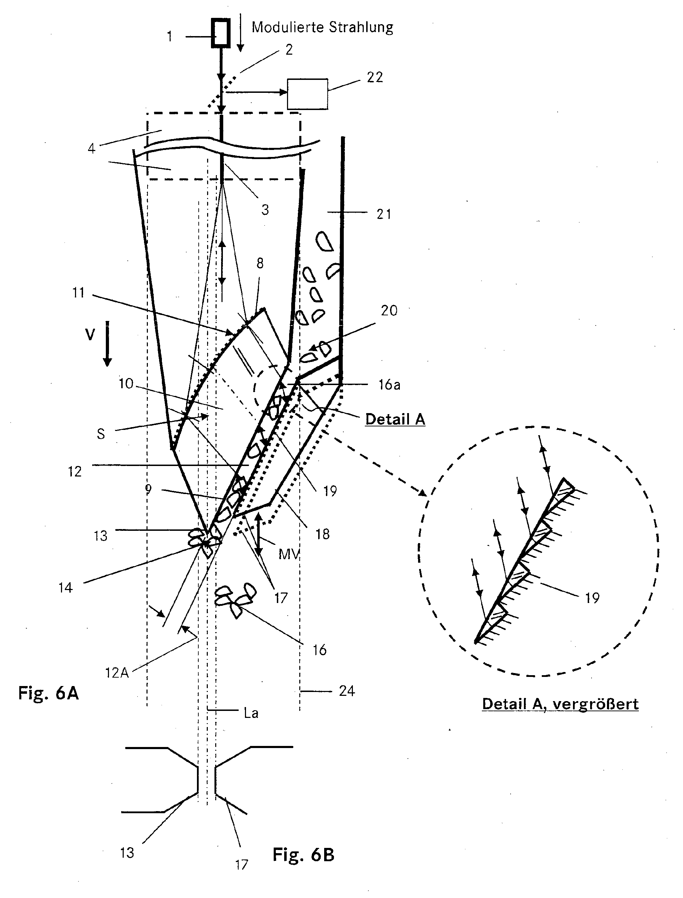

Fig. 6A zeigt eine Messsonde gemäß einem vierten Beispiel und eine beispielhafte Anordnung zur spektralen Absorptionsmessung umfassend die Messsonde. Wie bei den vorigen Beispielen umfasst die Anordnung zur spektralen Absorptionsmessung eine Lichtquelle 1 und eine Einrichtung zur spektralen Analyse 22, wobei das von der Lichtquelle 1 ausgestrahlte und optional modulierte Licht in eine Faser 4 eingekoppelt wird. Das vom Kern 3 der Faser 4 kommende divergente Strahlenbündel fällt auf die gekrümmte (sphärische oder asphärische) Eintrittsfläche 8 mit dem Kompensations-Beugungsgitter 11 des Fensterelements 10 ein. Die Eintrittsfläche 8 und das Kompensations-Beugungsgitter 11 sind derart ausgelegt, dass das an die Eintrittsfläche 8 gebrochene Strahlenbündel S zumindest näherungsweise kollimiert wird. Das aus Diamant gebildete Fensterelement 10 weist ferner eine Fensterfläche 9 auf, durch welche das kollimierte Strahlenbündel S in den Messspalt 12 eingekoppelt wird. Der Messspalt 12 ist ferner von dem Endreflektor 19 begrenzt, der an einem kufenförmigen Endreflektor-Trägerelement 18 angeordnet ist. Der Endreflektor 19 kann - wie oben beschrieben - als ein diffraktives optisches Element, vorzugsweise als ein vergrabenes diffraktives optisches Element ausgebildet werden (vgl. Fig. 6A, Detail A, vergrößert). Der Endreflektor kann in einem Autoreflexionsmodus oder in einem Quasi-Autoreflexionsmodus arbeiten.

Die Messsonde weist ferner zwei Schneiden 13 und 17 auf, die die Spitze der Messsonde bilden, wobei die erste Schneide 13 integral mit dem Fensterelement 10 und die zweite Schneide 17 integral mit dem Endreflektor-Trägerelement 18 geformt ist. Die erste Schneide 13 ist in diesem Beispiel die Hauptschneide und macht den ersten Einstich. Die zweite Schneide 17 ist die Nebenschneide. Fig. 6B zeigt eine vergrößerte Ansicht (nicht maßstabgetreu) der beiden Schneiden, die die Spitze der Messsonde bilden.

Das Endreflektor-Trägerelement 18 kann entlang der Vorschubrichtung V der Messsonde (die im Wesentlichen parallel zur Längsachse La der Messsonde ist) bewegt werden bzw. entlang dieser Richtung vibrieren. Das Endreflektor-Trägerelement 18 kann optional entlang einer zweiten Richtung vibrieren, die schräg (z.B. senkrecht) zur der Vorschubrichtung ist. Durch die Vibration des Endreflektor-Trägerelements 18 mit der zweiten Schneide 17 kann der Schneidevorgang unterstützt werden. Ferner kann die Breite 12 des Messspalts 12 variiert werden, um eine Ausdünnung bzw. Verdichtung des sich im Messspalt befindlichen Gewebes zu erreichen.

Des Weiteren umfasst die Messsonde ein Kompensations-Beugungsgitter 11, das auf der Eintrittsfläche 8 des Fensterelements 10 angeordnet ist. Das Kompensations-Beugungsgitter 11 ist ausgelegt, in Kombination mit dem diffraktiven Endreflektor 19, eine Achromasie des optischen Systems zu erreichen. Die restlichen Elementen sowie die Funktionsweise der Messsonde und der Anordnung zur spektralen Absorptionsmessung in diesem Beispiel entsprechen weitgehend der Funktionsweise des in Fig. 3 gezeigten Beispiels.

Bei den in Fig. 3 bis 6 gezeigten Beispielen stellt der Endreflektor 19 zugleich eine der den Messspalt 12 begrenzenden Flächen dar. Anders ausgedrückt ist der Endreflektor 19 als Vorder-Endreflektor ausgebildet. Das Endreflektor-Trägerelement 18 muss folglich nicht transparent sein und kann aus einem beliebigen geeigneten Material gebildet werden. Da das Endreflektor-Trägerelement 19 keine Transmission und/oder keinen Faseranschluss aufweisen muss, eignet sich das Endreflektor-Trägerelement 18 für die Ausbildung als Vibrations-Mikrotom. In diesem Fall kann das Endreflektor-Trägerelement 18 fest mit der zweiten Schneide 17 verbunden werden und z.B. in Längsrichtung der Messsonde (auf und ab) zu vibrieren.

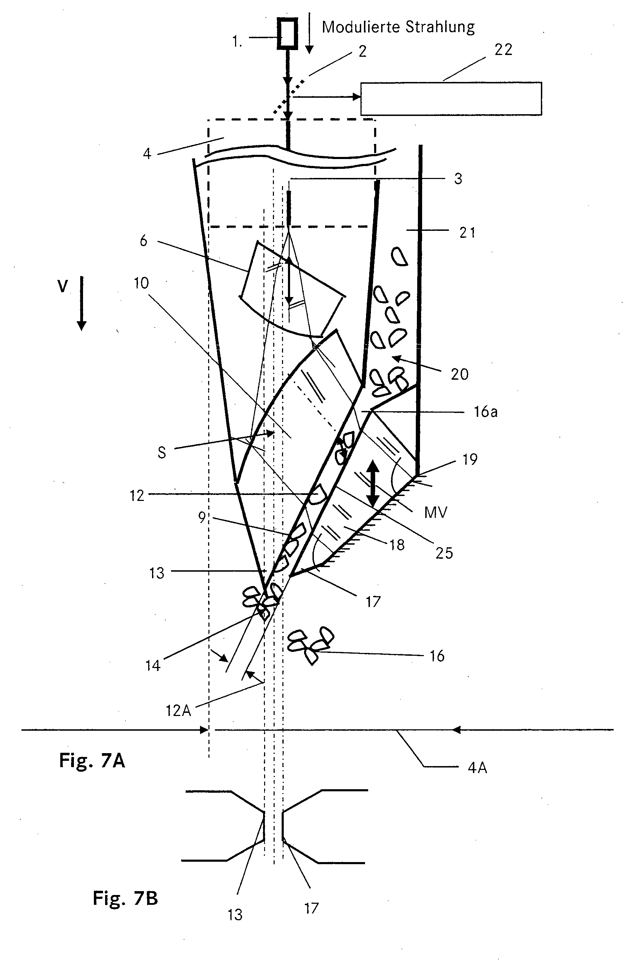

Es ist jedoch möglich, den Endreflektor 19 als ein Hinter-Endreflektor anzuordnen. **Fig. 7A** zeigt eine Messsonde mit einem Hinter-Endreflektor gemäß einem fünften Beispiel und eine beispielhafte Anordnung zur spektralen Absorptionsmessung umfassend die Messsonde. Wie bei den vorigen Beispielen umfasst die Anordnung zur spektralen Absorptionsmessung eine Lichtquelle 1 und eine Einrichtung zur spektralen Analyse 22, wobei das von der Lichtquelle 1 ausgestrahlte Licht in den Faserkern einer Faser 4 eingekoppelt wird.

Die Messsonde umfasst eine, z.B. asphärische, Linse 6 aus ZnSe oder aus einem anderen geeigneten transparenten Material. Ferner umfasst die Messsonde ein, vorzugsweise aus Diamant, gebildetes Fensterelement 10 und ein Endreflektor-Trägerelement 18, das ebenfalls aus einem transparenten Material, vorzugsweise Diamant, ist. Das Fensterelement 10 ist integral mit der ersten Schneide 13 ausgebildet (d.h. die erste Schneide 13 und das Fensterelement 10 formen einen Monolith). Ebenfalls ist das Endreflektor-Trägerelement 18 integral mit der zweiten Schneide 17 ausgebildet. Die erste Schneide 13 ist in diesem Beispiel die Hauptschneide und macht den ersten Einstich. Die zweite Schneide 17 ist die Nebenschneide. Fig. 7B zeigt eine vergrößerte Ansicht (nicht maßstabgetreu) der beiden Schneiden, die die Spitze der Messsonde bilden.

Der Messspalt 12 wird von der im Wesentlichen planen Fensterfläche 9 des Fensterelements 10 und der im Wesentlichen planen Fensterfläche 25 des transparenten Endreflektor-Trägerelements 18 begrenzt. Auf eine der anderen Flächen des prismen- oder kufenförmigen Endreflektor-Trägerelements 18 ist der Endreflektor 19 angeordnet. In diesem Beispiel ist der Endreflektor 19 ein Spiegel, z.B. aus Gold.

Das Ein-und Auskoppelement 10 ist als eine sphärische oder asphärische Diamantlinse oder ein Diamantprisma (mit einem Brechungsindex n=2,38 bei einer Wellenlänge von 8,6 µm) ausgebildet und weist eine gekrümmte Eintrittsfläche 8 auf. Die Linse 6 und das Fensterelement 10 sind so ausgelegt und angeordnet, das vom Faserende kommende divergierende Mess-Strahlenbündel zu kollimieren. Das kollimierte Mess-Strahlenbündel S wird durch die Fensterfläche 9 in den Messspalt 12 eingekoppelt, propagiert durch den Messspalt 12 und durch das transparente Endreflektor-Trägerelement 18, und wird vom Spiegel (Endreflektor) 19 zurückreflektiert. Der Spiegel 19 ist so angeordnet, dass das Messlicht im Wesentlichen senkrecht auf den Spiegel 19 einfällt, so dass das Mess-Strahlenbündel in sich zurückreflektiert wird. Das reflektierte Mess-Strahlenbündel propagiert erneut durch das Endreflektor-Trägerelement 18, den Messspalt 12 und das Fensterelement 10 und wird in die Faser 4 eingekoppelt. Mittels des Strahlteilers 2 wird das Messlicht zu der Einrichtung zur spektralen Analyse geleitet.

Wie in den obigen Beispielen ist das Endreflektor-Trägerelement 18 beweglich in Bezug zu Fensterelement 10. Insbesondere kann das Endreflektor-Trägerelement 18 mit der zweiten Schneide 17 entlang der vertikalen Richtung (die im Wesentlichen der Vorschubrichtung V der Messsonde und der Längsachse La der Messsonde entspricht) bewegt werden und/oder in diese Richtung vibrieren. Zusätzlich kann das Endreflektor-Trägerelement 18 entlang einer zweiten Achse, die in Bezug auf die Längsachse La geneigt ist, bewegt werden bzw. in diese Richtung vibrieren.

Die restlichen Elementen sowie die Funktionsweise der Messsonde und der Anordnung zur spektralen Absorptionsmessung (wie z.B. Probeaustritt 20, Entsorgungskanal 21, etc.) entsprechen im Wesentlichen den in Fig. 3 gezeigten Elementen.

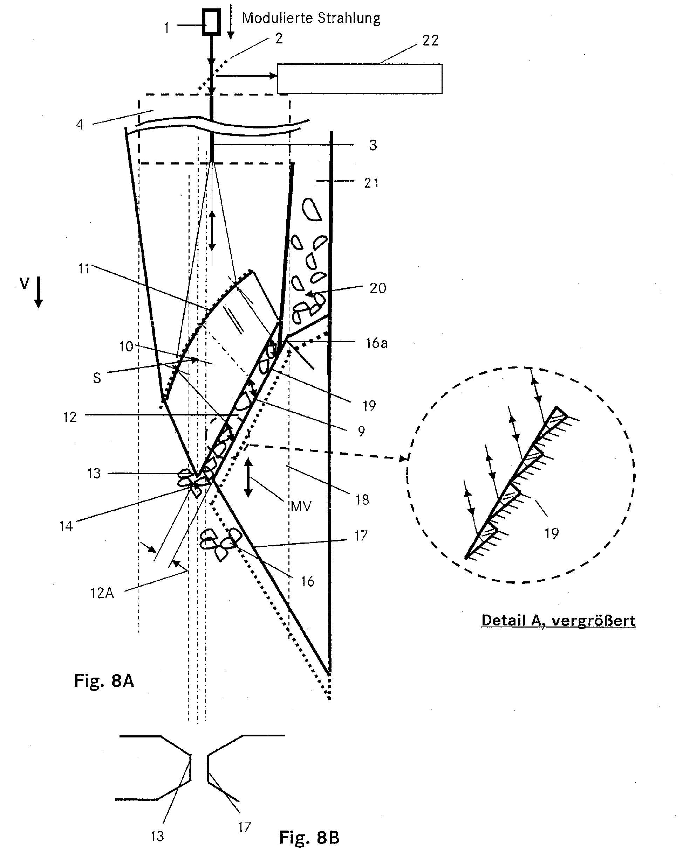

Fig. 8A zeigt eine Messsonde gemäß einem sechsten Beispiel und eine beispielhafte Anordnung zur spektralen Absorptionsmessung umfassend die Messsonde. Die optische Anordnung der in Fig. 8A gezeigten Messsonde entspricht der optischen Anordnung der in Fig. 6A gezeigten Messsonde, mit dem Unterschied, dass die zweite Schneide 17 als die Hauptschneide ausgebildet ist. Fig. 8B zeigt eine vergrößerte Ansicht (nicht maßstabgetreu) der beiden Scheiden 13 und 17, die die Spitze der Messsonde bilden.

Die entlang der Vorschubrichtung V bewegliche zweite Schneide 17 macht den ersten Einstich in das zu untersuchende Gewebe 16, gefolgt von der ersten Schneide 13. Das bewegliche, kufenförmige Endreflektor-Trägerelement 18 mit der integral geformten zweiten Schneide 17 kann aus Diamant oder aus einem anderen Hartwerkstoff gebildet sein. Da der Endreflektor 19 unmittelbar an den Messspalt 12 angrenzt, muss das Endreflektor-Trägerelement 19 nicht transparent sein.

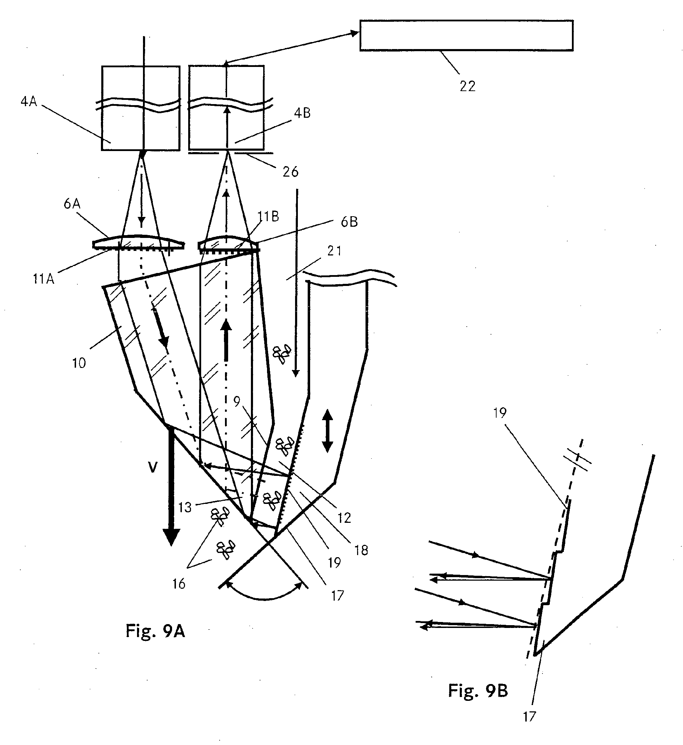

Die in den Fig. 1 bis 8 gezeigten Messsonden bzw. Anordnungen zur spektralen Absorptionsmessung weisen jeweils eine Faser 4 auf, die sowohl Teil des Beleuchtungs- als auch Teil des Detektionskanals ist. **Fig. 9A** zeigt eine Messsonde gemäß einem siebten Beispiel und eine beispielhafte Anordnung zur spektralen Absorptionsmessung umfassend die Messsonde, wobei der Beleuchtungskanal und der Detektionskanal der Anordnung zur spektralen Absorptionsmessung voneinander getrennt sind. **Fig. 9B** zeigt eine vergrößerte Detailansicht des diffraktiven Endreflektors 19.

Wie bei den vorigen Beispielen umfasst die Anordnung zur spektralen Absorptionsmessung eine Lichtquelle (nicht gezeigt) und eine Einrichtung zur spektralen Analyse 22. Das von der Lichtquelle ausgestrahlte Licht wird in den Faserkern einer Sende- bzw. Beleuchtungsfaser 4A eingekoppelt. Das vom Faserende kommende divergente Strahlenbündel wird von der ZnSe Linse 6A kollimiert und propagiert durch das Fensterelement 10. Auf der Austrittsfläche der ersten Linse 6A ist ein erstes Kompensations-Beugungsgitter 11A angeordnet, um Achromasie des optischen Systems zu erreichen. Das Kompensations-Beugungsgitter 11A kann ein geblaztes Transmissionsgitter mit eingeschriebener Linsen- und Korrektur-Phasen-Funktion und gegebenenfalls einem Keilfaktor sein. Das Messlicht wird in den Messspalt 12 eingekoppelt, propagiert durch den Messspalt 12, wird an dem Endreflektor 19 reflektiert, propagiert erneut durch den Messspalt 12 und wird mittels einer zweiten ZnSe Linse 6B in eine Detektionsfaser 4B eingekoppelt. Auf der Lichteintrittsfläche der zweiten Linse 6B ist ein zweites Kompensations-Beugungsgitter 11B angeordnet, z.B. ein geblaztes Transmissionsgitter mit eingeschriebener Linsen- und Korrektur-Phasen-Funktion und gegebenenfalls einem Keilfaktor. In der Fokalebene der zweiten ZnSe Linse 6B ist ferner eine Blende 26 angeordnet, die direkte Reflexionen ausblendet. Das zweite Kompensationsbeugungsgitter 11B ist derart ausgelegt, um in Kombination mit dem ersten Kompensations-Beugungsgitter 11A und dem als ein diffraktives optisches Element ausgebildeten Endreflektor 19 eine weitgehende Achromasie des optischen Systems zu erreichen.

Die Messsonde weist ferner eine erste Schneide 13, welche integral mit dem Fensterelement 10 ausgebildet ist und eine zweite Schneide 17, welche integral mit dem beweglichen Endreflektor-Trägerelement 18 ausgebildet ist. Ferner weist die Messsonde einen Entsorgungskanal 21 auf, der zwischen dem Fensterelement 10 und dem beweglichen Endreflektor-Trägerelement 18 angeordnet ist und in den Messspalt 12 mündet. Die restlichen Elemente und die Funktionsweise der Messsonde und der Anordnung zur spektralen Absorptionsmessung entsprechen im Wesentlichen den in den vorigen Figuren gezeigten Elementen.

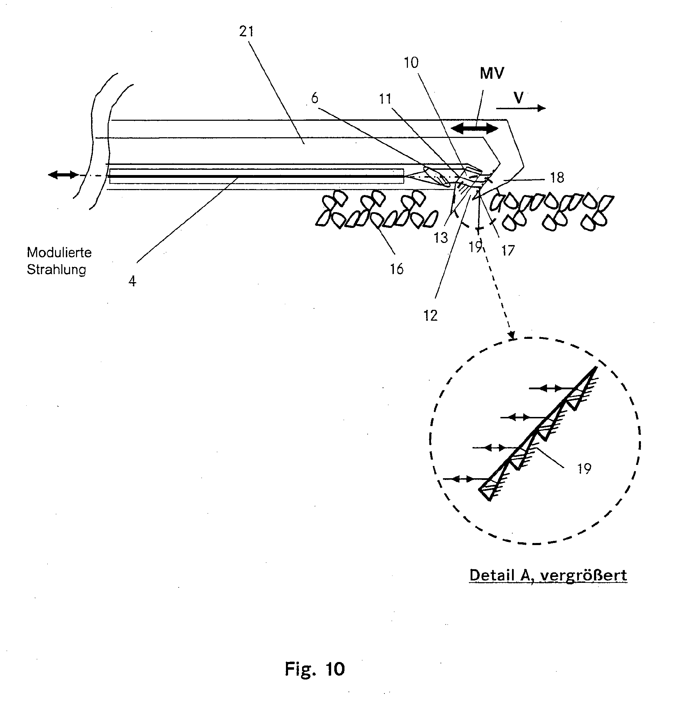

Fig. 10 zeigt eine Messsonde gemäß einem achten Beispiel. Die Messsonde kann horizontal zur Probeentnahme bewegt werden, d.h. die Vorschubrichtung V ist im Wesentlichen parallel zur horizontalen Richtung. Die Bewegung zur Probenentnahme ist vorzugsweise vibrationsunterstützt. Die Messsonde umfasst eine Beleuchtungs- und Detektionsfaser 4, eine Linse 6 (z.B. eine ZnSe Linse) zum Kollimieren des Messstrahls, ein Fensterelement 10 aus Diamant mit einer ersten Schneide 13 und einen diffraktiven Endreflektor 19. Der Endreflektor ist auf einem beweglichen Endreflektor-Trägerelement 18 angeordnet, welches einen Monolith mit der zweiten Schneide 17 bildet. Ferner umfasst die Messsonde einen Abtransportkanal 21, der mit einer Absaugvorrichtung (nicht gezeigt) verbunden ist. Die restlichen Elementen und die Funktionsweise der Messsonde und der Anordnung zur spektralen Absorptionsmessung entsprechend im Wesentlichen den in den vorigen Figuren gezeigten Elementen.

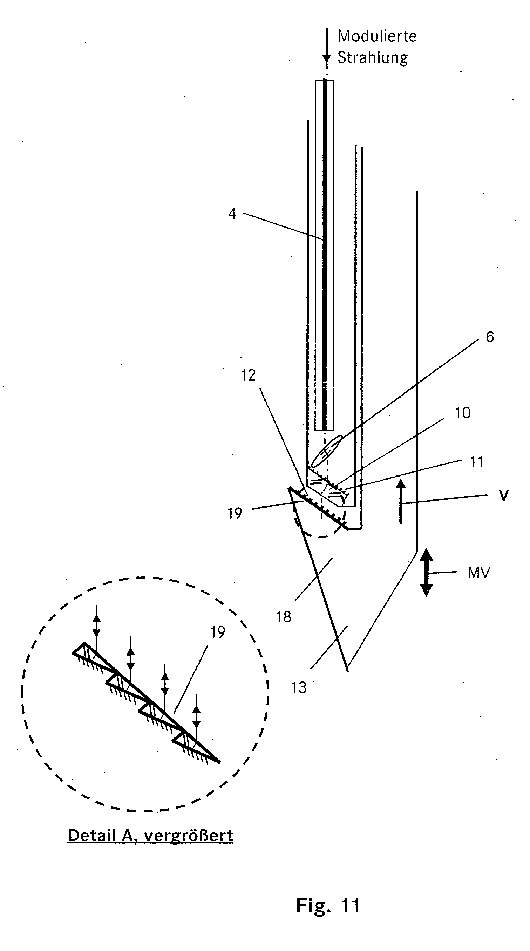

**Fig. 11** zeigt eine Messsonde gemäß einem neunten Beispiel. Die Messsonde umfasst eine Beleuchtungs- und Detektionsfaser 4, eine Linse 6 (z.B. eine ZnSe Linse) zum Kollimieren des Messstrahls, ein Fensterelement 10 aus Diamant und einen diffraktiven Endreflektor 19. Der Endreflektor ist auf einem beweglichen Endreflektor-Trägerelement 18 angeordnet, welches einen Monolithen mit einer ersten Schneide 13 bildet. Das Endreflektor-Trägerelement 18 ist beweglich entlang der Vorschubrichtung V, wobei die Bewegung des Endreflektor-Trägerelements vibrationsunterstützt ist. Die Probe kann eine Gewebeprobe sein, die ein wässriges Medium 16a und Probenpartikel (z.B. Gewebepartikel) umfasst. Die Entnahme einer zu vermessenden Probenschicht erfolgt in diesem Beispiel bei einer Auf-Bewegung des Endreflektor-Trägerelements mit der zweiten Schneide 13. Die restlichen Elemente und die Funktionsweise der Messsonde und die Anordnung zur spektralen Absorptionsmessung entsprechen im Wesentlichen den in den vorigen Figuren gezeigten Elementen.

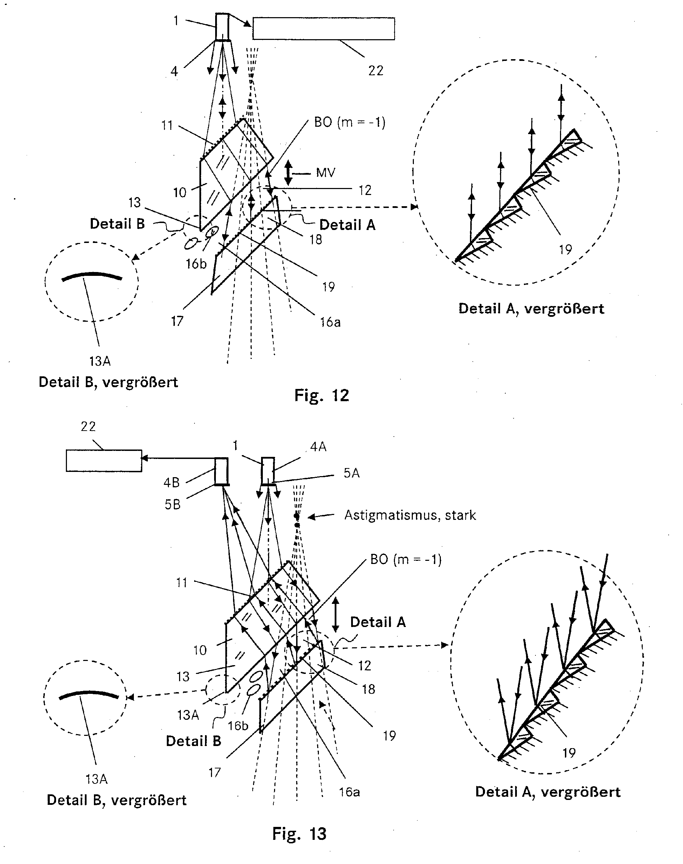

**Fig. 12** zeigt eine Messsonde gemäß einem zehnten Beispiel und eine beispielhafte Anordnung zur spektralen Absorptionsmessung umfassend die Messsonde. Die Anordnung zur spektralen Absorptionsmessung umfasst eine Lichtquelle 1 mit einem Faserausgang und eine Einrichtung zur spektralen Analyse 22. Das von der Lichtquelle emittierte Licht wird in eine Faser 4 eingekoppelt und zur Messsonde geführt. Die Messsonde umfasst ein Fensterelement 10 (z.B. aus Diamant) mit einer ersten Schneide 13 und einem Kompensations-Beugungsgitter 11. Die Schneidkante der ersten Schneide 13 kann gegebenenfalls etwas hohl sein (vgl. Fig. 12, Detail B, vergrößert). Ferner umfasst die Messsonde ein bewegliches (vorzugsweise vibrationsunterstützt) Endreflektor-Trägerelement 18 aus hartem Material bzw. Werkstoff mit einer zweiten Schneide 17 und einem diffraktiven Endreflektor 19. Der diffraktive Endreflektor 19 ist z.B. ein vergrabenes diffraktives optisches Element, das für Reflexion in die m = -1 Beugungsordnung ausgelegt ist. Der Endreflektor 19 ist für Retroreflexion bzw. "Quasi"-Retroreflexion ausgelegt, so dass das Beleuchtungslicht und das Messlicht nach einem zweifachen Durchgang durch die Probenschicht einen gemeinsamen Strahlengang aufweisen. Es ist somit möglich, die Messsonde kleiner zu bauen, vorzugsweise mit einem Durchmesser von 1 mm und kleiner. Die restlichen Elementen und die Funktionsweise der Messsonde und der Anordnung zur spektralen Absorptionsmessung entsprechend im Wesentlichen den in den vorigen Figuren gezeigten Elementen.

**Fig. 13** zeigt eine Messsonde gemäß einem elften Beispiel und eine beispielhafte Anordnung zur spektralen Absorptionsmessung umfassend die Messsonde. Wie bei dem in Fig. 9A gezeigten Beispiel umfasst die Messsonde bzw. die Anordnung zur spektralen Absorptionsmessung einen getrennten Beleuchtungs- und Detektionskanal.

Die Anordnung zur spektralen Absorptionsmessung gemäß dem elften Beispiel umfasst eine Lichtquelle 4A mit integrierter Faser mit einem Faserkern-Ende 5A, eine Detektionsfaser 4B mit einem Fasereingang 5B und eine Einrichtung zur spektralen Analyse 22. Die Messsonde umfasst ein Fensterelement 10 (z.B. aus Diamant) mit einer integrierten ersten Schneide 13 und einem Kompensations-Beugungsgitter 11. Die Schneidkante der ersten Schneide 13 kann gegebenenfalls etwas hohl sein (vgl. Fig. 13, Detail B, vergrößert). Ferner umfasst die Messsonde ein bewegliches (vorzugsweise vibrationsunterstützt) Endreflektor-Trägerelement 18 aus hartem Material bzw. Werkstoff mit einer integrierten zweiten Schneide 17 und einem diffraktiven Endreflektor 19. Der diffraktive Endreflektor 19 (vgl. Fig. 13, Detail A, vergrößert) ist z.B. ein vergrabenes diffraktives optisches Element, das für Reflexion in die m = -1 Beugungsordnung ausgelegt ist. Der Endreflektor 19 ist so ausgelegt, dass das Messlicht nach einem zweifachen Durchgang durch die Probenschicht und dem Fensterelement 10 in die Detektionsfaser 4B eingekoppelt wird. In diesem Beispiel weist der Endreflektor 19 einen starken Astigmatismus auf. Die restlichen Elemente und die Funktionsweise der Messsonde und die Anordnung zur spektralen Absorptionsmessung entsprechen im Wesentlichen den in den vorigen Figuren gezeigten Elementen (vgl. insbesondere Fig. 9A).

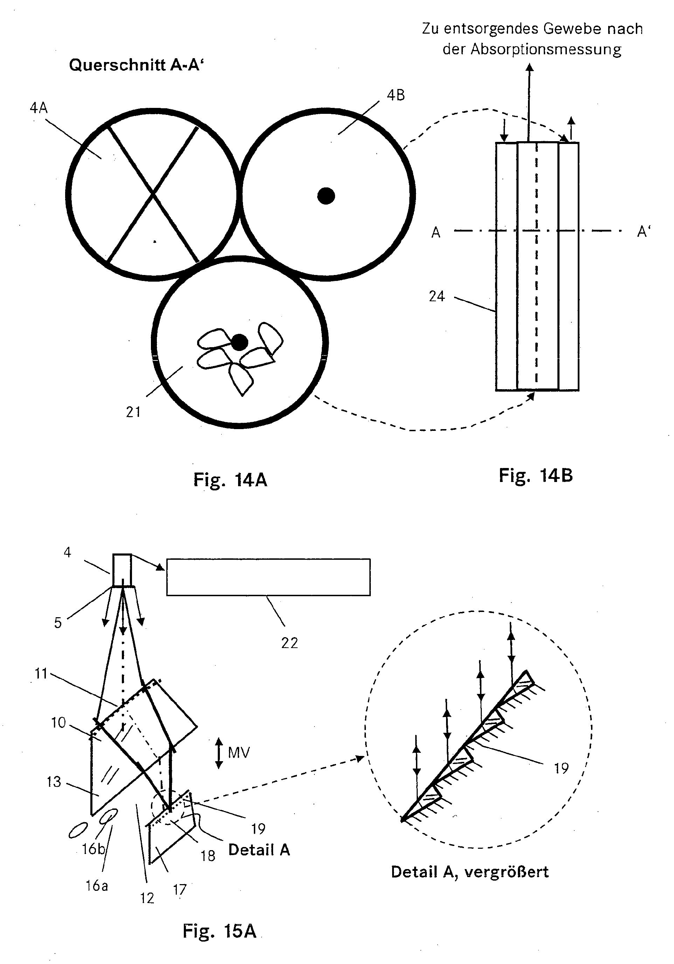

**Fig. 14A** zeigt eine Messsonde gemäß einem zwölften Beispiel, wobei Fig. 14A den Querschnitt durch die Messsonde entlang der Linie A-A' und Fig. 14B eine Seitenansicht der Messsonde zeigt (nicht maßstabgetreu).

Die Messsonde umfasst eine Beleuchtungsfaser 4A, eine Detektionsfaser 4B und einen Abtransportkanal (Entsorgungskanal) 21 für freigeschnittenes Gewebe nach der Absorptionsmessung (vgl. Fig. 14A). Die Beleuchtungsfaser 4A, die Detektionsfaser 4B und der Entsorgungskanal 21 sind in einem Gehäuse 24 integriert (vgl. Fig. 14B). Das Gehäuse kann z.B. aus Edelstahl oder aus einem anderen geeigneten biokompatiblen Material sein. Nach der Absorptionsmessung kann das vermessene Gewebe von einer geeigneten Absaugvorrichtung (nicht gezeigt) durch den Entsorgungskanal 21 abgesaugt werden, optional durch Mikro-Vibrations-Unterstützung. Wenn aus Platzgründen (z.B. um die Messsonde möglichst dünn zu machen) die Messsonde keinen Entsorgungskanal für das vermessene Gewebe aufweisen soll, kann das vermessene Gewebe bzw. die vermessenen Gewebestücken und/oder Zellen mittels fs-Laser-Pulsen durch Wärmebildung abgetötet werden und anschließend in das Körpergewebe entlassen bzw. entsorgt werden, wo es/sie resorbiert wird/werden. Die restlichen Elemente und die Funktionsweise der Messsonde und der Anordnung zur spektralen Absorptionsmessung entsprechen im Wesentlichen den in den vorigen Figuren gezeigten Elementen.

**Fig. 15A** zeigt eine Messsonde gemäß einem dreizehnten Beispiel und eine Anordnung zur spektralen Absorptionsmessung umfassend die Messsonde. Wie in den obigen Beispielen umfasst die Anordnung zur spektralen Absorptionsmessung eine Lichtquelle 1 mit integrierter Faser mit einem Faserkern-Ende 5A und eine Einrichtung zur spektralen Analyse 22. Die Messsonde umfasst ein Fensterelement 10 mit einer ersten Schneide 13 und einem Kompensations-Beugungsgitter 11. Ferner umfasst die Messsonde ein bewegliches (vorzugsweise vibrationsunterstützt) Endreflektor-Trägerelement 18 aus hartem Material bzw. Werkstoff mit einer zweiten Schneide 17 und einem diffraktiven Endreflektor 19, welcher für Retroreflexion bzw. "Quasi"-Retroreflexion ausgelegt ist. In diesem Beispiel wird ein fokussierter bzw. konvergierender Messstrahl verwendet. Der Fokusdiameter ist z.B. ungefähr 200 µm, andere Werte sind ebenfalls möglich. Wegen des relativ großen Erfassungsaperaturwinkels weist diese optische Anordnung eine relativ hohe Effizienz bei der Detektion auf. Die Intensität des Beleuchtungslichts kann aufgrund des relativ kleinen Messfelds relativ gering sein. Die restlichen Elemente und die Funktionsweise der Messsonde und der Anordnung zur spektralen Absorptionsmessung entsprechen im Wesentlichen den in den vorigen Figuren gezeigten Elementen.

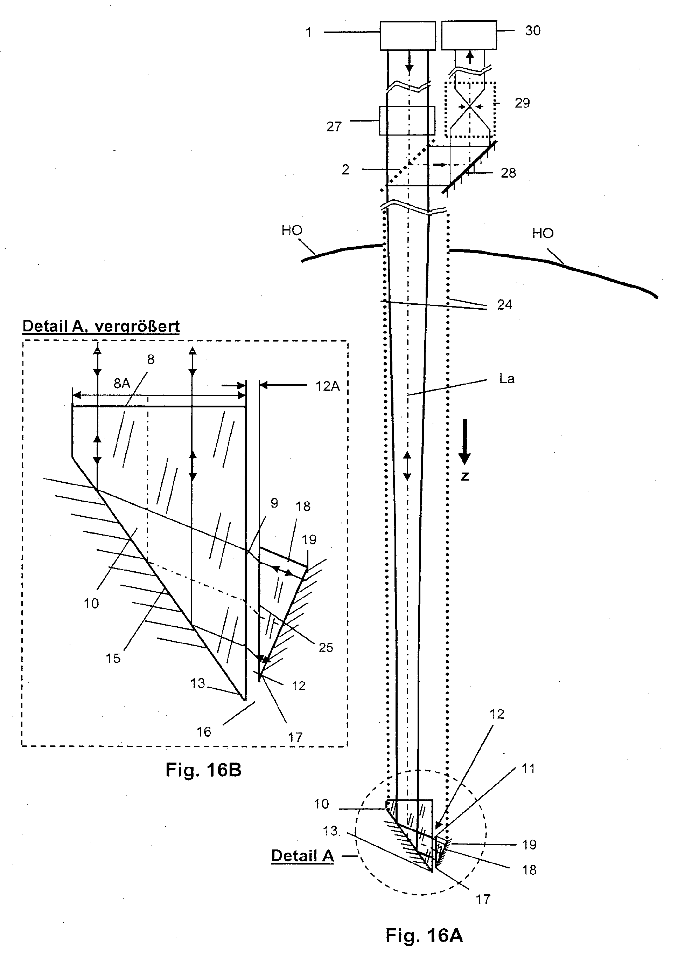

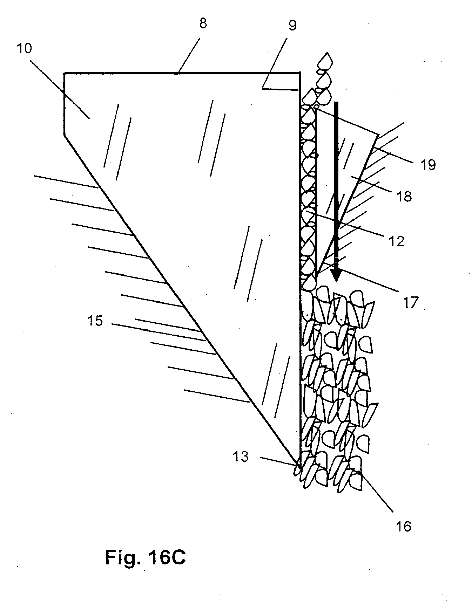

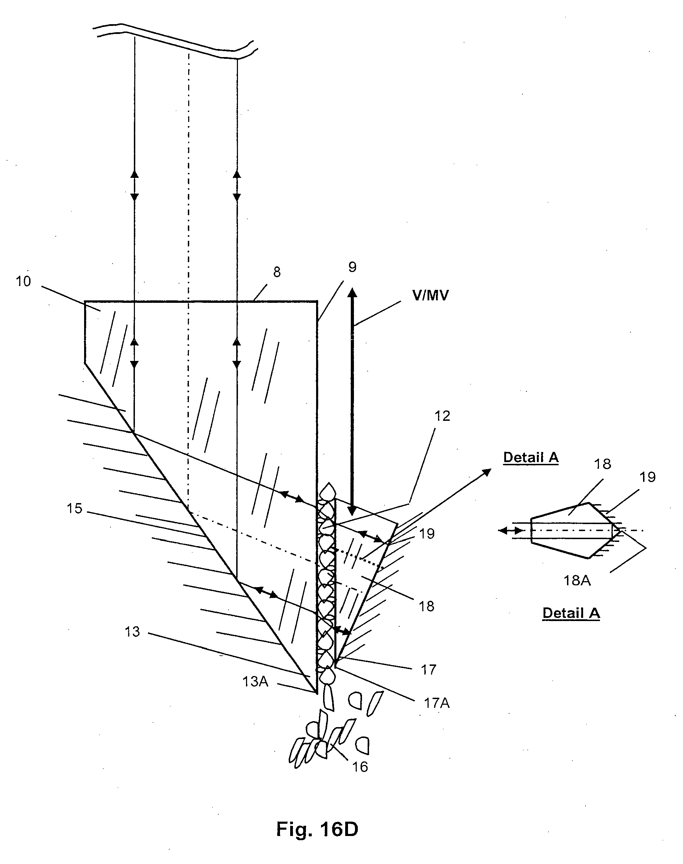

Bei den in Fig. 3 bis 15 gezeigten Beispielen ist der Messspalt 12 schräg zur Längsachse La der Messsonde angeordnet. **Fig. 16** bis **19** zeigen beispielhafte Anordnungen zur spektralen Absorptionsmessung mit Messsonden, bei welchen der Messspalt 12 im Wesentlichen parallel zur Längsachse La der Messsonde angeordnet ist.

**Fig. 16** zeigt eine Messsonde gemäß einem vierzehnten Beispiel und eine Anordnung zur spektralen Absorptionsmessung umfassend die Messsonde, wobei **Fig. 16A** einen Überblick der Anordnung zur spektralen Absorptionsmessung mit der Messsonde zeigt; **Fig. 16B** eine vergrößerte Darstellung des Vorderteils der Messsonde zeigt; **Fig. 16C** eine schematische Ansicht des Vorderteils des Messsonde bei einer ersten Stellung während des Schneidevorgangs zeigt und **Fig. 16D** eine schematische Ansicht des Vorderteils der Messsonde bei einer zweiten Stellung während des Schneidevorgangs zeigt. In diesem Beispiel ist der Endreflektor ein Spiegel, z.B. ein Goldspielgel, welcher als Hinter-Reflektor angeordnet ist.

Wie bei den obigen Beispielen umfasst die Anordnung zur spektralen Absorptionsmessung eine Lichtquelle 1. Die Lichtquelle kann z.B. eine durchstimmbare Quantenkaskaden-Laser-Batterie mit einem Wellenlängen-Durchstimmbereich von 7 µm bis 10 µm sein. Das von der Lichtquelle 1 ausgestrahlte Licht wird mittels Strahlformungsoptik 27 zumindest näherungsweise zu einem parallelen Mess-Strahlenbündel geformt. Der Mess-Strahlenbündel wird mittels des Fensterelements 10 der Messsonde in den Messspalt 12 eingekoppelt und nach zweifachem Durchgang durch den Messspalt 12 aus dem Messspalt 12 ausgekoppelt.

Das Fensterelement 10 ist als ein Diamantprisma mit einer superscharfen Hauptschneide 13 ausgebildet. Das Diamantprisma dient somit sowohl als Fensterelement 10 als auch als Hauptschneidestück. Die Hauptschneide 13 weist eine Schneidespitze bzw. Schneidekante 13A mit einem Winkel kleiner als 40° auf. Das Diamantprisma 10 weist ferner eine Eintrittsfläche 8, eine verspiegelte Fläche 15 und eine Fensterfläche 9 auf. Die Eintrittsfläche 8 des Fensterelements 10 ist in diesem Beispiel eine plane Fläche mit einer Breite 8A von ungefähr 0,8 mm, die senkrecht zur Längsachse La der Messsonde angeordnet ist. Die Fensterfläche 9 ist eine im Wesentlichen plane Fläche, die parallel zur Längsachse La der Messsonde ist. Die Fensterfläche 9 stellt eine der Begrenzungsflächen des Messspalts dar.

Die Messsonde umfasst ferner ein Endreflektor-Prisma 18, das ebenfalls aus Diamant gefertigt ist. Das Endreflektor-Prisma 18 weist eine scharfe Schneide 17 auf, hier als Nebenschneide bezeichnet, und dient sowohl als Endreflektor-Trägerelement als auch als Nebenschneidestück. Die Nebenschneide 17 weist eine Schneidespitze bzw. Schneidekante 17A mit einem Winkel von 15° auf. Das Endreflektor-Prisma 18 weist eine erste transparente, im Wesentlichen plane Fläche 25 (Fensterfläche) auf, welche im Wesentlichen parallel zur Längsachse La der Messsonde ist. Der Endreflektor 19 ist als Hinter-Spiegel ausgebildet und ist auf die zur Längsrichtung La geneigte Fläche des Endreflektor-Prismas 18 angeordnet. Der Endreflektor 19 ist als ein Retroreflektor ausgebildet, so dass das auf den Endreflektor 19 einfallende Messlicht in sich zurückreflektiert wird. Einen Querschnitt durch das Endreflektor-Prisma 18 mit dem Endreflektor 19 (vorzugsweise als Dachkante ausgebildet) ist in Fig. 16D, rechts (Detail A) gezeigt.

Der Messspalt 12 wird von der Fensterfläche 9 des Diamantprismas 10 und der Fensterfläche 25 des Endreflektor-Prismas 18 begrenzt. Im gezeigten Beispiel weist der Messspalt zwei zueinander parallel angeordnete Begrenzungswände auf, die jeweils parallel zur Längsachse der Messsonde sind. Es ist jedoch ebenfalls möglich, den Messspalt 12 keilförmig zu gestalten. Der Messspalt kann z.B. einen kleinen Keilwinkel aufweisen, der vorzugsweise eine mittlere Wellenlänge Lambda-mittel über der Spaltlänge beträgt.

Fig. 16B zeigt eine vergrößerte Ansicht des Vorderteils der Messsonde und des Strahlengangs des Messlichts. Hier ist der Messspalt 12 mit Wasser gefüllt, um den Strahlengang zu zeigen, der auch bei Gewebe im Messspalt gegeben ist, da der Brechungsindex von wässrigem Gewebe dem von Wasser sehr ähnlich ist. Das kollimierte Mess-Strahlenbündel trifft senkrecht auf die Eintrittsfläche 8 des Diamantprismas 10, wird an Spiegel bzw. an die verspiegelte Fläche 15 reflektiert und durch die Fensterfläche 9 in den Messspalt 12 eingekoppelt. Das Messlicht propagiert durch den Messspalt 12 mit der sich im Messspalt befindlichen Probenschicht und wird vom Endreflektor 19 zurückreflektiert. Nach Reflexion an dem Endreflektor geht das reflektierte Mess-Strahlenbündel im Wesentlichen in sich zurück, propagiert erneut durch den Messspalt 12, wird an Spiegel 15 reflektiert und tritt als im Wesentlichen paralleles Strahlenbündel aus dem Diamantprisma 10 aus. Mittels des Strahlteilers 2 und der Umlenkoptik 28 (z.B. Umlenkspiegel) wird das Messlicht in eine teleskopische Fokussieroptik 29 mit konfokaler Spaltblende zur Ausblendung von Störlicht eingekoppelt. Nach Filtern des Störlichts wird das Messlicht zu einem Detektor 30 geleitet (als Teil der Einrichtung zur spektralen Analyse). Der Detektor 30 kann z.B. ein Detektor für Strahlung im mittleren Infrarot sein, z.B. ein Quecksilber-Cadmium-Tellurid-Detektor (MCT). Das detektierte Licht wird anschließend mittels geeigneter Methoden analysiert.

Das Endreflektor-Prisma 18 mit der Nebenschneide 17 ist beweglich relativ zum Diamantprisma 10 mit der Hauptschneide 13. Insbesondere kann das Endreflektor-Prisma 18 entlang der Vorschubrichtung V der Messsonde bzw. entlang der Längsachse La der Messsonde bewegt werden. Das Endreflektor-Prisma 18 kann z.B. durch ein präzises Positionierungssystem (hier nicht dargestellt) mit Mikroantrieb geführt werden. Zu diesem Zweck kann in der Messsonde bzw. in der Anordnung zur spektralen Absorptionsmessung entsprechend Bauraum für ein Positionierungssystem mit Mikro-Antriebssystem vorgesehen werden.

Während des Schneide- und Messvorgangs wird zunächst, wie in Fig. 16C gezeigt, die Hauptschneide 13 bzw. das Diamantprisma 10 mit der Hauptschneide 13 durch die Hautoberfläche HO in eine Tiefe des zu untersuchenden Gewebes 16 gebracht. Anschließend wird die Nebenschneide 17 bzw. das Endreflektor-Prisma mit der Nebenschneide 17 ebenfalls vertikal nach unten und relativ zur der Hauptschneide 13 bewegt, um einen neuen Gewebelappen bzw. eine neue Probenschicht freizuschneiden. In Fig. 16D wird die Nebenschneide 17 des Endreflektor-Prismas 18 als bereits "nachgezogen" dargestellt, wobei ein neues Gewebelappenstück bzw. Probenschicht zur Messung freigeschnitten wurde. Das Endreflektor-Prisma 18 kann zur Unterstützung des Trennvorganges der Gewebeprobe 16 auch eine vergleichsweise hochfrequente Mikroschwingbewegung MV ausführen.

Das Diamantprisma 10, das Endreflektor-Prisma 18 und gegebenenfalls weitere Elemente (wie z.B. ein Entsorgungskanal, optische Faser, etc.) sind in einem Gehäuse 24 integriert, das aus einem biokompatiblen Material ist.

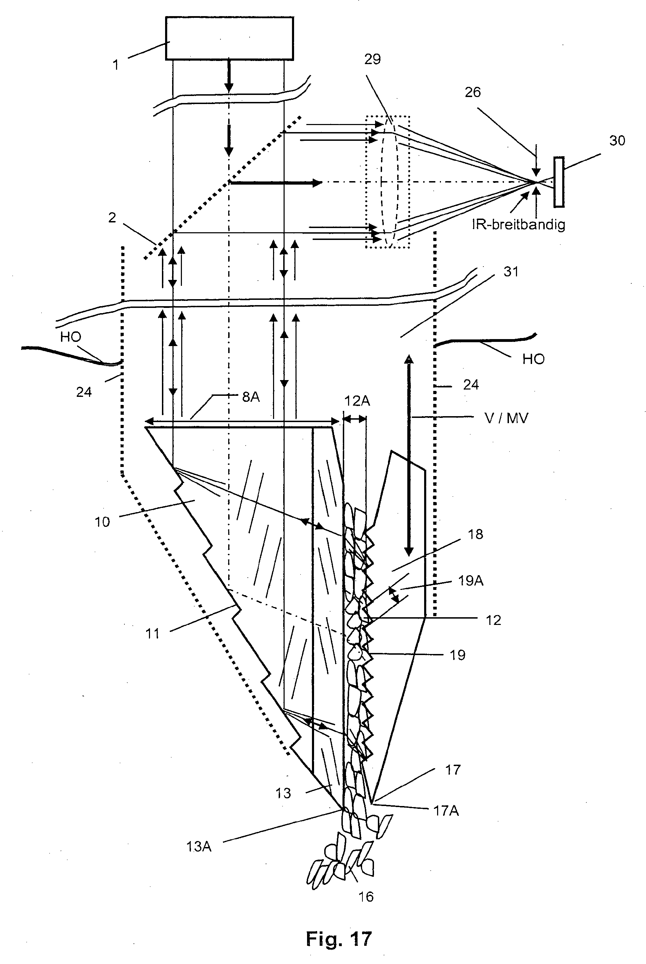

**Fig. 17** zeigt eine Messsonde gemäß einem fünfzehnten Beispiel und eine Anordnung zur spektralen Absorptionsmessung umfassend die Messsonde. Die optische Anordnung der Messsonde bzw. der Anordnung zur spektralen Absorptionsmessung entspricht weitgehend der optischen Anordnung der in Fig. 16 gezeigten Messsonden bzw. Anordnungen zur spektralen Absorptionsmessung, mit den folgenden Unterschieden:

Bei dem in Fig. 17 gezeigten Beispiel ist der Endreflektor 19 als ein geblaztes Reflexions-Beugungsgitter mit einer Gitterperiode 19A und einer Gitterhöhe 19B ((in den Figuren nicht maßstabgetreu dargestellt) ausgebildet und als Vorder-Spiegel angeordnet. Ferner wird anstelle des Spiegels 15 ein geblaztes Reflexionsgitters 11 (Kompensations-Beugungsgitter) eingesetzt. Des Weiteren ist das optische Fensterelement 10 aus zwei Teilen gebildet (einem ZnSe Prisma 10A und einem zweiten optischen Element aus Diamant 10B), die eine kompakte optische und mechanische Einheit bilden, wobei das ZnSe Prisma 10A hermetisch abgekapselt ist, um Gewebekontakt völlig zu vermeiden.

Das Diamantstück 10B weist eine scharfe Hauptschneide 13 auf, die spitzer als 40° ist. Der Messspalt 12 wird von einer der planen Flächen des Diamantstücks 10B und dem planen Endreflektor 19 begrenzt. Der Endreflektor 19 ist ausgelegt, beim nichtsenkrechten Auftreffen des Messlichts auf die Gitterfläche ein Teil des Messlichts (z.B. das in der ersten Beugungsordnung gebeugte Messlicht) etwa senkrecht in sich zurückgehen zu lassen. Durch die Verwendung eines geblazten Beugungsgitters kann ferner eine Verminderung des so genannten "Channelled Spektrum"-Effekts erzielt werden.

Das Reflexions-Beugungsgitter des Endreflektors 19 kann für die erste oder für eine andere Beugungsordnung (außer 0-te) ausgelegt sein. Das Reflexions-Beugungsgitter kann eine Periode von ungefähr 3 µm und eine Höhe von ungefähr 2 µm aufweisen.

Der als Vorder-Reflektor ausgebildete Endreflektor 19 ist auf ein Endreflektor-Trägeretement 18 angeordnet, das z.B. aus einem geeigneten harten Material (wie z.B. aus Diamant) oder als einem anderen geeigneten Material sein kann. Das Endreflektor-Trägerelement 18 mit der Nebenschneide 17 ist - wie bei der in Fig. 16 gezeigten Messsonde - beweglich relativ zum Fensterelement 10 mit der Hauptschneide 13. Insbesondere kann das Endreflektor-Trägerelement 18 entlang der Vorschubrichtung der Messsonde bzw. entlang der Längsachse La der Messsonde bewegt werden. Das Endreflektor-Trägerelement 18 kann ferner zur Unterstützung des Trennvorganges am Gewebe auch eine vergleichsweise hochfrequente Mikroschwingbewegung ausführen. Das Endreflektor-Trägerelement 18 kann z.B. durch ein präzises Positionierungssystem (hier nicht dargestellt) mit Mikroantrieb geführt werden. Zu diesem Zweck kann in der Messsonde bzw. in der Anordnung zur spektralen Absorptionsmessung entsprechend Bauraum 31 für ein Positionierungssystem mit Mikro-Antriebssystem vorgesehen werden. Der Schneidevorgang erfolgt wie bei der in Fig. 16 gezeigten Messsonde.

Das optische System der Messsonde ist ferner mittels des geblazten Reflexionsgitters 11 (Kompensations-Beugungsgitter) achromatisiert, das auf eine der Flächen des ZnSe-Prismas 10A angeordnet ist. Das Kompensations-Beugungsgitter 11 wird dabei zweimal im Strahlengang genutzt. Vorzugsweise wird das optische System der Messsonde derart achromatisiert, dass das hinlaufende spektral breitbandige Mess-Strahlenbündel für alle Wellenlängen im durchgestimmten Spektrum gut kollimiert ist. Ferner bevorzugt ist nach dreimaligem Gitterkontakt (2 Mal am Kompensations-Beugungsgitter 11 und 1 Mal am geblazten Reflexions-Beugungsgitter 19) das rücklaufende Mess-Strahlenbündel ebenfalls gut kollimiert. Da das Messlicht, welches das ZnSe-Prisma 10A verlässt, zumindest näherungsweise kollimiert ist, kann ein vergleichsweise großer Anteil des Messlichts die in der Fokalebene der achromatischen Fokussieroptik 29 angeordnete Blende 26 (Abschattblende bzw. Hilfsblende) passieren. Der große Anteil des Störlichts wird dagegen durch die Blende ausgefiltert. Das ermöglicht eine Verminderung des so genannten "Channelled Spectrum"-Effekts und des die Messungen verfälschenden Störlichts. Das gefilterte Messlicht wird anschließend mittels IR-Detektor 30 detektiert und anschließend analysiert.

Die restlichen Elemente und die Funktionsweise der Messsonde und der Anordnung zur spektralen Absorptionsmessung entsprechen im Wesentlichen den in den vorigen Figuren gezeigten Elementen (siehe insbesondere Fig. 16).

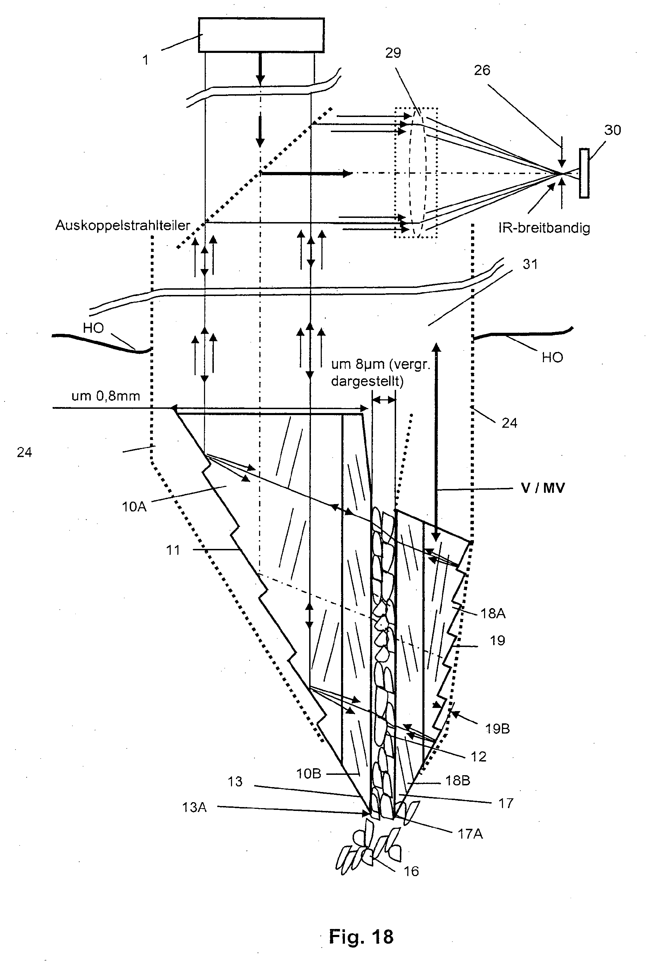

**Fig. 18** zeigt eine Messsonde gemäß einem sechszehnten Beispiel und eine Anordnung zur spektralen Absorptionsmessung umfassend die Messsonde. Wie bei dem in Fig. 17 gezeigten Beispiel ist der Endreflektor 19 als ein geblaztes Reflexions-Beugungsgitter ausgebildet.

Das optische System der in Fig. 18 gezeigten Messsonde bzw. Anordnung zur spektralen Absorptionsmessung entspricht im Wesentlichen dem optischen System der in Fig. 17 gezeigten Messsonde bzw. Anordnung zur spektralen Absorptionsmessung, mit dem Unterschied, dass der Endreflektor als Hinter-Reflektor ausgebildet ist. Ferner ist der Endreflektor-Trägerelement 18 ebenfalls aus einem Diamantstück 18B und einem ZnSe-Stück 18A ausgebildet, wobei das Diamantstück 18B und das ZnSe Stück eine kompakte optische und mechanische Einheit bilden. Ein Vorteil des ZnSe als Material ist, das es für das IR-Messlicht transparent ist. Zudem ist ZnSe gut bearbeitbar, was das Herstellen eines geblazten Reflexions-Beugungsgitter erleichtert. Das ZnSe-Stück ist mittels eines Edelstahlmantels 24 (Gehäuse) gut abgekapselt, so dass die Messsonde auch zur in-vivo Untersuchungen verwendet werden kann.

Die restlichen Elemente und die Funktionsweise der Messsonde und der Anordnung zur spektralen Absorptionsmessung entsprechen im Wesentlichen den in den vorigen Figuren gezeigten Elementen (siehe insbesondere Fig. 16 und 17).

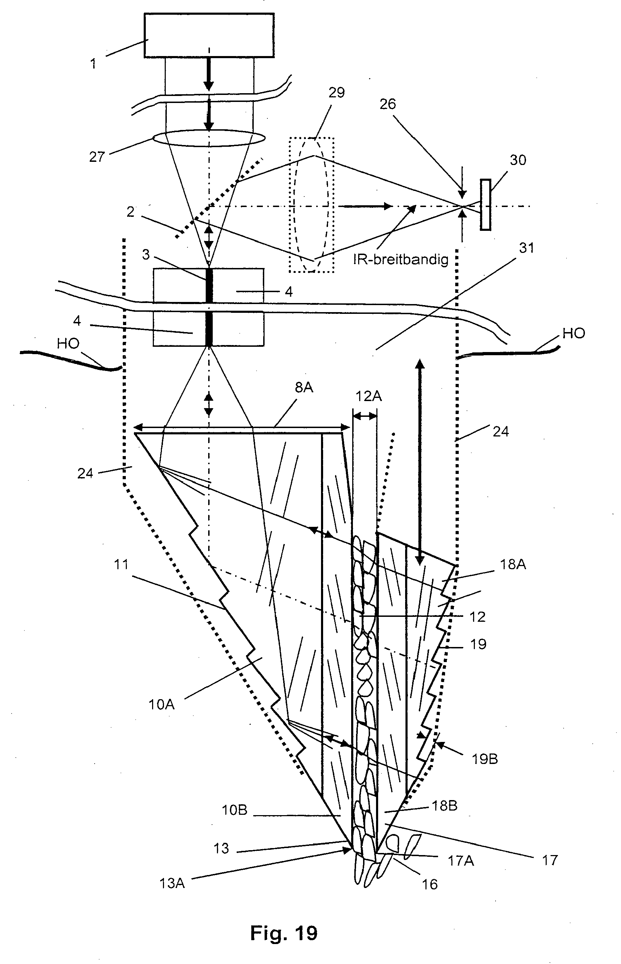

**Fig. 19** zeigt eine Messsonde gemäß einem siebzehnten Beispiel und eine Anordnung zur spektralen Absorptionsmessung umfassend die Messsonde. Das optische System der in Fig. 19 gezeigten Messsonde bzw. Anordnung zur spektralen Absorptionsmessung entspricht im Wesentlichen dem optischen System der in Fig. 17 gezeigten Messsonde bzw. der Anordnung zur spektralen Absorptionsmessung, mit dem Unterschied, dass die Messsonde bzw. die Anordnung zur spektralen Absorptionsmessung eine Faser 4 (beispielsweise eine MIR-Faser) mit einem Faserkern 3 umfasst. Die Faser kann z.B. eine Silber-Halide-Faser sein. Die Faser 4 ist sowohl Bestandteil des Beleuchtungs- als auch des Detektionspfads. Somit kann die Lichtquelle in einer etwas größeren Entfernung von der Messsonde, z.B. in einer konstruktiv günstigen Lage angeordnet sein.

Die Lichtquelle kann z.B. eine breitbandige oder eine durchstimmbare Lichtquelle sein. Das optische System der Messsonde ist achromatisiert und derart ausgelegt, dass das hinlaufende Mess-Strahlenbündel nach dem Kompensations-Beugungsgitter 11 (z.B. ein geblaztes Reflexions-Beugungsgitter) in Richtung Endreflektor 19 für die Mittenwellenlänge im breitbandigen oder durchgestimmten Spektrum kollimiert. Nach dreimaligem optischem Gitterkontakt (einmal an dem als Reflexions-Beugungsgitter ausgebildeten Endreflektor und zweimal an dem Kompensations-Beugungsgitter 11) gelangt das rücklaufende Bündel vorzugsweise für alle Wellenlängen im breitbandigen oder durchgestimmten Spektrum wieder in den Faserkern 3. Durch eine Blende (Abschattblende) 26 werden alle starken (schädlichen) Reflexionen ausgeblendet. Durch den vergleichsweise kleinen Endreflektor und die Verwendung einer Faser für Hin- und Rücklauf des Messlichts wird zusätzlicher Bauraum 31 frei, um z.B. ein Mikro-Antriebssystem und Positioniersystem zur Erzeugung eines für die Absorptionsmessung frei geschnittenen Gewebelappens oder andere Bauelemente anzuordnen. Ferner ist durch diese Anordnung Bauraum gegeben, um bei Bedarf auch einen Kanal zur "Entsorgung" geschnittenen Gewebes nach der Messung unterzubringen.

In allen obigen Beispielen (vgl. Fig. 3 bis 16) weist die Messsonde ein Gehäuse 24 in Form eines Edelstahlmantels auf, in dem die optischen Komponenten eingekapselt sind. Der Durchmesser des Edelstahlmantels 24 ist z.B. 1,8 mm. Während des Messvorgangs weist der Messspalt eine Breite von ungefähr 5µm bis 10µm auf, beispielsweise eine Breite von 8µm. Die gezeigten Messsonden sind insbesondere zur in-vivo Gewebemessung, z.B. während einer chirurgischen Operation geeignet.

In den obigen Beispielen kann die Messsonde bzw. die Anordnung zur spektralen Absorptionsmessung einen Filter umfassen, der ausgelegt ist, vorbestimmte Spektralbanden aus dem breitbandigen Spektrum herauszufiltern. Häufig liegt die gesuchte spektrale Signatur nur bei einer kleineren Anzahl (beispielsweise 20) von Absorptionsbanden und deren Verhältnis zueinander. Die - in einem definierten Kontext - relevanten Absorptionsbanden können vorbekannt sein und in einer Datenbank abgespeichert werden. Um die beim Absorptionsmessen eingebrachte Strahlungslast zu reduzieren, können mittels des Filters nur diejenigen Spektralbanden, bei denen Information erwartet wird, zur Absorptionsmessung dem Gewebe (vorzugsweise mit einer gewissen spektralen Verbreiterung) zugeführt werden. Der Filter kann z.B. einen Frequenzkamm-Filter umfassen, der im Wesentlichen nur die zur Absorptionsmessung relevanten Spektralanteile passieren lässt, d.h. die Spektralanteile, wo sich mit einer gewissen Wahrscheinlichkeit die (z.B. zur Krebsdiagnose) relevanten Absorptionsbanden befinden. Der Frequenzkamm-Filter kann z.B. wie ein dispersives Spektrometer mit einem Beugungsgitter aufgebaut sein. Im Bereich der spektralen Zerlegung des dispersiven Frequenzkamm-Filters können sich stark reflektierende und stark absorbierende Bereiche befinden, wobei die unerwünschten Spektralanteile durch die schwarzen bzw. stark absorbierenden Bereiche dunkelgeschaltet werden. Dies kann 80% der Intensität und mehr betreffen. Die stark reflektierten (erwünschten, relevanten) Spektralanteile können mittels Diffraktion an einem Beugungsgitter wieder in einem Fokusfleck vereinigt und möglichst gut in eine Faser (z.B. eine Monomode-Faser) eingekoppelt werden.

Bei einem Fourier-Ansatz (Fourier-Spektroskopie) kann dem Frequenzkamm-Filter ein scannendes Zweistrahl-Interferometer vor- oder nachgeordnet sein. Vorzugsweise wird dem Zweistrahl-Interferometer bereits durch a priori-Wissen adaptierte elektromagnetische Strahlung mit einem Kammspektrum zugeführt. Vorzugsweise ist die zu untersuchende Probe (z.B. das zu untersuchende Gewebe) wegen der Minimierung der Strahlungslast sowohl nach dem Frequenzkamm-Filter als auch nach dem scannenden Zweistrahl-Interferometer angeordnet. Der Strahlungs-Detektor (als Teil der Einrichtung zur spektralen Analyse) folgt vorzugsweise möglichst direkt der Probe (z.B. dem Gewebe) bzw. der Messsonde, um Signalverluste und zusätzlichen Rausch zu minimieren.

Bei einer Lichtquelle mit einem adressierbaren Spektrum kann für jede der relevanten Wellenlängen bzw. Wellenlängenbereiche (die den jeweiligen Absorptionsbanden entsprechen) eine Lock-in-Detektion durchgeführt werden. Da es sich um eine geringe Zahl von Absorptionsbanden (z.B. 20) handelt, deren mittlere Wellenzahl in der Regel sehr genau bekannt ist, kann auf diese geringe Anzahl von adressierten Frequenzen bzw. Frequenzbereichen eine Lock-in-Detektion durchgeführt werden.

Wenn als Lichtquelle eine ein MIR-Laser oder eine Batterie von MIR-Lasern zur Verfügung steht, welcher bzw. welche die benötigten Absorptionsbanden durch einen Wellenlängen-Scan abdeckt bzw. abdecken, kann das oben beschriebene Ausdünnen des Breiband-Spektrums entfallen. Die für die Krebsdiagnose nichtrelevanten Spektralbereiche können dann im Scan ausgelassen oder vergleichsweise schnell durchfahren werden.

Die Messergebnisse können in Echtzeit mit den vorab und unter vergleichsweise ähnlichen Bedingungen erzeugten spektralen Referenz-Signaturen der zu untersuchenden Probe (z.B. des zu untersuchenden Gewebe) verglichen werden. Die Referenz-Signaturen können z.B. als benign, premalign, malign oder auch feiner klassifiziert werden. Die spektralen Referenz-Signaturen können z.B. durch eine Lock-in-Detektion gewonnen werden. Vorzugsweise wird eine Zuordnung bzw. Bewertung der mittels der Messsonde ermittelten frisch gemessenen spektralen Signaturen in Echt- oder Quasi-Echtzeit (z.B. unter OP Bedingungen) durchgeführt. Der Detektionsvorgang kann eine Akkumulation von Interferogramm-Daten umfassen, wie es aus dem Stand der Technik bekannt ist. Vorzugsweise ist die Akkumulationszahl (d.h. die Anzahl der akkumulierten Interferogramme) n=1, da sich die Sonde im Gewebe bewegt (z.B. in das Gewebe gleitet). Für die Akkumulation von Interferogramm-Daten stellt ein Step-by-step-Betrieb eine Voraussetzung dar, da sonst optische Daten verschiedener Gewebeabschnitte in einer Messung vermischt werden.

Zum Messen verbleibt das Gewebe bzw. die Probenschicht vorzugsweise stets in der Messsonde. Während des Messvorgangs kann sich die Messsonde im Körper eines Patienten befinden (in-vivo Messung). Es ist jedoch möglich, die Messsonde zum Gewebe-Messen aus dem Körper des Patienten zu entfernen und an eine Messstation, z.B. über einen Faser-Port zu koppeln. In diesem Fall kann die Messdauer erhöht werden (z.B. auf einige 10 Sekunden), um eine bessere laterale Auflösung zu erhalten. Derweil kann mit einer anderen Sonde am Patienten weitergemessen werden. Zu diesem Zweck kann ein Vorrat an Messsonden in einem Magazin bereitgestellt werden. Es besteht ebenfalls die Möglichkeit, mit mehreren Messsonden gleichzeitig zu arbeiten und mit diesen an einem Port mit mehreren Andockstellen parallel messen.

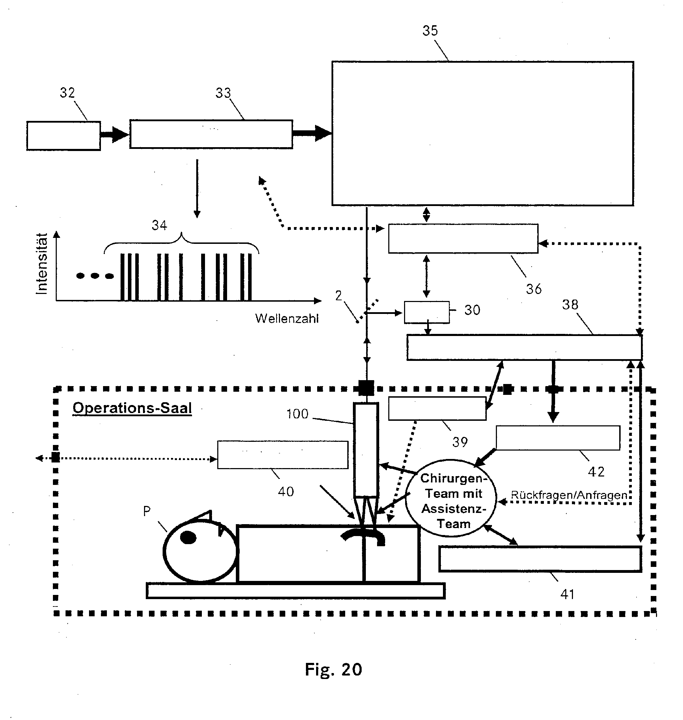

**Fig. 20** bis **25** zeigen beispielhafte Anordnungen zur spektralen Absorptionsmessung an einem Patienten P, z.B. unter OP-Bedingungen. Die Anordnungen zur Absorptionsmessung umfassen jeweils eine Lichtquelle, z.B. eine Multi-Wellenlängen-Lichtquelle mit mindestens zwei Wellenlängenbereichen, eine spektral breitbandige Lichtquelle, eine Wellenlängen-durchstimmbare Quelle, eine Lichtquelle mit adressierbarem Spektrum. Die Lichtquelle kann ferner einen Filter 33 zum Filtern von bestimmten Wellenlängenbereiche bzw. Wellenlängen umfassen. Ebenfalls kann die Lichtquelle einen Lichtmodulator zum Modulieren von bestimmten Wellenlängen oder Wellenlängenbereichen umfassen. Die Modulation kann eine zeitliche und/oder räumliche Modulation sein.

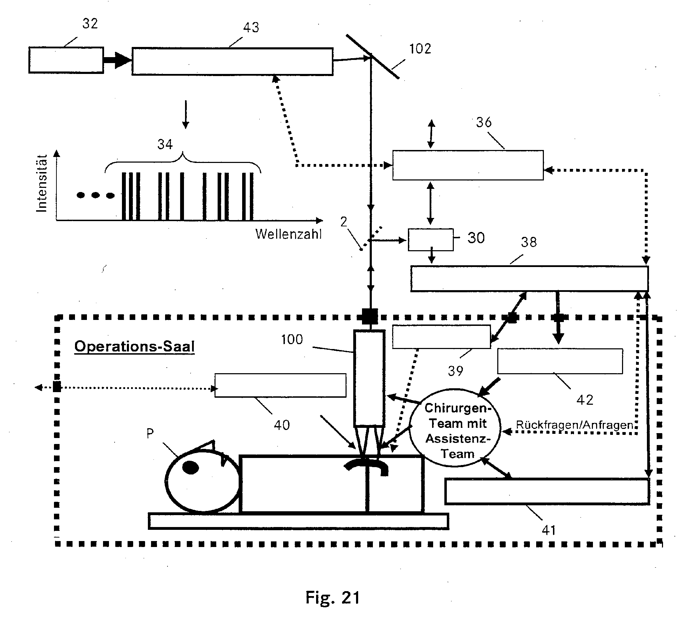

Ferner umfassen die Anordnungen zur Absorptionsmessung eine Einrichtung zur spektralen Analyse des von der Messsonde kommenden Signals. Die Einrichtung zur spektralen Analyse umfasst einen Detektor 30, z.B. einen gekühlten MCT-Detektor. Die Anordnung zur spektralen Absorptionsmessung kann ferner ein Interferometer 35 (Fig. 20 und 23 bis 25), ein Spektrometer 43 (Fig. 21), eine Vorrichtung zur zeitlichen Signalanalyse (z.B. zur zeitlichen Frequenzanalyse), etc. umfassen.

Die in Fig. 20 gezeigte Anordnung zur spektralen Absorptionsmessung umfasst eine breitbandige MIR Quelle 32, einen Filter 33 und ein Zweistrahl-Interferometer 35, der dem Filter 33 nachgeordnet ist.

Der Filter 33 kann, wie oben beschrieben, zum Filtern von bestimmten Wellenlängenbereichen bzw. Wellenlängen, die für die Informationsgewinnung von Interesse sind, ausgelegt sein. Der Filter kann z.B. ein Filter mit adaptierter Schmalband-Pass-Filter-Charakteristik sein. In einem Beispiel kann der Filter speziell für einen bestimmten Patienten bzw. für eine bestimmte Anwendung erstellt bzw. konfiguriert sein. So kann der Filter mit vorab erfassten Krebspatient-Daten, wie z.B. vorab erkannten hinreichend schmalen Absorptionsbanden von krebskranken und/oder krebsfreien Gewebe konfiguriert werden. Die Absorptionsbanden können z.B. durch eine spektrale Vermessung von Bereichen, die zweifelsfrei (z.B. durch bildgebende Verfahren und/oder histopathologische Untersuchungen) als krebsfrei und/oder krebskrank eingestuft sind bestimmt werden. Die Gewinnung der Daten, anhand deren der Filter erstellt bzw. konfiguriert wird, erfolgt vorzugsweise zeitnah vor der eigentlichen Messung (z.B. erfolgt ungefähr 12 Stunden vor einer chirurgischen Operation, bei der Messungen an Organen/Gewebe des Patienten durchgeführt werden).

Das durch den Filter 33 gefilterte und gegebenenfalls modulierte Spektrum 34 des Beleuchtungslichts weist folglich mehrere mit Intensität ausgestattete Spektralbereiche auf, die gemäß vorab ermittelter Daten (z.B. Krebs-Marker-Banden eines Patienten) festgelegt werden. Nur diese "Banden" bzw. Spektralbereiche sind als "Marker" zur Informationsgewinnung relevant. Dadurch kann die Strahlenbelastung durch Wärmestrahlung (MIR) erheblich reduziert werden, z.B. auf nur noch 5 bis 10% der Strahlenbelastung durch ungefiltertes Beleuchtungslicht. Dies minimiert das Risiko eines "Verbrennens" der untersuchten Probe (z.B. des untersuchten Gewebes) während des Messvorgangs. Die MIR-Quellen-Energie kann dann bis an die Maximal-Gewebe-Belastung "hochgefahren" werden, was das theoretisch schnellstmögliche Messen der Krebs-Marker-Banden ohne eine zu schnelle Gewebe-Zerstörung (vor Ende der Messung) ermöglicht. Dies setzt jedoch vergleichsweise starke MIR-Laser-Quellen voraus.

Das Interferometer 35 kann ein sehr schnelles und sehr robustes ("ruggedized") Zweistrahl-Interferometer sein, das vibrationsfest und langzeitstabil ist. Das Interferometer kann z.B. nach dem Drehreflektor-Prinzip (Double pendulum interferometer) mit Echtzeit FFT oder Echtzeit-Lock-in-Auswertung, arbeiten. Beispielhafte Interferometer sind z.B. aus DE3005520 A1, US4383762, DD210973A1, DE3400389A1, DE 42 12 143 A1 und GB2154019B sowie aus dem Fachartikel von K. Körner, "Ein neues Interferometer für die Fourier-Spektroskopie", Optik, Vol. 68, S. 217-223 (1984) bekannt.

Die Anordnung zur spektralen Absorptionsmessung umfasst ferner eine Messsonde 100, die z.B. eine der oben beschriebenen Messsonden sein kann. Es kann ferner ein Magazin mit Messsonden bereitgestellt werden, dass eine größere Anzahl (z.B. 16) von Messsonden aufweist. Somit kann der Chirurg oder der Chirurgie-Roboter nach Gebrauch einer Messsonde zur Detektion an weiteren (gegebenenfalls gesunden) Gewebebereichen eine neue sterile Messsonde einsetzen. Das ist vor allem sinnvoll, wenn an vielen Stellen optisch angetastet werden soll und Infektionsgefahr besteht oder eine Kontamination von gesundem Gewebe mit krankem Gewebe (wie z.B. mit Krebsgewebe-Zellen) unbedingt ausgeschlossen werden soll oder muss.

Das gefilterte und gegebenenfalls modulierte Licht der Lichtquelle wird mittels des Strahlteilers 2 in der Messsonde 100 eingekoppelt. Mittels des Strahlteilers 2 wird ebenfalls das Messlicht nach dem zweifachen Durchgang durch die sich im Messspalt der Messsonde befindliche Probenschicht zum Detektor 30 geführt.

Zur in-vivo Messung wird die Messsonde 100 mit oder ohne Chirurgie-Roboter-Unterstützung zunächst im tiefen Raum des zu untersuchenden Organs eingeführt, wobei die Vorschubrichtung der Messsonde 100 im Wesentlichen der Längsachse La der Messsonde entspricht. Vorzugsweise wird die Messsonde 100 Roboterarm-gestützt in das Organ bzw. in das Gewebe eines Patienten P eingebracht. Die Anordnung zur spektralen Absorptionsmessung kann dementsprechend eine Vorrichtung zum Einführen der Messsonde 41 umfassen. Die Vorrichtung kann z.B. ein Chirurgie-Roboter sein oder einen Chirurgie-Roboter umfassen. Das Einstechen der Messsonde 100 beim Messen erfolgt vorzugsweise mit zumindest näherungsweise konstanter Geschwindigkeit, z.B. =0,1 mm/s bis 0,5 mm/s. Liegen bereits Informationen bezüglich des zu untersuchenden Gewebes vor, können die als "sicher gesund" eingestuften Gewebebereiche schnell (z.B. mit 5 mm/s) durchfahren werden (Sonden-Anfahrt-Modus), da eine spektrale Messung in diesen Bereichen nicht notwendig ist.

Die Position der Messsonde 100 in Bezug auf das untersuchte Organ kann anhand von vorab ermittelten Daten bezüglich der räumlichen Orientierung des Patienten-Körpers und/oder des untersuchten Organs bestimmt werden. Zusätzlich kann die Position der Messsonde im untersuchten Organ mittels geeigneter Messverfahren (z.B. CT, Ultraschall, Mikroskop, etc.), vorzugsweise in Echtzeit, gemessen werden und auf einer geeigneten Anzeige (z.B. einem Bildschirm 40) dargestellt werden. Anhand der Daten bezüglich der Position der Messsonde kann der Messvorgang gestartet werden. Die Anordnung zur spektralen Absorptionsmessung kann dementsprechend eine Vorrichtung zur 3D Ortserfassung 39 der Position der Messsonde in Bezug auf die untersuchte Probe (z.B. in Bezug auf das untersuchte Organ) umfassen.

Zusätzlich zu den erfassten Daten bezüglich der Position der Messsonde 100 kann z.B. die globale Körperform (Körperverlagerungen) des Patienten gemessen werden, z.B. mittels schneller 3D-Streifen-Projektions-Messtechnik. Die erfassten 3D-Daten können zur aktiven Patienten-Fixierung genutzt werden. Vorab installierte Referenzpunkte des Patienten-Körpers (Marken) können z.B. zum Roboterarm durch ein OP-Tisch-System lagestabilisiert werden.

Das detektierte Messlicht wird mittels eines Kontroll- und/oder Rechensystems 38 spektral ausgewertet. Das Kontrol- und/oder Rechensystem 38 kann eine Datenbank umfassen, in der Referenz-Spektren gespeichert werden. Die Referenz-Spektren können bei der Analyse der detektierten Messdaten und/oder bei dem Anpassen des Filters 33 verwendet werden.

Die Referenz-Spektren können anhand von statistischen Untersuchungen ermittelt werden. Vorzugsweise werden die Referenz-Spektren anhand von Untersuchungen des konkreten Patienten, vorzugsweise zeitnah vor einer chirurgischen Operation ermittelt. Die Referenz-Spektren können z.B. durch Biopsie mit anschließender spektralen Messung ermittelt werden. Ein Grund ist, dass der Zellstoffwechsel im Körper eines Menschen teilweise größeren Schwankungen unterliegt, z.B. aufgrund schnell ablaufender entzündlicher Prozesse im Körper, Wasser- und Nahrungshaushalts-Dynamik, chronischer Stoffwechsel-Erkrankungen wie Diabetes, oder colitis ulcerosa, Hormonstatus, insbesondere bei Frauen, spezieller Medikamentengaben, etc. Vorzugsweise werden daher in einer (ersten kleineren) chirurgischen Diagnose-Operation oder Untersuchung (DOP) im Operationsgebiet Proben von Gewebe (z.B. in-vivo) spektral gemessen und histopathologisch bewertet. Vorzugsweise erfolgt die Vermessung in einem Zeitraum von weniger als 24 Stunden, besonders bevorzugt weniger als 12 Stunden vor der eigentlichen chirurgischen Operation. Die histopathologisch ermittelten Merkmale werden z.B. durch einen Histopathologen in Klassen unterteilt (z.B. benign, premalign, malign oder optional feinere Klassen) und den jeweiligen gemessenen Spektren zugeordnet. Die Merkmale bzw. Klassen und die Referenz-Spektren werden in der Patienten-Datenbank abgelegt. Die abgelegten Referenz-Spektren und die Klassifizierung können automatisch oder von dem Chirurgen bzw. Chirurgenteam abgerufen werden.

In einer anschließenden großen chirurgischen Operation (SOP, "Surgical Operation") werden mit der Messsonde bzw. Anordnung zur spektralen Absorptionsmessung Spektren von bisher nicht untersuchten Gewebeproben, die dem Chirurgen nun mechanisch und gegebenenfalls auch visuell erstmals frei zugänglich sind, gewonnen. Durch den Abgleich mit den gespeicherten patienten-bezogenen Referenz-Spektren (die in der DOP gewonnen wurden), kann eine Bewertung des während des SOP vermessenen Gewebematerials durchgeführt werden. Zur spektralen Analyse können bekannten Verfahren der statistischen Mathematik, wie z.B. Korrelationstechniken, PCA, hierarchische Verfahren, etc. eingesetzt werden.

In einem weiteren Ansatz kann vor der SOP zweifelsfrei ein Krebstumor oder eine andere pathologische Gewebeveränderung bei dem Patienten diagnostiziert worden sein. Diese Diagnose kann z.B. durch bildgebende Verfahren und histopathologischer Untersuchung erfolgen. Durch Femtosekunden-Blitze im infraroten Spektralbereich (vorzugsweise um die 3 µm Wellenlänge) kann freigeschnittene Gewebe in der Messsonde (z.B. das freigeschnittene Gewebe in einem kleinen Volumen an der Spitze der Messsonde bzw. in dem Messspalt) erwärmt, entwässert und/oder verdampft werden. Anschließend kann das verdampfende (vaporisierte) Gewebe mit einem dispersiven Single-Shot-Spektrometer oder einem anderen geeigneten Spektrometer nach der Messsonde zeitaufgelöst in MIR-Spektralbereich (z.B. im Bereich von 7µm bis 11µm) analysiert werden. Insbesondere kann das freigeschnittene Gewebe in der Messsonde mittels MIR-Strahlung zur spektroskopischen Analyse beleuchtet werden, so dass die von vaporisiertem Gewebe zurückkommende Strahlung hoher räumlicher Kohärenz optimal in den Spalt des dispersiven Single-Shot-Spektrometers eingekoppelt werden kann. Femtosekunden-Blitze und MIR-Strahlung können zeitversetzt gesendet werden, wobei das Spektrometer vor Femtosekunden-Blitzen vorzugsweise geschützt wird (z.B. durch einen steuerbaren optischen Verschluss). Mittels dieser Vorgehensweise können Referenz-Spektren gewonnen werden. Es ist ebenfalls möglich, diese Vorgehensweise auch bei der Gewinnung von spektralen Daten von bisher nicht untersuchten Gewebeproben in der SOP einzusetzen.

Anhand der gewonnen Spektraldaten während der SOP kann eine 2D oder 3D Karte 42 des untersuchten Gewebe, z.B. eine 2D oder 3D Tumorkarte erstellt werden. Die 3D Karte 42 kann in Echtzeit angezeigt werden.

Für die Operation kann ein detaillierter Ablaufplan für die Bewegung der Messsonde vorab erarbeitet und in einem Rechnersystem (z.B. im Rechensystem 38) für einen OP-Roboter hinterlegt werden. Aufgrund der Sachlage kann vorzugsweise der Plan für die Bewegung der Messsonde vergleichsweise schnell geändert werden und somit während der OP neue Schnittlinien für die Entfernung von Gewebe anhand der Sonden-Daten und gegebenenfalls weiterer Daten und Erkenntnisse (z.B. visueller Eindruck des Chirurgen, weitere Kameradaten, Tastbefund, allgemeine Situation des Patienten, Durchhaltevermögen des Patienten in der OP, Kreislauf-Situation) vorgeschlagen bzw. errechnet werden.

Das Kontrol- und/oder Rechensystem 38 kann ferner in Signalverbindung mit einer Gerätesteuerungsvorrichtung 36 stehen. Die Gerätesteuerungsvorrichtung kann z.B. den Filter 33, das Interferometer 35 und/oder andere optische und/oder mechanische Komponente der Anordnung zur spektralen Absorptionsmessung regeln und/oder steuern. Die Gerätesteuerungsvorrichtung 36 kann als Bestandteil des Kontrol- und/oder Rechensystems 38 oder als separates Modul ausgebildet sein.

**Fig. 21** zeigt eine andere beispielhafte Anordnung zur spektralen Absorptionsmessung. Der Aufbau und die Komponenten der Anordnung zur spektralen Absorptionsmessung entsprechen weitgehend den Aufbau und den Komponenten der in Fig. 20 gezeigten Anordnung, mit dem Unterschied, dass die Anordnung ein Spektrometer 43 umfasst. Das Spektrometer 43 kann ein SLM-Gitter-Spektrometer mit selektiver Frequenz-Spreizung sein. Das Spektrometer 43 kann ferner ein Filter mit einer Schmalband-Pass-Filter-Charakteristik aufweisen, wie in Zusammenhang mit Fig. 20 beschrieben. Das (modulierte) Messlicht wird über den Umlenkspiegel 102 und den Strahlteiler 2 in die Messsonde 100 eingekoppelt.

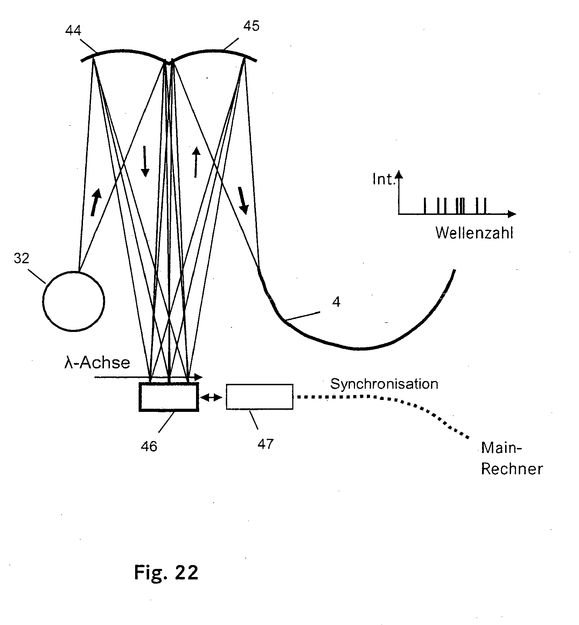

Bei den obigen Beispielen kann die Lichtquelle eine Lichtquelle (z.B. eine Lichtquelle im MIR) mit adressiertem Spektrum sein. **Fig. 22** zeigt eine beispielhafte Lichtquelle mit adressiertem bzw. adressierbarem Spektrum. Die Lichtquelle umfasst eine breitbandige Lichtquelle 32 mit einem kontinuierlichen oder diskreten Spektrum (z.B. eine brillante breitbandige Lichtquelle). Mittels eines ersten Beugungsgitters 44 werden die unterschiedlichen Spektralkomponenten des Spektrums räumlich voneinander getrennt und auf einen räumlichen Lichtmodulator 46 abgebildet, wobei die einzelnen Spektralkomponenten auf unterschiedliche Bereiche des räumlichen Lichtmodulators treffen. Der räumliche Lichtmodulator kann z.B. ein adressiertes Digital Micro Mirror Device mit Goldverspiegelung der Mikrospiegel-Elemente für Schmalband-Pass-Filter sein. Andere räumliche Lichtmodulatoren (z.B. LCDs) können ebenfalls eingesetzt werden. Mittels des räumlichen Lichtmodulators 46 werden die einzelnen Spektralkomponenten zeitlich moduliert, wobei die Modulationsfrequenzen der einzelnen Spektralkomponenten voneinander unterschiedlich sind. Der räumliche Lichtmodulator wird von einer Einrichtung zur Frequenzansteuerung 47 gesteuert bzw. geregelt.

Mittels eines zweiten Beugungsgitters 45 werden die einzelnen modulierten Spektralkomponenten wieder vereint und in Faser 4 eingekoppelt. Das Spektrum des von der breitbandigen Lichtquelle 32 erzeugten Lichts weist folglich mehrere Spektralkomponenten auf, die z.B. jeweils mit unterschiedlichen Frequenzen zeitlich moduliert sind. Die mittels eines Detektors (z.B. eines zwei-dimensionalen Detektors) detektierten Spektralkomponenten können mittels zeitlicher Fourier-Analyse, Wavelet-Analyse oder andere Frequenzanalyse voneinander getrennt werden. Eine geeignete Vorrichtung zur spektralen Analyse umfassend einer Lichtquelle mit adressiertem Spektrum ist z.B. in der deutschen Patentanmeldung DE 10 2014 002 514.4 beschrieben, auf die Bezug genommen wird.

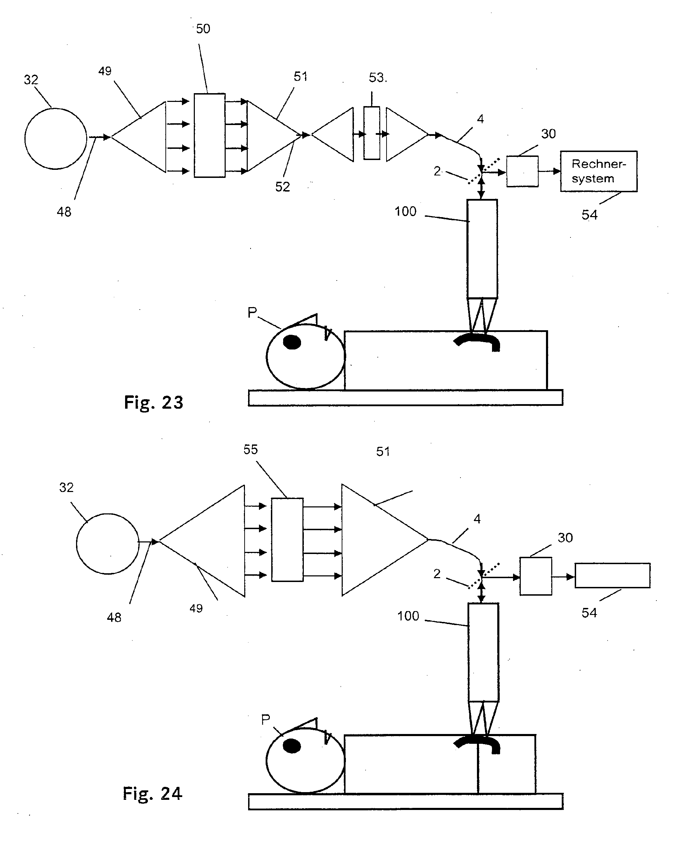

Fig. 23 zeigt eine andere beispielhafte Anordnung zur spektralen Absorptionsmessung. Die Anordnung umfasst eine brillante Quelle 32 (z.B. eine brillante Synchrotronquelle oder ein Laser-Paket, vorzugsweise im MIR Bereich), eine Vorrichtung zur spektralen Zerlegung 49, vorzugsweise mittels Diffraktion, eine Maske oder eine Vorrichtung zur statischen Lichtmodulation 50 (z.B. ein Filter), eine Vorrichtung zur räumlichen spektralen Vereinigung 51 der räumlich getrennten spektralen Komponenten (beispielsweise mittels Diffraktion), ein Interferometer 53 (z.B. ein Michelson-Interferometer), sowie optional Faser bzw. Lichtwellenleiter 52 und 4 (z.B. Monomodefaser). Die Vorrichtung zur statischen Lichtmodulation 50 kann, wie zuvor beschrieben, zum Ausdünnen des breitbandigen Spektrums der Quelle 32 dienen und z.B. einen räumlichen Lichtmodulator, z.B. einen reflektierenden SLM umfassen. Die Anordnung zur spektralen Absorptionsmessung umfasst ferner eine Messsonde 100 (z.B. eine der zuvor beschriebenen Messsonden), einen Detektor 30 und ein Kontrol- und/oder Rechensystem 38. Die Funktionsweise dieser Anordnung ist ähnlich der in Fig. 20 gezeigten Anordnung.

**Fig. 24** zeigt eine andere beispielhafte Anordnung zur spektralen Absorptionsmessung. Die Anordnung umfasst eine Lichtquelle mit adressierbarem Spektrum, wie z.B. die in Fig. 22 gezeigte Lichtquelle. Die Lichtquelle umfasst eine brillante Quelle 32 (z.B. eine brillante Synchrotronquelle oder ein Laser-Paket, vorzugsweise im MIR Bereich), eine Vorrichtung zur spektralen Zerlegung 49, zum Beispiel mittels Diffraktion, eine Vorrichtung zur dynamischen Lichtmodulation 55, eine Vorrichtung zur räumlichen spektralen Vereinigung 51 der räumlich getrennten und modulierten spektralen Komponenten (vorzugsweise mittels Diffraktion) und ein Interferometer 53 (z.B. ein Michelson-Interferometer). Das Licht mit adressierbaren Spektrum kann optional in einer Faser 4 (z.B. Monomodefaser) eingekoppelt werden und der Messsonde 100 zugeführt werden. Die Vorrichtung zur dynamischen Lichtmodulation 55 kann z.B. einen reflektierenden SLM umfassen und das Spektrum der breitbandigen Quelle 32 wie oben beschrieben adressieren. Optional kann die Vorrichtung zur dynamischen Lichtmodulation 55 ebenfalls zum vorbestimmten Ausdünnen des Spektrums ausgelegt sein. Die Anordnung zur spektralen Absorptionsmessung umfasst ferner einen Detektor 30 und ein Kontrol- und/oder Rechensystem 38, das ausgelegt ist, eine Frequenzanalyse des detektierten Messsignals durchzuführen. Die Funktionsweise dieser Anordnung ist ähnlich der in Fig. 20 gezeigten Anordnung.

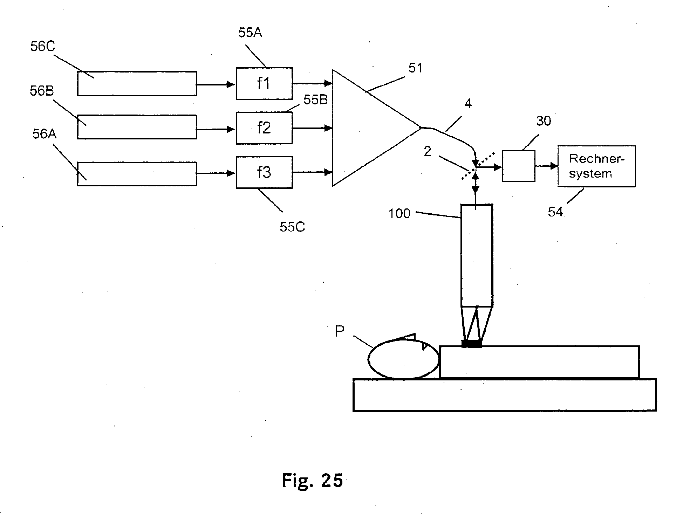

Fig. 25 zeigt eine andere beispielhafte Anordnung zur spektralen Absorptionsmessung mit einer Lichtquelle mit adressierbarem Spektrum. Die Lichtquelle mit adressierbarem Spektrum umfasst mehrere monochromatische Lichtquellen 56A, 56B und 56C, z.B. 3 Laser mit Wellenlängen von jeweils 1084 cm⁻¹, 1235 cm⁻¹ und 1650 cm⁻¹. Jeder der Lichtquellen 56A-C ist eine separate Modulationsvorrichtung 55A-C zugeordnet, die ausgelegt ist, das Licht der jeweiligen Lichtquellen 56A-C mit jeweils eigener Frequenz f1, f2, f3 zeitlich zu modulieren. Mittels der Vorrichtung zur räumlichen spektralen Vereinigung 51 werden die einzelnen modulierten monochromatischen Lichtstrahlen, zum Beispiel mittels Diffraktion, vereinigt und über eine Faser 4 in die Messsonde 100 eingekoppelt. Die Anordnung zur spektralen Absorptionsmessung umfasst ferner einen Detektor 30 und ein Kontrol- und/oder Rechensystem 38, das ausgelegt ist, eine Frequenzanalyse des detektierten Messsignals durchzuführen. Die Funktionsweise dieser Anordnung ist ähnlich der in Fig. 20 gezeigten Anordnung.

In den obigen Beispielen kann die Analyse der detektierten Daten eine Hauptachsentransformation (PCA, **P**rincipal **C**omponent **A**nalysis) für die Gewinnung spektraler Merkmale aus der untersuchten Probe umfassen. Das numerische Verfahren der PCA kann der spektroskopischen Analyse als numerisches selektives Werkzeug nachgeschaltet werden. Dazu können in Datenbanken abgelegte Spektren, deren spektrale Merkmale den zu suchenden Stoffen zugeordnet sind, herangezogen werden. Andere numerische Verfahren sind ebenfalls möglich.

Die Kopplung von Fourier-Transformations-Spektroskopie und PCA oder anderen numerischen Tools erfordert, dass die bereitzustellenden Spektren eine minimale spektrale Auflösung aufweisen bzw. nicht unterschreiten, um eine gute Merkmalsschärfe zu erhalten. Dies gewährleistet eine sichere Identifizierung und Klassifizierung der vermessenen Probe. Weiterhin dürfen die spektralen Daten ein minimales Signal/Rausch-Verhältnis nicht unterschreiten, um die Zuverlässigkeit der Aussagen nicht zu gefährden. Anhand der Analyse kann eine Klassifikation des untersuchten Gewebes (z.B. Krebsgewebe, gesundes Gewebe, gegebenenfalls mit Zwischenstufen) vorgenommen werden. Die Klassifikation der vermessenen Probe erfolgt vorzugsweise in Echt- oder Quasi-Echtzeit. Dadurch kann z.B. ein Chirurg bei der Entscheidung, ob und in welchem Umfang ein noch operabler Tumorherd entfernt werden soll, wirksam unterstützt werden. Insbesondere kann mittels der vorgeschlagenen Messsonden sowie Anordnung und Verfahren zur spektralen Absorptionsmessung das Auffinden von Schnittlinien für den Chirurgen im Sub-mm Bereich unterstützt werden. Die gewonnenen Daten können ebenfalls in einer Vorab-Untersuchung unter Narkose/örtliche Betäubung genutzt werden.

Die Spektren-Aufnahmezeit pro Messort im Messmodus liegt vorzugsweise von ungefähr 1s bis 10s, in einigen Fällen auch bis zu 100s. Kürzere Spektren-Aufnahmezeiten sind ebenfalls möglich. Eine gewisse laterale Verschmierung der Aufnahme-Orte für Spektraldaten wegen des Gleitens der Messsonde beim Messen kann gegebenenfalls akzeptabel sein. Es ist möglich, die Messung in einem Step-by-step-Modus durchzuführen, um eine hohe laterale (örtliche) Auflösung zu erreichen. Die Gesamtaufnahmezeit verlängert sich jedoch entsprechend.

Die Ortsauflösung der Messung liegt beispielsweise im Bereich von 0,05 mm bis 1 mm, vorzugsweise von 0,1 mm bis 0,3 mm. So kann z.B. eine ortsaufgelöste spektrale Signatur über die Gewebe oder entlang eines relevanten und zu definierenden Kanals im Gewebe erstellt werden. Da die Messsonde in Form einer volumen-minimierten, vergleichsweise dünnen Nadel bzw. Sonde gestaltet werden kann, ist es möglich eine in-vivo-Gewebe-Diagnostik durchzuführen und komplette Gewebe-Tiefen-Profile zu erstellen. Dies kann z.B. einer vergleichsweise schnelle Durchführung einer chirurgischen Operation mit minimalem Verlust an gesundem Organ-Weichteilgewebe für den Patienten dienen, indem die aus medizinischer Sicht optimalen Schnittlinien im Weichteilgewebe, vorzugsweise dreidimensional, ermittelt werden. Dies ermöglicht eine genaue OP-Planung oder eine datengestützte Anpassung einer OP in Echtzeit. Die Ausführung der OP kann anhand der mittels spektralen Absorptionsmessung gewonnen Daten (gegebenenfalls in Kombination mit anderen Daten) durch z.B. Roboterarm-Unterstützung erfolgen.

Die Messsonde bzw. die Anordnung zur spektralen Absorptionsmessung kann ferner mit anderen Vorrichtungen zum Erfassen von Daten kombiniert werden, z.B. OCT-Sonden, RAMAN-Sonden, etc. Insbesondere können die mittels spektralen Absorptionsmessung gewonnen Daten direkt verwendet werden (z.B. um Aussagen über die Beschaffenheit der Probe zu treffen) oder mit bereits vorhandenen Daten durch Messungen anderer Art (z.B. bildverarbeitende Mikroskopie im UV, VIS oder NIR, einschließlich Phasenkontrast-Mikroskopie, Fluoreszenz-Spektroskopie im NIR, Raman-Spektroskopie, NIR-Streulicht-Spektroskopie, CT, PET, MRT) kombiniert werden, so dass ein multivariater Datensatz, vorzugsweise in Echtzeit oder quasi-Echtzeit gebildet werden kann. Anhand dieses Datensatzes können z.B. von einem Chirurgen belastbare Entscheidungen für die Durchführung einer OP unter OP-Bedingungen getroffen werden.

Anwendungen der Messsonde, Anordnung und Verfahren zur spektralen Absorptionsmessung umfassen z.B. die Erkennung von Tumoren oder komplexen in-vivo Stoffwechselprozessen, z.B. in der chirurgischen OP Phase und/oder bei Gabe von Medikamenten (zeitaufgelöste Reaktionskinetik). Andere Anwendungen umfassen schnelle und zuverlässige Identifikationen von vorab bekannten (z.B. gefährlichen oder verbotenen) organischen Stoffen in einer hochkomplexen Situation in biologischen oder technischen Weichgewebe (in-vivo, ex-vivo). Das Vorhandensein bestimmter organischer Stoffen (wie z.B. Medikamentenrückstände, organische Gifte bei Tötungsdelikten, Drogen im Sport, undeklarierte oder verbotene Zusätze in Mastfleisch, etc.) kann z.B. sicher durch Molekülspektroskopie quantitativ bestätigt oder im Rahmen der gegebenen Messauflösung ausgeschlossen werden.

Ebenfalls sind Applikationen im technischen Bereich möglich, z. B. auch die Messung in weichen Kunststoffen und/oder Pflanzen.

Ein weiteres beispielhaftes Verfahren zur spektralen Absorptionsmessung umfasst folgende Schritte:

Es wird bei einer medizinischen Untersuchung ein dünner Gewebelappen bzw. eine dünne Gewebeschicht (Probenschicht) zumindest näherungsweise konstanter Dicke, auch im Tiefraum (ggf. auch Zentimetertief) von Gewebe zu diagnostischen Zwecken direkt in einer optischen Anordnung zur spektralen Absorptionsmessung erzeugt.

Der dünne Gewebelappen wird durchstrahlt, um Absorptionsspektren von Biomolekülen zu diagnostischen Zwecken direkt am Gewebe zu messen. Die genutzte Strahlung weist vorzugsweise einen spektralen Bereich im mittleren Infrarot auf.

Der erzeugte dünne Gewebelappen wird in einer Anordnung (mit seinen großen Flächen):
entweder von zwei zumindest näherungsweise planen Fensterflächen begrenzt (Endreflektor "im Hintergrund", wobei der Endreflektor dann ein Planspiegel sein kann); oder
von einer näherungsweise planen Fensterfläche (der Hauptschneide zugeordnet) und einem Endreflektor (ausgebildet als mikroprofiliertes (geblaztes) Reflexionsgitter) begrenzt.

Es wird mit einem Parallelbündel oder mit einem schwach fokussierten Bündel gearbeitet, das auf den Endreflektor trifft und dort stets reflektiert wird. Dabei werden die eine oder die zwei zumindest näherungsweise planen Fensterflächen schräg durchstrahlt. So schräg, dass kein an den Fensterflächen direkt reflektierter Lichtanteil genau denselben Weg zurück in Richtung Lichtquelle nehmen kann.

Der dünne Gewebelappen wird ein erstes Mal durchstrahlt, um dort Absorptionen zu erzeugen. Anschließend gelangt das Licht auf den Endreflektor. Nach Reflexion am Endreflektor wird der dünne Gewebelappen ein zweites Mal durchstrahlt, um noch einmal Absorptionen an demselben zu erzeugen (ergibt Verstärkung des Messeffekts).

Das vom Endreflektor reflektierte Licht - nach zweifachem Durchgang durch den Gewebelappen- geht dann
entweder zumindest näherungsweise in sich selbst zurück, wo anschließend durch Strahlteilung eine Auskopplung in den Detektionskanal erfolgt,
oder es breitet sich in einem vom Eingangsbündel-Strahlengang separierten Detektionskanal aus.

Der Endreflektor "im Hintergrund", vorzugsweise ausgebildet als Planspiegel, weist eine Neigung zur planen Fensterfläche der Hauptschneide auf. Damit wird ein unerwünschtes registrierbares "channelled spectrum" vermieden oder in seiner das Messergebnis verfälschenden Wirkung abgeschwächt.

Das Parallelbündel kann z.B. einem Laser, einem Quantenkaskaden-Laser im MIR entstammen. Der Quantenkaskaden-Laser ist vorzugsweise Wellenlängen-durchstimmbar ausgebildet. Alternativ kann der Laser als IR-Breibandlaser in Verbindung mit einem Spektrometer ausgebildet sein.

Das Erzeugen des dünnen Gewebelappens erfolgt vorzugsweise durch mechanisches Trennen mittels scharfer Schneiden. Der dünne Gewebelappen besitzt in Relation zu seiner geringen Dicke zwei vergleichsweise groß ausgedehnte Begrenzungsflächen. Diese liegen entweder an den beiden Fenstern an oder an einem Fenster und dem Endspiegel. Vorzugsweise wird jede Begrenzungsfläche des dünnen Gewebelappens durch eine eigene Schneide durch Trennen erzeugt. Die zwei ausgedehnten Begrenzungsflächen werden vorzugsweise durch mechanisches Trennen erzeugt.

Die Hauptschneide der Messsonde dringt bei einer medizinischen Untersuchung beim "Anfahren" des Diagnose-Zielgebietes stets als erste Schneide in tiefere Gewebeabschnitte ein. Die Nebenschneide kann sich relativ zu Hauptschneide bewegen. Die Nebenschneide bleibt jedoch stets zurück, befindet sich nie vor der Hauptschneide. Grund: Die Nebenschneide kann kleiner und schwächer sein.

Die Nebenschneide kann sich vorzugsweise auch durch eine Auf- und Ab-Schwingbewegung relativ zur Hauptschneide bewegen. Das kann das Erzeugen eines sehr dünnen Gewebelappens sehr begünstigen. Diese Schwingbewegung kann eine kleine Schwingamplitude hoher Frequenz aufweisen. Der mechanische Trennvorgang der Nebenschneide wird vorzugsweise durch Vibration im Ultraschall-Bereich unterstützt.

Der Vorschub eines Bewegungsvorganges einer Schneide entspricht vorzugsweise mindestens der halben Länge des lateralen Abtastbereichs beim Messen. Dabei bestimmt die Länge des Abtastbereichs die örtliche Auflösung der Messsonde.

Eine weitere beispielhafte Anordnung zur spektralen Absorptionsmessung weist folgende Merkmale auf:

Die spektrometrische Anordnung kann z.B. eine Breitbandquelle und ein Spektrometer oder eine Wellenlängen-durchstimmbare Quelle und einen Detektor und mindestens eine Messsonde mit optischem Kontakt zum Gewebe, welches von Licht durchstrahlt wird, umfassen. Die Wellenlängen-durchstimmbarer Quelle ist vorzugsweise ein Quantenkaskaden-Laser im mittleren IR.

In der Messsonde gibt es eine Haupt- und eine Nebenschneide zum Trennen von Gewebe, um einen vergleichsweise dünnen Gewebelappen zu erzeugen. Der dünne Gewebelappen kann für Messungen im mittleren Infrarot beispielsweise eine Dicke zwischen 1 µm und 30 µm besitzen, typischerweise um 4 µm bis 12 µm.

Dem Problem des unerwünschten "Channeled Spektrums", in dieser beispielhaften Anordnung hervorgerufen z.B. durch Mehrfachreflexionen im nahezu parallelen Messspalt, wird zum einen durch eine nicht zu geringe Absorption im Gewebelappen aufgrund der gewählten Lappen-Dicke, begegnet. Ferner werden die direkten Reflexionen an den Fensterflächen bei dieser Anordnung mit zumindest näherungsweise planen Fensterflächen an schräg durchstrahlten Fensterflächen entstehen, so dass die einzelnen Reflexionen an diesen eher nicht zu einem unerwünschten "Channeled Spektrum" führen können. Jedoch kann ein - durch eine ZickZack-Reflexion an den Fensterflächen erzeugtes unerwünschtes - Teilbündel-Paar sich mit dem Messbündel kohärent überlagern, da es am Parallelspalt dann die gleiche Ausbreitungsrichtung aufweist. Schon bei einer geringen Kippung des unerwünschten Teilbündels zum Messbündel schwächt sich der Effekt des unerwünschten "Channeled Spektrums" weiter ab. Dies setzt hierzu einen etwas keilförmigen Messspalt voraus. Der optimale Kippwinkel kann wie folgt quantitativ bestimmt werden: etwa eine halbe mittlere Wellenlänge auf die effektive Messlänge, um durch den Integrationseffekt über die Messfläche auf die erste Nullstelle der Interferenz-Modulation zu kommen. Bei wässrigem Medium genügt eine Keildicken-Änderung um 3µm bis 4µm auf die Messlänge (Spalthöhe/Spaltlänge). Allerdings ist dann wegen des keilförmigen Gewebelappens die Absorption merklich abhängig vom Ort des Messspaltes, was nicht optimal ist. So erfolgt eine Gewichtung des Mess-Feldes.

Ferner kann eine Antireflex-Schicht auf den Fensterflächen sehr helfen, den Effekt des unerwünschten "Channeled Spektrums" zu verringern. Für Diamant gibt es jedoch keine einsetzbaren Antireflex-Schichten. Hier kann das Herausrechnen (z.B. numerisches Differenzieren der Spektraldaten) helfen. Aufgrund des kleinen Spaltes, was nur wenige Wellenlängen optische Weglänge (OPD) für den Effekt des unerwünschten "Channeled Spektrums" bedeutet, ist dieses ja ohnehin eher sehr langperiodische Modulationen über der Wellenzahl.

In diesem Beispiel sind die Haupt- und Nebenschneide aus transparentem Material im IR-Bereich oder mit Komponenten aus transparentem Material im IR-Bereich starr verbunden (auf kürzestem Weg, in optischem Kontakt). Dem Hauptschneidestück ist vorzugsweise eine optische Fensterfläche zugeordnet. Der Nebenschneide ist vorzugsweise entweder eine optische Fensterfläche mit Hinterspiegel zugeordnet oder nur ein Vorder-Endspiegel (geblaztes Reflexionsgitter). An jeder Fensterfläche liegt im Messvorgang ein Teil des zumindest teilweise freigeschnittenen Gewebelappens oder von vereinzelten Gewebestücken an. Über die optischen Fensterflächen ist der optische Kontakt mit dem Gewebelappen hergestellt. Diese optischen Fensterflächen sind zumindest näherungsweise parallel.

Die Hauptschneide ist vorzugsweise Teil eines Prismas mit einer Lichteintrittsfläche (Hauptschneiden-Prisma). Der Nebenschneide ist ein Endreflektor zugeordnet. Der Endreflektor kann z.B. als Planspiegel (dann als Hinterspiegel), oder als Dachkantspiegelprisma (dann als Hinterspiegel), oder als mikroprofiliertes Reflexionsgitter (dann als geblazter Vorderspiegel) ausgebildet sein. Die optische Fensterfläche am Hauptschneiden-Prisma schließt entweder vorzugsweise einen spitzen Winkel mit der Längsachse La der Messsonde ein, oder ist vorzugsweise parallel zur Längsachse La der Messsonde. Ferner ist die Lichteintrittsfläche am Hauptschneide-Prisma vorzugsweise etwas geneigt, so dass Reflexionen an demselben durch die Blende (Abschattblende) im Detektionsstrahlengang (weitestgehend) geblockt werden.

Ferner ist im Detektionskanal und außerhalb der Messsonde vor dem Detektor eine Fokussieroptik mit einer feinen Blende (Abschattblende) in deren Brennebene angeordnet, um die (vielen starken) unerwünschten Reflexionsspots auszublenden. Dieses gilt insbesondere bei Anwendung eines Lasers (QCL). Die feine Blende ist vorzugsweise als Spaltblende ausgebildet.

Die Nebenschneide ist zur Hauptschneide feinverschiebbar ausgebildet. Zur feinen und hochpräzisen und reibungsarmen Verschiebbarkeit der Nebenschneide ist in die Messsonde vorzugsweise ein aerostatisches Mikro-Lager integriert, dass die Nebenschneide lagert. Dazu wird die Messsonde mit (gereinigter) Druckluft versorgt. Die Mikroschwingungen zur Verbesserung der Trennwirkung am Gewebe (vorzugsweise auch im Ultraschall-Bereich) wirken vorzugsweise auf die Nebenschneide, auf die Hauptschneide und/oder auf die Messsonde. Vorzugsweise ist im Detektionsstrahlengang ein Auskoppel-Strahlteiler zum Auskoppeln des Lichts nach dem zweifachen Passieren des Gewebelappens angeordnet. Dem Auskoppel-Strahlteiler ist eine Blende (Abschattblende) nachgeordnet.

Im Falle der Ausbildung des Endreflektors als Reflexionsbeugungsgitter ist vorzugsweise ein Kompensations-Beugungsgitter angeordnet, um das optische System bis zur Detektion zumindest näherungsweise zu achromatisieren. Damit ist eine bessere Effizienz für die IR-Messstrahlung und auch eine Unterdrückung von Störlicht (Reflexionen) im Detektionskanal verbunden.

Das geblazte Kompensations-Beugungsgitter ist vorzugsweise am Hauptschneidenstück angeordnet. Da Diamant nur schwer zu mikroprofilieren ist, bietet sich hier eine Kombination mit einem Zinkselenid-Stück an, das gut - auch rechnergesteuert mittels Diamantbearbeitung - herstellbar ist. Das Zinkselenid-Stück weist das geblazte Kompensations-Beugungsgitter auf. Das Zinkselenid-Stück ist mit einem Diamant-Schneidenstück verbunden.

Ferner kann es Hilfsschneiden (nicht dargestellt) geben, die den Gewebelappen auch an den schmalen Seiten freischneiden, damit dieser "nach oben" (nach draußen) entfernt werden kann. Das freigeschnittene und nach der Messung aus dem Körper abtransportierte Gewebe kann der weiteren externen medizinischen Untersuchung dienen (in der Histologie).

### Bezugszeichenliste

- 1: Lichtquelle zur IR Spektroskopie
- 2: Strahlteiler (Ein- und Auskoppelstrahlteiler)
- 3: Faser- bzw. Lichtwellenleiterkern
- 4, 4A, 4B: Lichtwellenleiter bzw. Faser (z.B. MIR-Faser)
- 4A: Sende- bzw. Beleuchtungsfaser bzw. -wellenleiter
- 4B: Detektionsfaser bzw. -wellenleiter
- 5, 5A, 5B: Faserkern-Ende
- 6, 6A, 6B: Linse (z.B. asphärische Linse)
- 7: Freiformfläche der asphärischen Linse
- 8: Eintrittsfläche des Fensterelements
- 8A: Breite der Eintrittsfläche
- 9: Fensterfläche
- 10: Fensterelement
- 10A, 10B: Komponente des Fensterelements
- 11: Kompensations-Beugungsgitter
- 12: Messspalt
- 12A: Breite des Messspalts
- 13: erste Schneide (Hauptschneide)
- 13A: Schneidkante bzw. -spitze
- 14: Probe-Einlauf
- 15: Spiegel
- 16: Probe (z.B. Gewebeprobe bzw. Gewebe)
- 16a: wässriges Medium
- 16b: Probepartikel (z.B. Gewebepartikel)
- 17: zweite Schneide (Nebenschneide)
- 17A: Schneidekante bzw. -spitze
- 18: Endreflektor-Trägerelement (z.B. Hartmetall- oder Diamantkörper)
- 18A, 18B: Komponente des Endreflektor-Trägerelements
- 19: Endreflektor
- 19A: Gitterperiode
- 19B: Gitterhöhe
- 20: Ausgang des Messkanals (Probeaustritt)
- 21: Abtransportkanal (Entsorgungskanal)
- 22: Einrichtung zur spektralen Analyse
- 23: Lichtmodulator (z.B. Michelson-Interferometer)
- 24: Gehäuse
- 25: Fensterfläche des Endreflektor-Trägerelements
- 26: Hilfsblende
- 27: Strahlformungsoptik
- 28: Umlenkoptik (z.B. Umlenkspiegel)
- 29: Fokussieroptik
- 30: Detektor
- 31: Bauraum (z.B. für ein Positionierungssystem für den Endreflektorträger)
- 32: Breitbandige Lichtquelle
- 33: Filter
- 34: Moduliertes Lichtspektrum
- 35: Zweistrahl-Interferometer
- 36: Gerätesteuerungsvorrichtung
- 38: Kontrol- und/oder Rechensystem
- 39: Vorrichtung zur 3D Ortserfassung
- 40: Bildschirm
- 41: Vorrichtung zum Einführen der Messsonde
- 42: 3D Karte
- 43: Spektrometer
- 44, 45: Beugungsgitter
- 46: räumlicher Lichtmodulator
- 47: Einrichtung zur Frequenzansteuerung
- 48, 52: Lichtwellenleiter
- 49: Vorrichtung zur spektralen Zerlegung
- 50: Vorrichtung zur statischen Lichtmodulation
- 51: Vorrichtung zur räumlichen spektralen Vereinigung
- 53: Interferometer (z.B. Michelson Interferometer)
- 54: Rechnersystem zur spektralen Analyse
- 55, 55A-C: Vorrichtung zur dynamischen Lichtmodulation
- 56A-C: monochromatische oder quasi-monochromatische Lichtquellen
- 100: Messsonde
- 102: Umlenkspiegel
- La: Längsachse der Anordnung zur Absorptionsmessung (z-Achse)
- V: Vorschubrichtung (Bewegungsrichtung der Messsonde)
- T: Probentransportrichtung (z.B. Gewebetransportrichtung)
- MV: Mikrovibrationen
- S: Messstrahl (vorzugsweise kollimiert)
- BO: Beugungsordnung
- P: Patient
- HO: Hautoberfläche

## Patentansprüche

1. Messsonde (100) zur spektralen Absorptionsmessung, umfassend
eine Schneideeinrichtung (13, 17), die ausgelegt ist, eine Probenschicht der zu vermessenden Probe (16), abzuschneiden;
einen Messspalt (12), der ausgelegt ist, die Probenschicht aufzunehmen;
ein optisches Fensterelement (10) zum Ein- und Auskoppeln von Messlicht in den bzw. aus dem Messspalt (12); und
einen Endreflektor (19), der ausgelegt und angeordnet ist, das durch den Messspalt propagierte Messlicht zurück in den Messspalt (12) zu reflektieren.

2. Messsonde gemäß Anspruch 1, wobei die Schneideeinrichtung zumindest eine Schneide (13; 17) aufweist, welche
Bestandteil des optischen Fensterelements (10) ist oder mit dem optischen Fensterelement (10) verbunden ist; oder
Bestandteil eines Endreflektor-Trägerelements (18) ist oder mit dem Endreflektor-Trägerelement (18) verbunden ist.

3. Messsonde (100) gemäß Anspruch 1 oder 2, wobei die zumindest eine Schneide eine Schneidespitze oder Schneidekante (13A; 17A) mit einem Winkel kleiner als 90°, vorzugsweise kleiner oder gleich 40°, besonders bevorzugt kleiner oder gleich 30° aufweist.

4. Messsonde (100) gemäß Anspruch 2, wobei die Schneideeinrichtung eine erste Schneide (13) und eine zweite Schneide (13) umfasst, wobei die zweite Schneide (17) vorzugsweise verschiebbar zur ersten Schneide (13) ausgebildet ist.

5. Messsonde (100) gemäß einem der Ansprüche 2 bis 4, wobei die zumindest eine Schneide (13; 17) aus einem für das Messlicht transparentem Material ist.

6. Messsonde (100) gemäß einem der vorangegangenen Ansprüche, wobei
der Messspalt (12) parallel oder keilförmig (12) ist, wobei der Keilwinkel weniger als 1°, vorzugsweise weniger als 0,1° beträgt; und/oder
der Messspalt (12) eine variable Breite aufweist.

7. Messsonde (100) gemäß einem der vorangegangenen Ansprüche, wobei die Messsonde (100) eine längliche Form mit einer Längsachse (La) aufweist, und wobei der Messspalt (12) in einem von Null unterschiedlichen Winkel zu der Längsachse (La) der Messsonde (100) angeordnet ist, wobei der Winkel zwischen der Längsrichtung des Messspalts (12) und der Längsachse (La) der Messsonde (100) vorzugsweise von 3° bis 70°, weiter bevorzugt von 10° bis 60°, besonders bevorzugt von 20° bis 50° beträgt.

8. Messsonde (100) gemäß einem der Ansprüche 1 bis 6, wobei die Messsonde (100) eine längliche Form mit einer Längsachse (La) aufweist und wobei der Messspalt (12) im Wesentlichen parallel zu der Längsachse (La) angeordnet ist.

9. Messsonde (100) gemäß einem der vorangegangenen Ansprüche, wobei
der Endreflektor (19) ein Retroreflektor ist; und/oder
der Endreflektor (19) eine der den Messspalt (12) begrenzenden Flächen bildet; und/oder
der Endreflektor (19) parallel oder geneigt zu einer Fensterfläche des Fensterelements (9) angeordnet ist, wobei die Fensterfläche (9) so ausgelegt und angeordnet ist, dass das Messlicht durch die Fensterfläche (9) in den Messspalt (12) eingekoppelt und aus dem Messspalt (12) ausgekoppelt wird.

10. Messsonde (100) gemäß einem der vorangegangenen Ansprüche, wobei die Messsonde (100)
mindestens ein Kompensations-Beugungsgitter (11) umfasst, das ausgelegt ist, das optische System der Messsonde (100) zumindest näherungsweise zu achromatisieren; und/oder
mindestens einen spektralen Filter (33) umfasst, der ausgelegt ist, vorbestimmte Spektralbanden aus einem breitbandigen Spektrum oder aus einem Spektrum mit mehreren Wellenlängen oder Wellenlängenbereiche herauszufiltern.

11. Messsonde (100) gemäß einem der vorangegangenen Ansprüche, wobei die Messsonde (100) derart ausgelegt ist, dass der Lichteinfall des Messlichts auf die refraktiven optischen Flächen der Messsonde (100) nicht senkrecht ist.

12. Anordnung zur spektralen Absorptionsmessung umfassend:
eine Messsonde (100) gemäß einem der vorangegangenen Ansprüche;
eine Lichtquelle (1), wobei die Lichtquelle eine spektral breitbandige Lichtquelle, eine Multi-Wellenlängen Lichtquelle, eine Wellenlängen-durchstimmbarer Quelle, oder eine Lichtquelle mit adressierbarem Spektrum umfasst; und
eine Einrichtung zur spektralen Analyse (22) des von dem Messspalt (12) ausgekoppelten Messlichts.

13. Verfahren zur spektralen Absorptionsmessung einer Probe mittels einer Messsonde nach einem der Ansprüche 1 bis 11 oder einer Anordnung zur spektralen Absorptionsmessung nach Anspruch 12, umfassend
Aufnehmen einer Probenschicht in dem Messspalt (12) der Messsonde (100);
Durchführen zumindest einer spektralen Messung der sich im Messspalt befindlichen Probenschicht, umfassend
Einkoppeln von Messlicht in den Messspalt (12) durch das Fensterelement (10) und Durchstrahlen der sich im Messspalt (12) befindlichen Probenschicht;
Reflektieren des einmal durch die Probenschicht propagierten Messlichts an dem Endreflektor (19) zurück in den Messspalt (12) und erneutes Durchstrahlen der sich im Messspalt (12) befindlichen Probenschicht;
Auskoppeln des, durch die sich im Messspalt befindliche Probenschicht, zweifach propagierten Messlichts aus dem Messspalt (12) durch das Fensterelement (10);
Detektieren zumindest eines Teils des ausgekoppelten Messlichts.

14. Verfahren nach einem der Ansprüche 13 oder 14, wobei
das Messlicht nach dem Auskoppeln aus dem Messspalt (12) zumindest näherungsweise in sich selbst zurückgeht und anschließend in einem Detektionskanal eingekoppelt wird; oder
das Messlicht nach dem Auskoppeln aus dem Messspalt (12) in einem vom Eingangsbündel-Strahlengang separierten Detektionskanal eingekoppelt wird.

15. Verfahren gemäß Anspruch 13 oder 14, ferner umfassend
eine Verdichtung oder Dehydrierung der in dem Messspalt (12) aufgenommenen Probenschicht; und/oder
ein Filtern eines von einer Lichtquelle emittierten breitbandigen spektralen Lichts, um vorbestimmte spektrale Bereiche passieren zu lassen, wobei die zumindest eine spektrale Messung mit Messlicht in den vorbestimmten spektralen Bereichen durchgeführt wird.
